# EUROPEAN PATENT APPLICATION

(11) **EP 2 607 364 A1**
(43) Date of publication of application: **26.06.2013**
(21) Application number: 11195214.9
(22) Date of filing: 22.12.2011
(51) Int. Cl.: C07D 471/14, C07D 519/00, A61K 31/519, A61P 25/00

(54) **Tetraaza-cyclopenta[a]indenyl and their use as positive allosteric modulators**

(71) Applicant: Ares Trading S.A., 1170 Aubonne (CH)
(72) Inventor: Swinnen, Dominique, 74160 Beaumont (FR); Montagne, Cyril, 01630 Saint-Genis-Pouilly (FR); Pomel, Vincent, 74160 Groisy (FR); Quattropani, Anna, 1207 Geneva (CH); Molette, Jérôme, 01210 versonnex (FR); Gerber, Patrick, 1163 Etoy (CH)
(74) Representative: Merck Serono S.A. Intellectual Property

(57) **Abstract**

The present invention provides compounds of Formula (I) as M1 receptor positive allosteric modulators for the treatment of diseases mediated by the muscarinic M1 mediator.

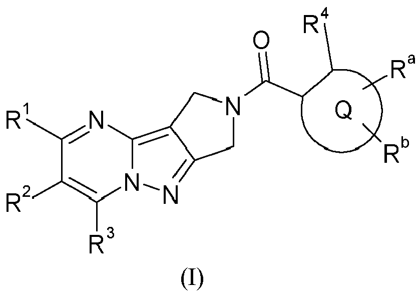

## Description

The invention is directed to a class of tetraaza-cyclopenta[a]indenyl compounds, their salts, pharmaceutical compositions comprising them and their use in therapy of the human body. In particular, the invention is directed to a class of tetraaza-cyclopenta[a]indenyl compounds, which are muscarinic M1 receptor positive allosteric modulators, and hence are useful in the treatment of Alzheimer's disease, schizophrenia and other diseases mediated by the muscarinic M1 receptor.

### BACKGROUND OF THE INVENTION

Alzheimer's disease is a common neurodegenerative disease affecting the elderly, resulting in progressive memory impairment, loss of language and visuospatial skills, and behavior deficits. Characteristics of the disease include degeneration of cholinergic neurons in the cerebral cortex, hippocampus, basal forebrain, and other regions of the brain, neurofibrillary tangles, and accumulation of the amyloid β peptide (Aβ is a 39-43 amino acid produced in the brain by processing of the beta-amyloid precursor protein (APP) by the beta-amyloid protein cleaving enzyme ("beta secretase" or "BACE") and gamma-secretase. The processing leads to accumulation of Aβ in the brain.

The cholinergic pathway is involved in a variety of Central Nervous System (CNS) functions like information processing, attention, learning and memory, nociception, regulation of sleep-wake cycles, motor control. Agents that regulate cholinergic transmission are used to treat various CNS disorders including chronic and neuropathic pain, sleep disorders, epilepsy, schizophrenia, Alzheimer's disease, Parkinson's disease, and other movement disorders and memory disorders (Jeffrey Conn et al. Trends in Pharmacological Sciences Vol 30, N° 30, p148, 2009, Gregory Digby et al. Mol Biosystems 2010, 6, 1345-1354).

Activation of muscarinic receptors is a way to counteract cholinergic hypofunction. Muscarinic receptors are prevalent throughout the body. Five distinct muscarinic receptors (M1-M5) have been identified in mammals. In the central nervous system, muscarinic receptors are involved in cognitive, behavior, sensory, motor and autonomic functions like cardiovascular functions, renal and gastrointestinal functions. The muscarinic M1 receptor, which is prevalent in the cerebral cortex, hippocampus and striatum, has been found to have a major role in cognitive processing and in the pathophysiology of Alzheimer's disease (Eglen et al, TRENDS in Pharmacological Sciences, 2001, 22:8, 409-414).

M1 agonists have the potential to treat the underlying disease mechanism of Alzheimer's disease. The cholinergic hypothesis of Alzheimer's disease is linked to both β-amyloid and hyperphosphorylated tau protein. Formation of β-amyloid may impair the coupling of the muscarinic receptor with G-proteins. Stimulation of the M1 muscarinic receptor has been shown to increase formation of the neuroprotective aAPPs fragment, thereby preventing the formation of the Aβ peptide. Thus, M1 agonists may alter APP processing and enhance aAPPs secretion (Fisher, Jpn J Pharmacol, 2000, 84:101-112).

Non selective muscarinic ligands which have been developed and studied for Alzheimer's disease have produced side effects, such as sweating, nausea and diarrhea (Spalding et al, Mol Pharmacol, 2002, 61:6, 1297-1302).

The muscarinic receptors are known to contain one or more allosteric sites, which may alter the affinity with which muscarinic ligands bind to the primary binding or orthosteric sites ( S. Lazareno et al, Mol Pharmacol, 2002, 62:6, 1491-1505; S. Lazareno et al, Mol Pharmacol, 2000, 58, 194-207).

Positive allosteric modulation has several advantages in the treatment of CNS disorders. In particular, it mimics neurotransmission under physiological conditions, with greater subtype selectivity. Also, the maximum effect reached by an allosteric modulator is not exceeded by increasing the dose (Jan Jakubik, Pharmaceuticals, 2010, 3, 2838).

Furthermore, the antipsychotic potential of M1 allosteric modulation provides a promising way of treating schizophrenia, dementia, and related disorders like hallucination, delusions, paranoia and other disorganized behaviors (Thomas Bridge et al. Drugnews&perspectives 2010, 23, 229).

Thus the compounds of the present invention, which are muscarinic M1 receptor positive allosteric modulators, are useful in the treatment of CNS disorders including Alzheimer's disease, Parkinson's disease, schizophrenia, and other diseases mediated by the muscarinic M1 receptor like movement disorders and memory disorders, chronic and neuropathic pain, sleep disorders, epilepsy.

The present invention also provides a method of synthesis of the compounds of Formula (I) as well as pharmaceutical formulations containing them.

More particularly the compounds of the present invention are compounds of Formula (I) 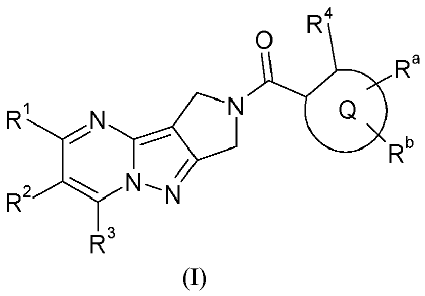 Wherein
- R¹, R², R³: are independently from each other selected from H, linear or branched C₁-C₆-alkyl, linear or branched C₁-C₆-alkoxy, Hal, or hydroxyl;
- R^{a}, R^{b}: are independently from each other selected from H, Hal, hydroxyl or A;
- Q: denotes a 6-membered aromatic group or a 5-6-membered heteroaromatic group having 1 to 4 heteroatoms independently selected from N, O and S.
- R⁴: denotes G, OG, SG, NR⁵G, -COOG, or OCOG;
- R⁵: denotes H or a linear or branched alkyl having 1 to 6 carbon atoms.
- G: denotes -CH₃, -CF₃, -CH₂-A, Het, Cyc, Ar, -CH₂-Het, -CH₂-Cyc, -CH₂-Ar, Hal, hydroxyl;
- Hal: denotes F, Cl, Br or I, preferably F, Cl or Br;
- A: is a linear or branched carbon chain having 1 to 6 carbon atoms, wherein 1 to 3 non adjacent -CH₂-groups may be independently from each other replaced by a group selected from O, NR⁵, S, SO, SO₂, CO, and wherein 1 to 5 hydrogen atoms may be independently from each other replaced by Het, Cyc, Ar, or Hal;
- Het: denotes a saturated, unsaturated or aromatic ring, being monocyclic or bicyclic or fused-bicyclic and having 3- to 8- members and containing 1 to 4 heteroatoms independently selected from N, NR⁵, O, S, CO, SO or SO₂, which may be substituted by 1 to 3 substituents independently selected from A, Hal, OH and Het¹;
- Het¹: denotes a 4, 5 or 6 membered carbocyclic ring wherein 1 or 2 carbon atom are replaced by Oxygen atoms.
- Ar: denotes a 6-membered carbocyclic aromatic ring or a fused or non fused byclic aromatic ring, and optionally substituted by 1 to 3 substituents independently selected from A or Hal;
- Cyc: denotes a saturated or unsaturated carbocyclic ring having from 3 to 8 carbon atoms and optionally substituted by 1 to 3 substituents independently selected from A or Hal;
as well as pharmaceutically acceptable salts, isomers and tautomers thereof.

In a specific embodiment, the compounds of the present invention are compounds of Formula (I) wherein
- Q: is a phenyl ring;
- R^{a},: R^{b} are independently selected from H, Hal, Hydroxy, or a linear or branched alkyl group having 1 to 6 carbon atoms and wherein 1 to 3 hydrogen atoms may be replaced by Hal;
- R⁴: is G or OG;

In another specific embodiment, the present invention provides compounds of Formula (I) and related Formulae wherein Q bears R⁴ and the rest of the molecule on two adjacent atoms.

In another specific embodiment, the group 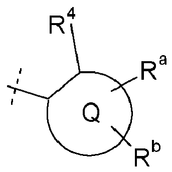 is selected from one of the following groups

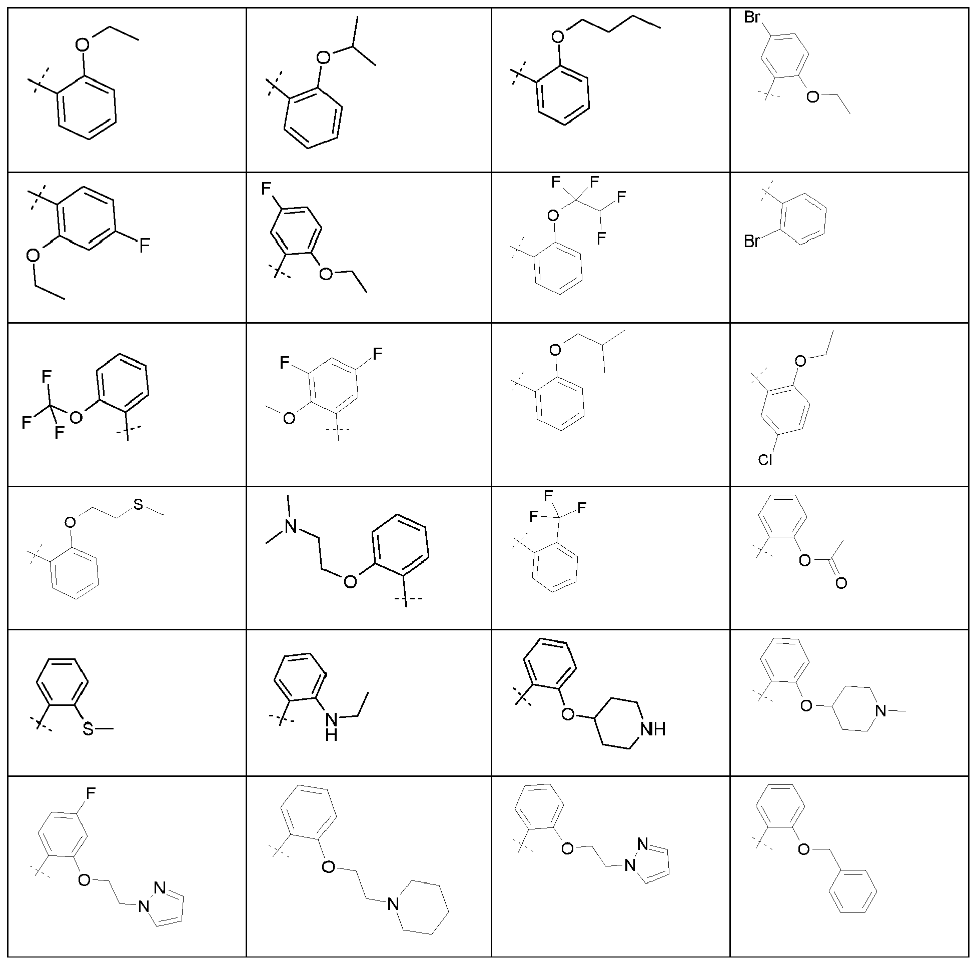

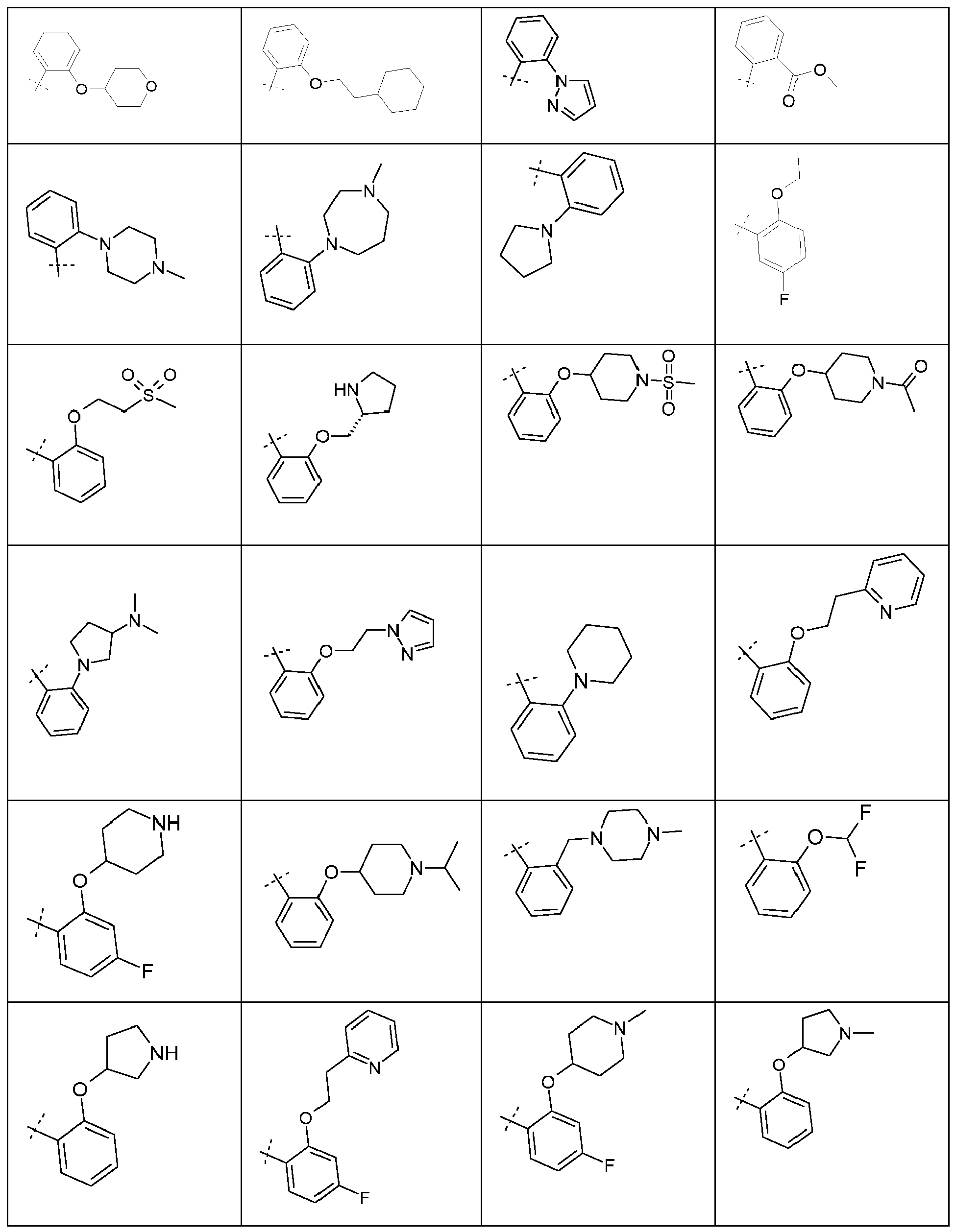

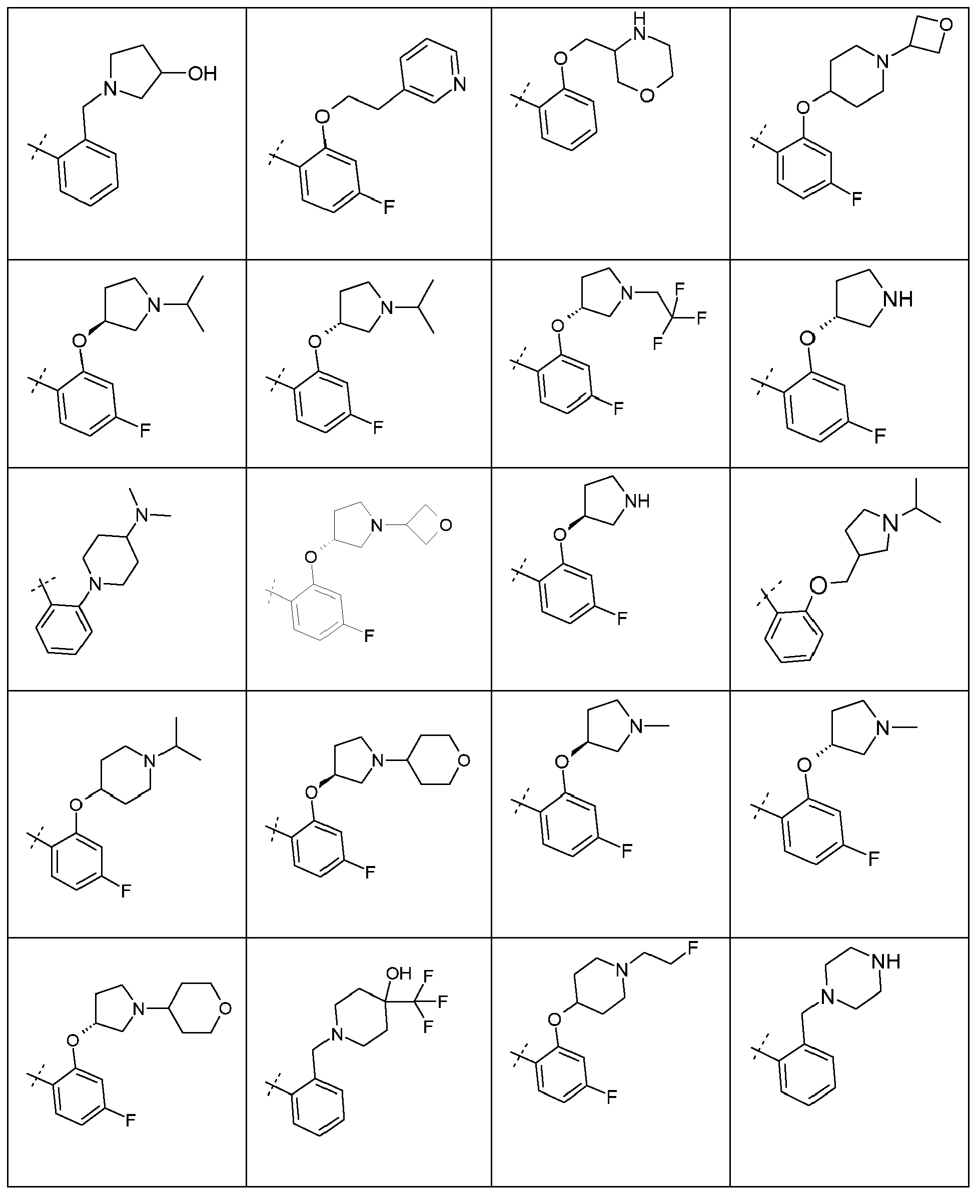

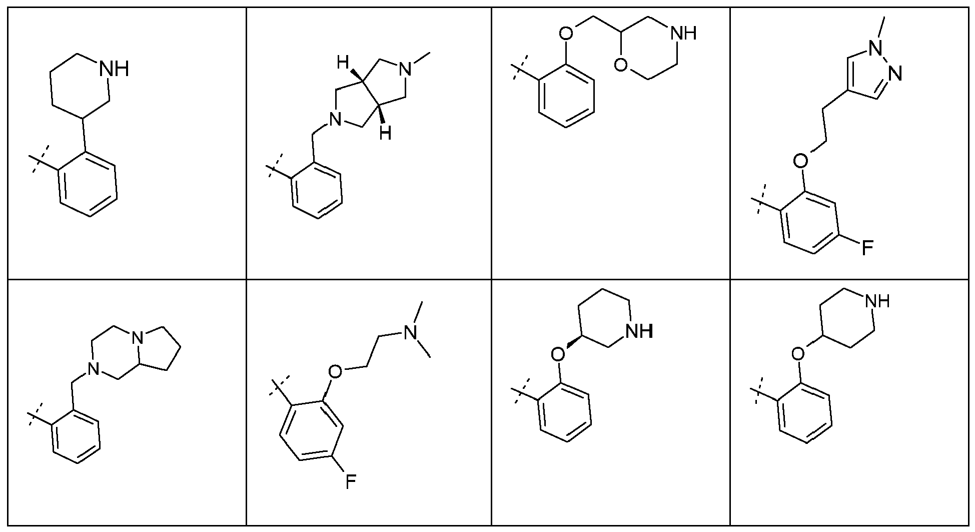

Examples of compounds provided by the present invention are the following:

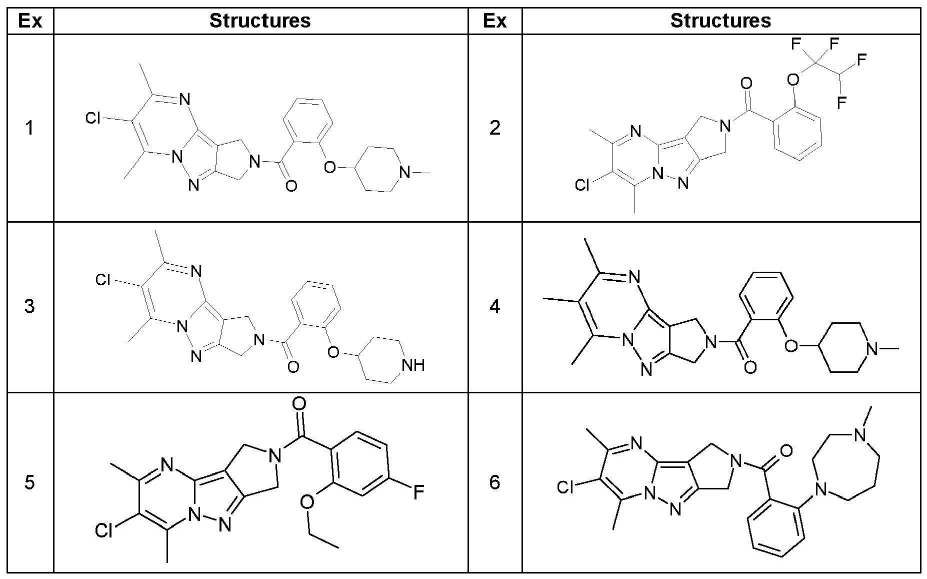

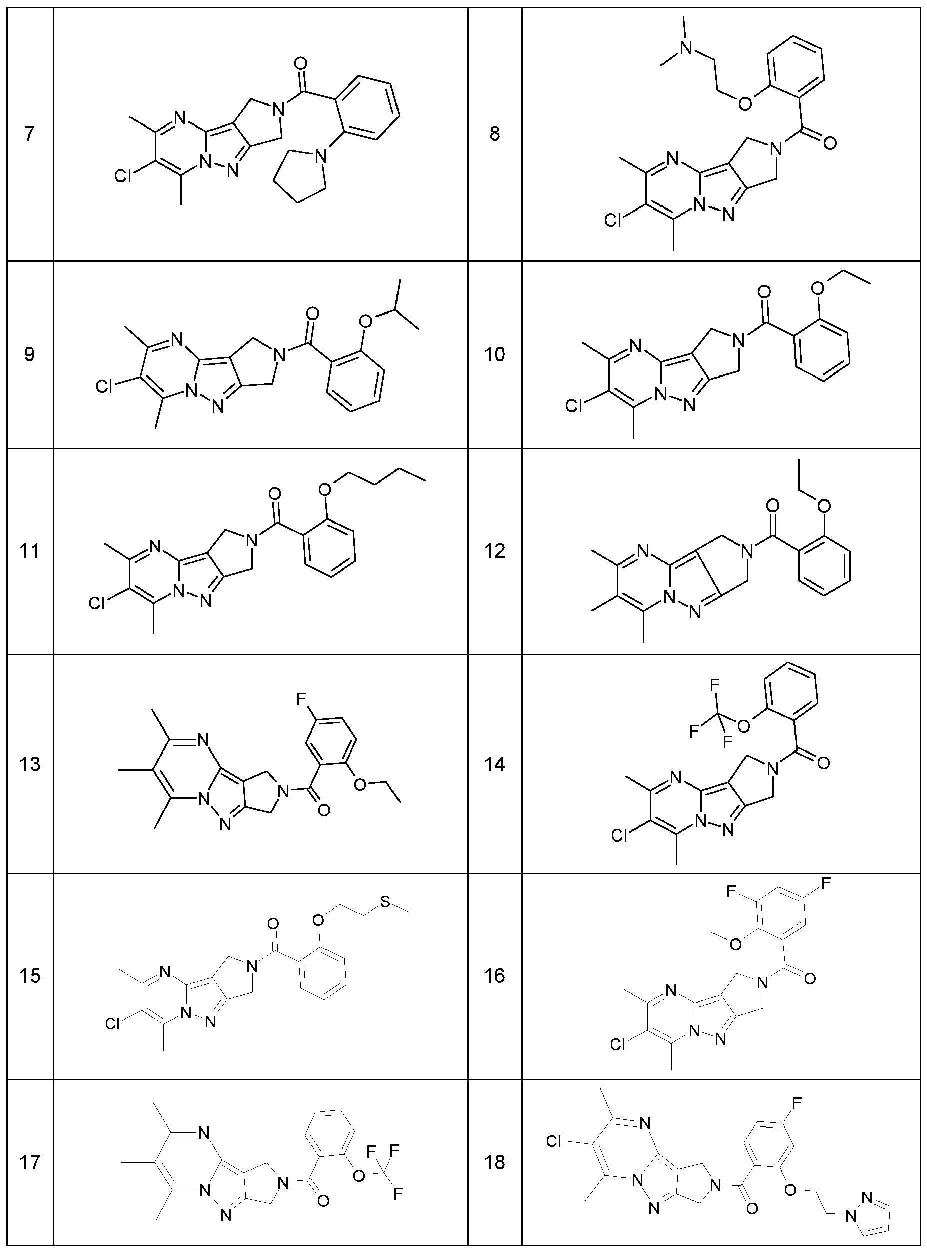

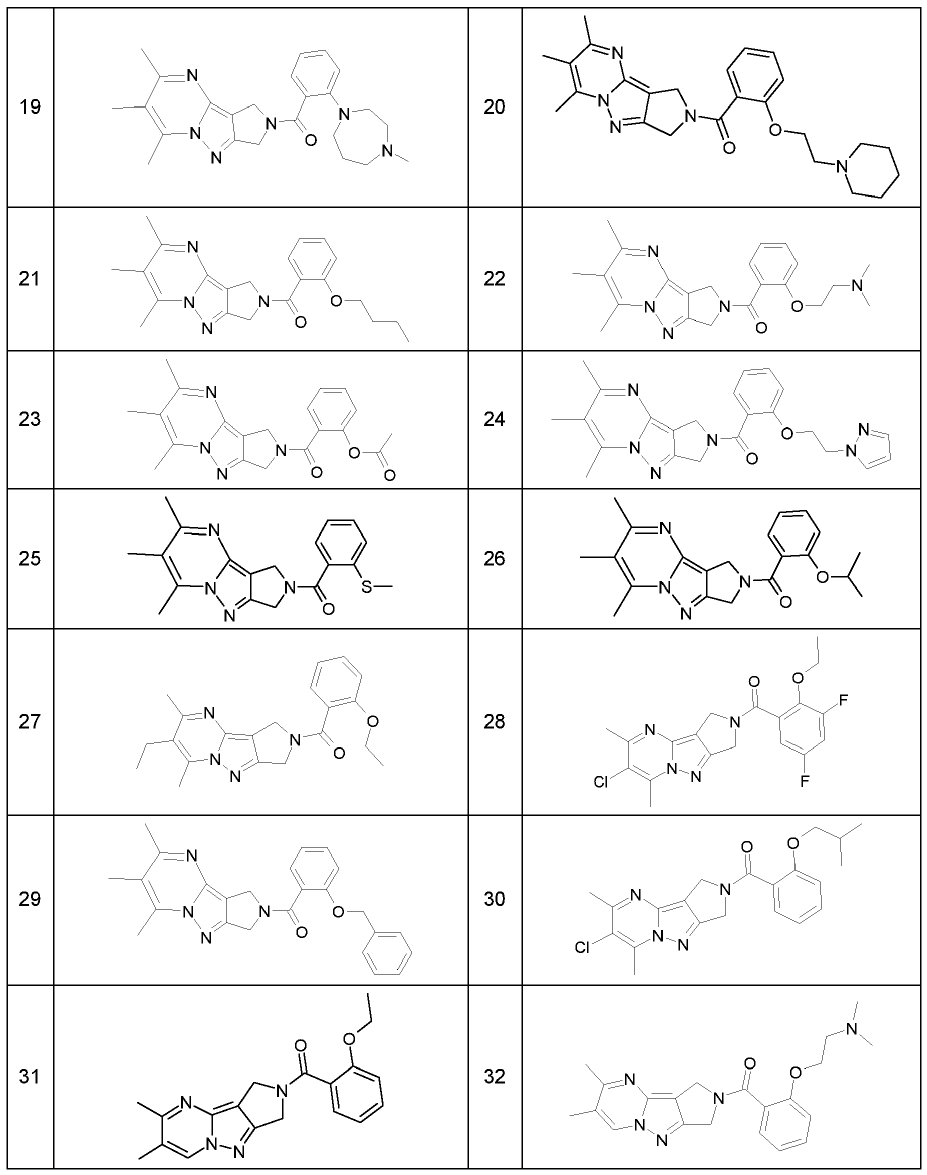

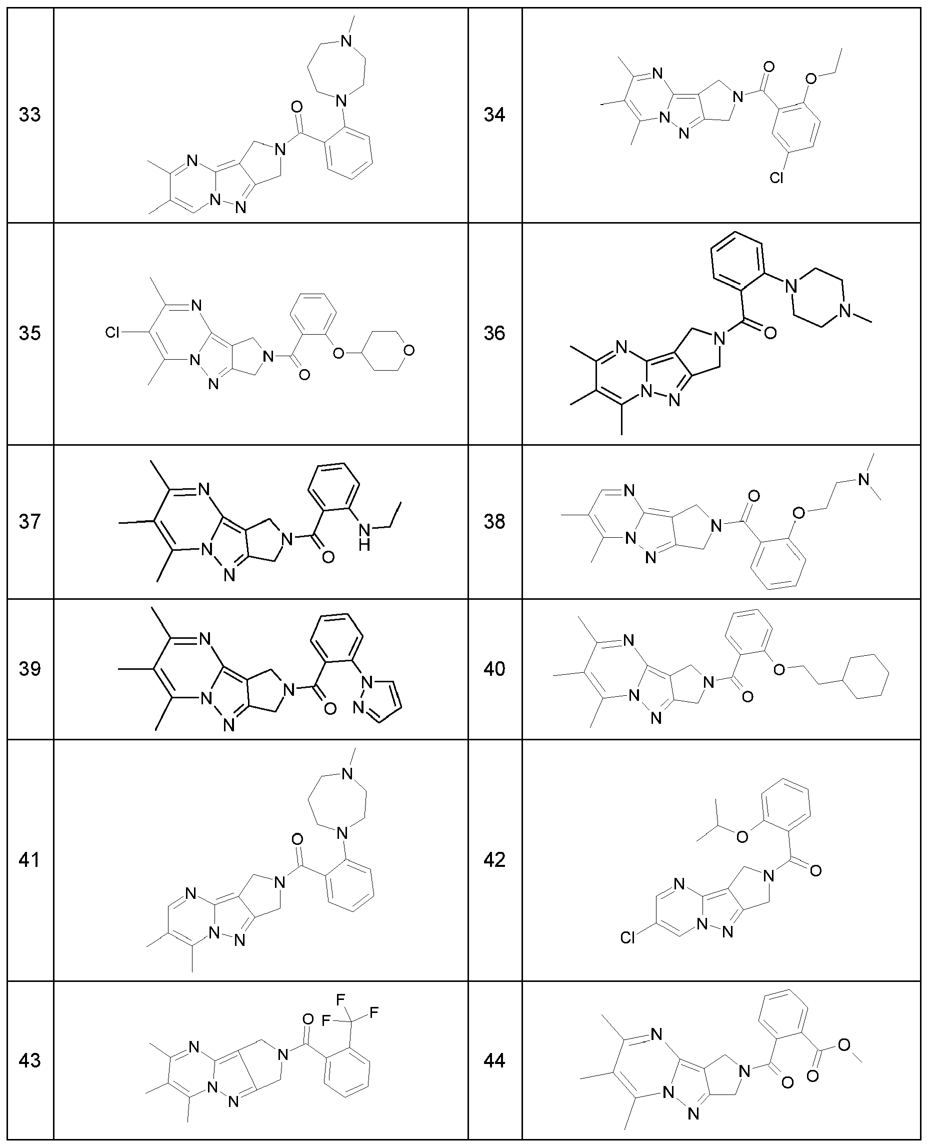

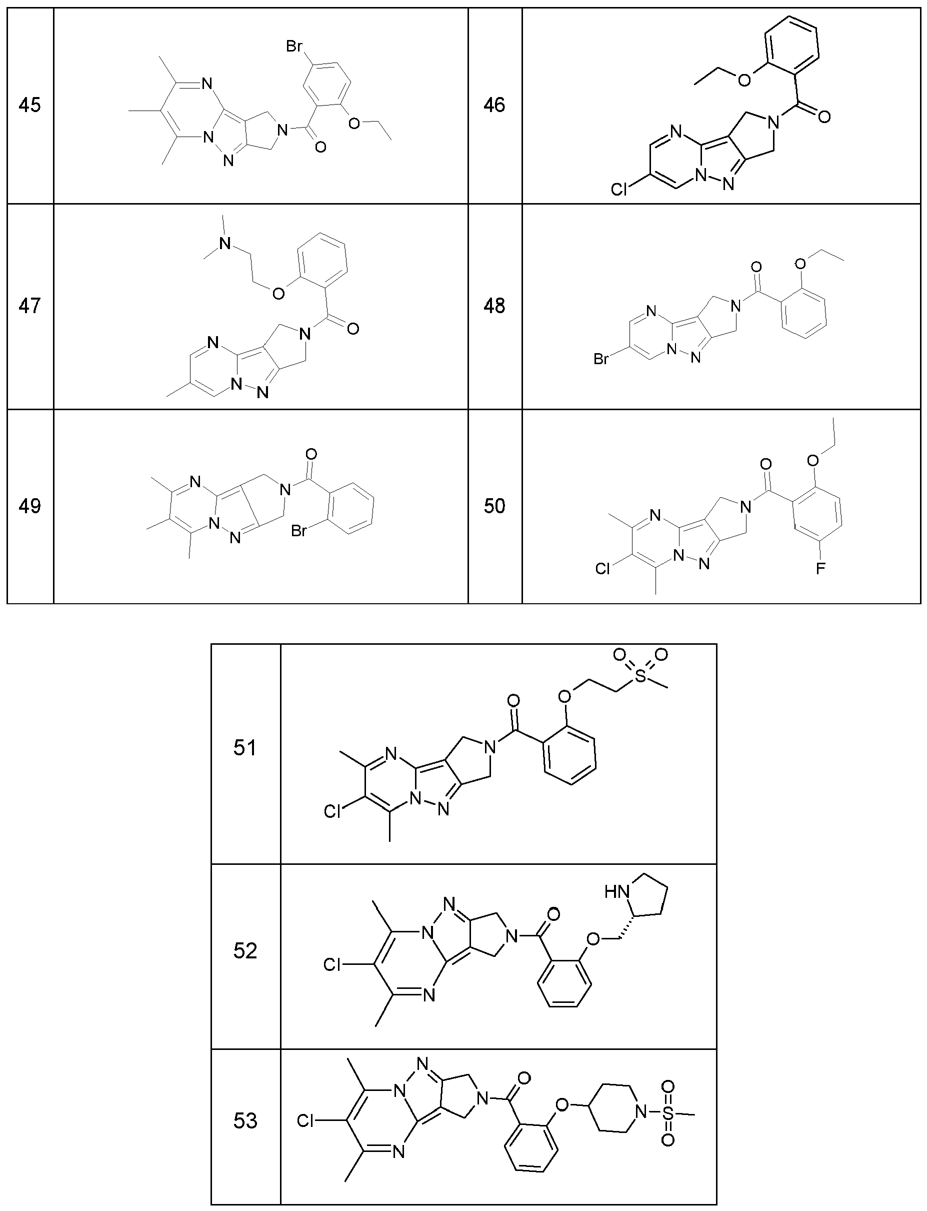

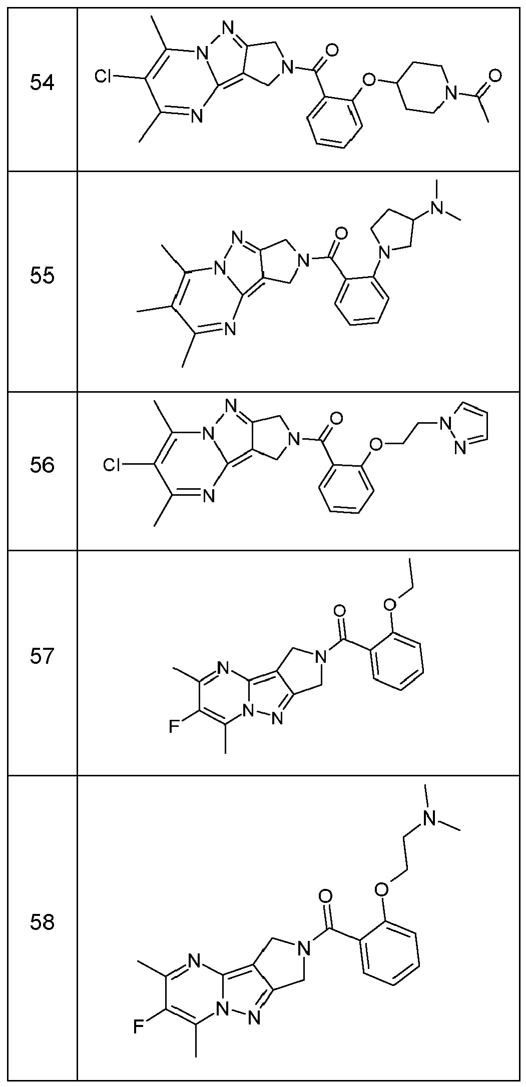

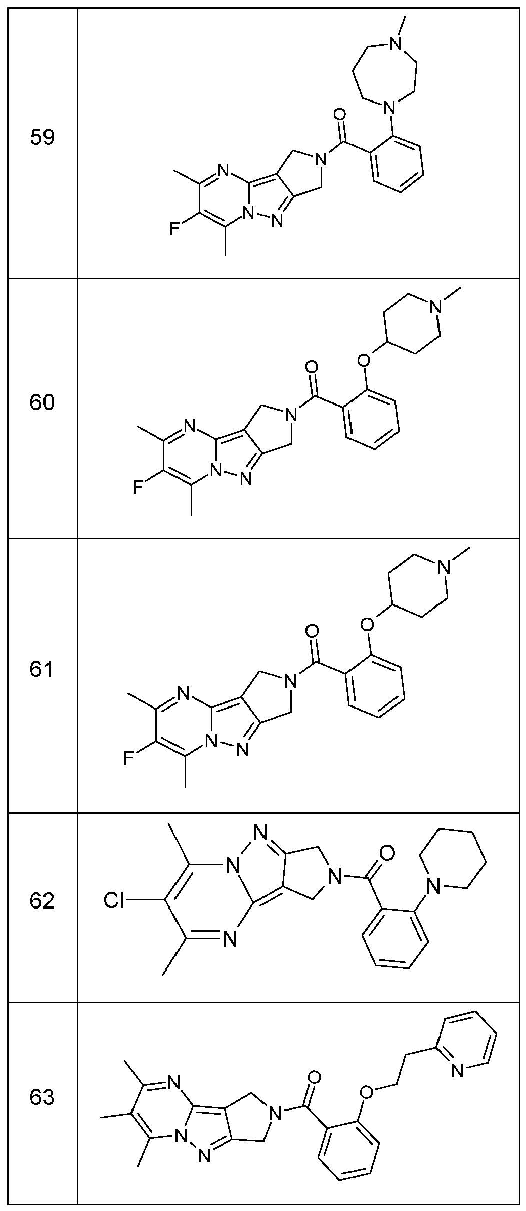

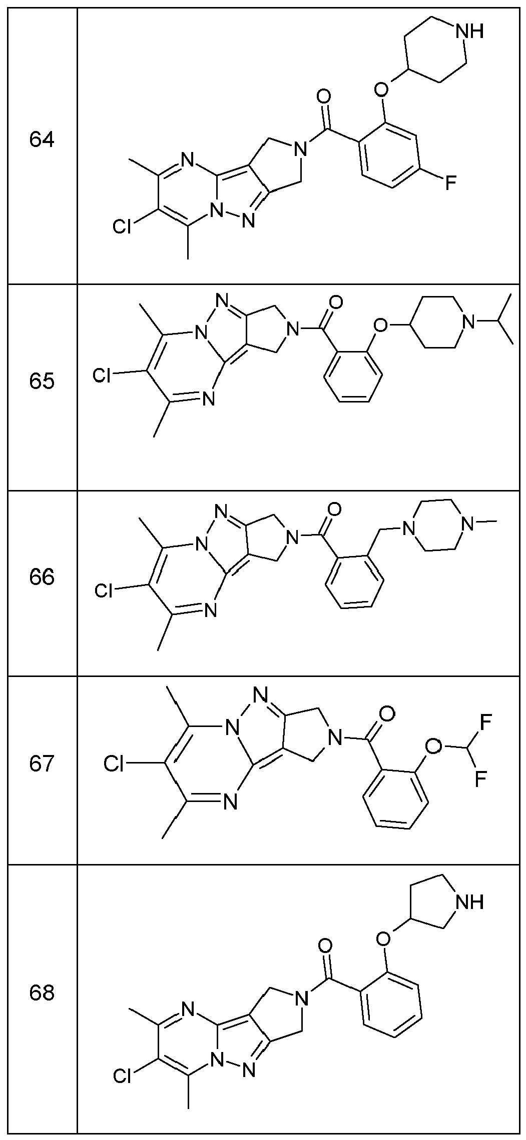

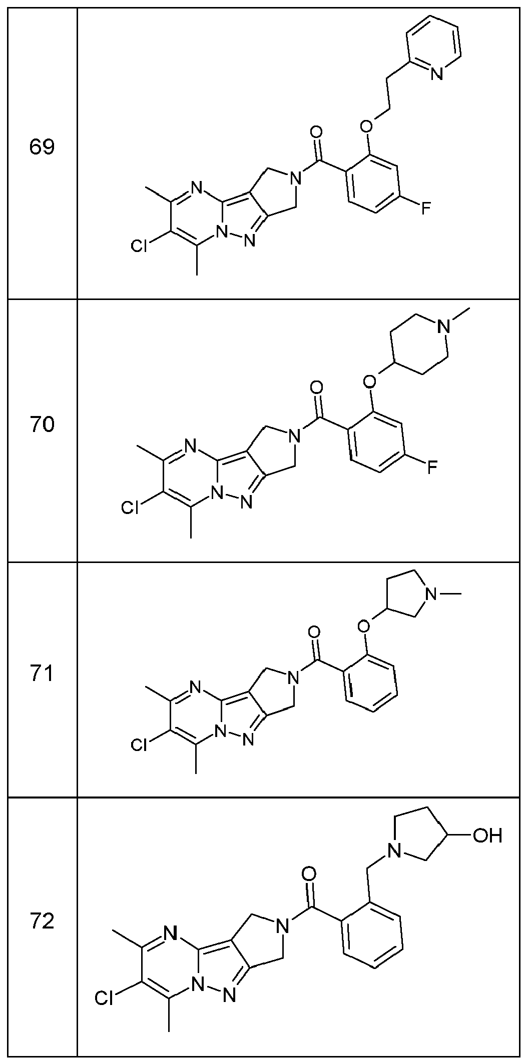

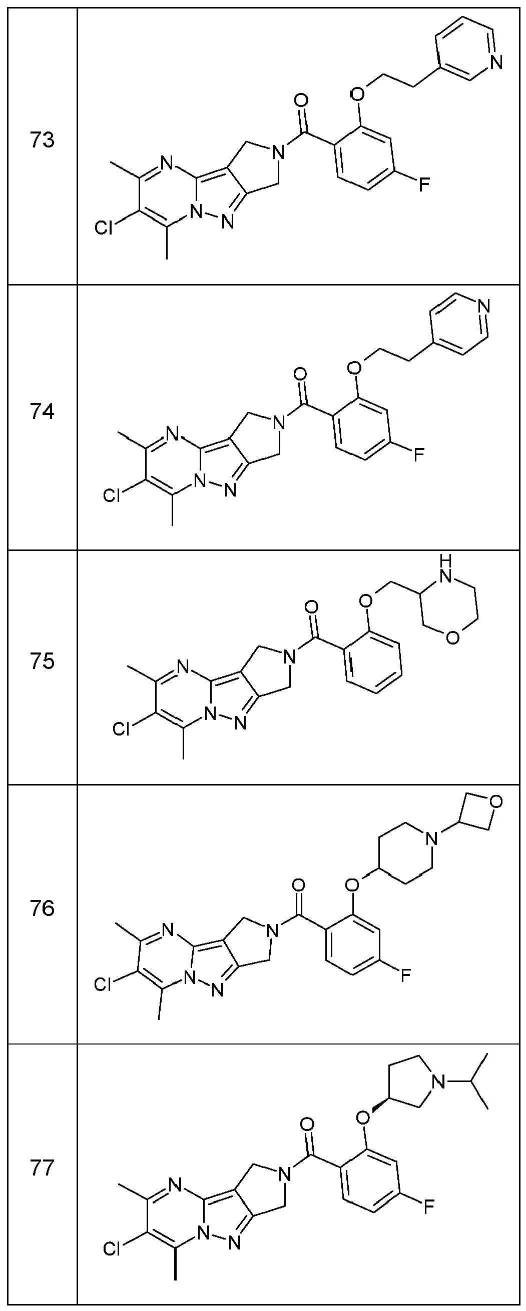

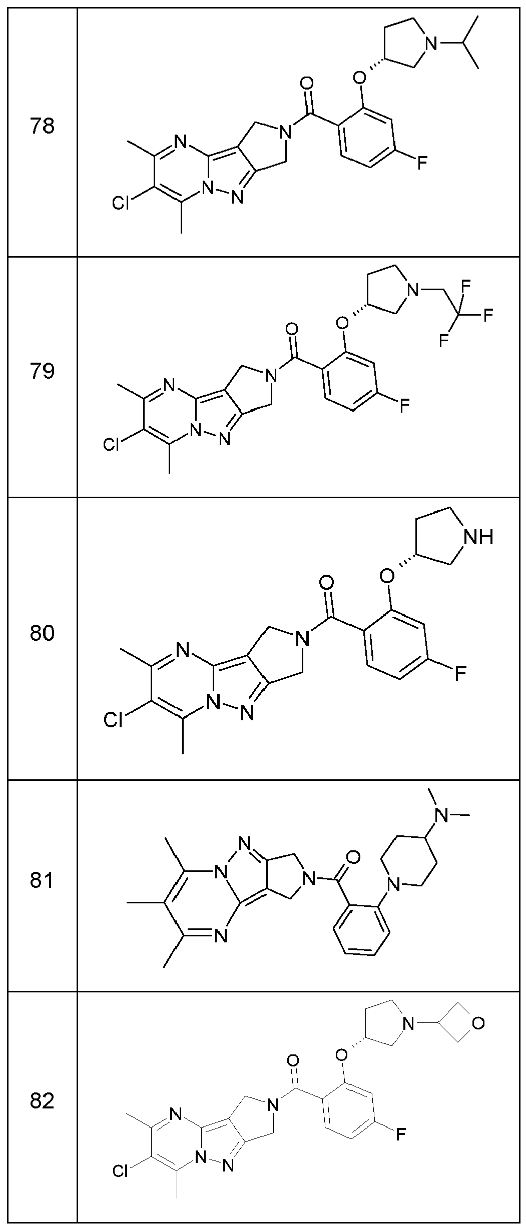

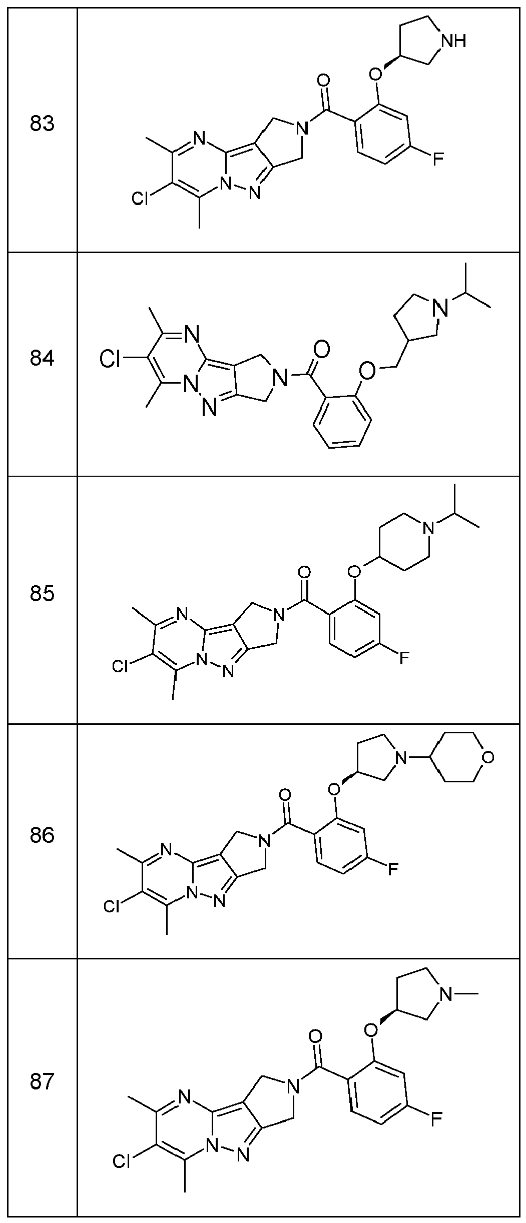

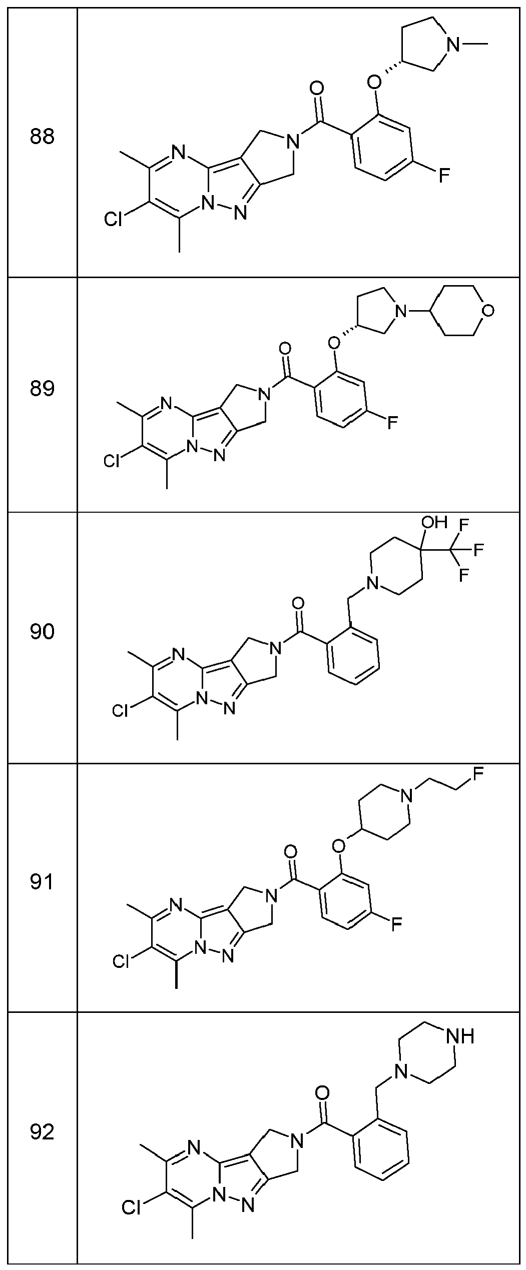

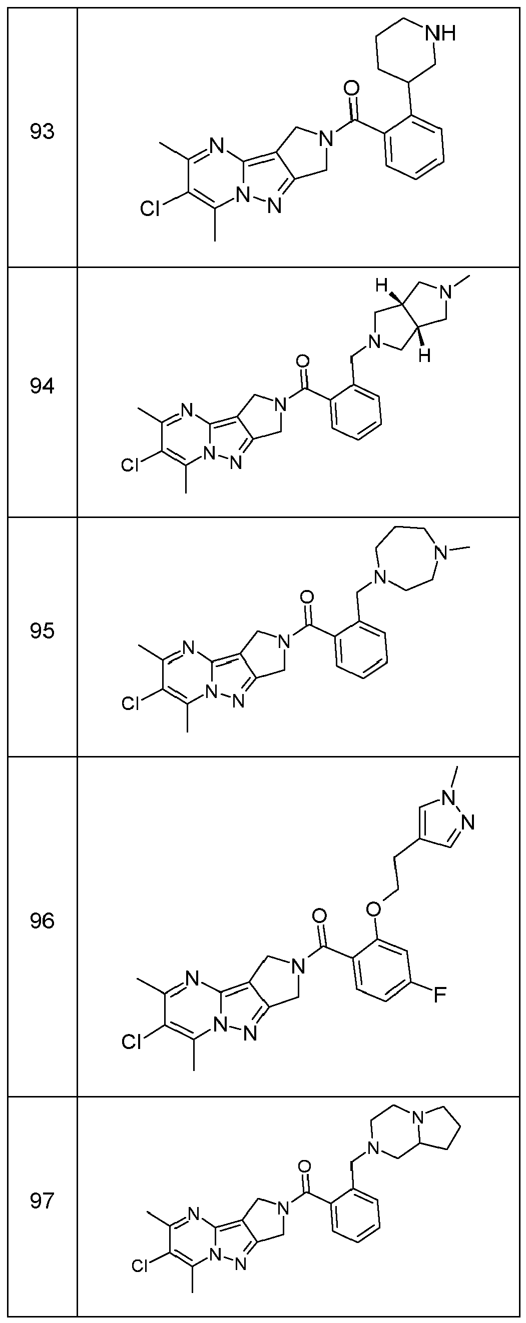

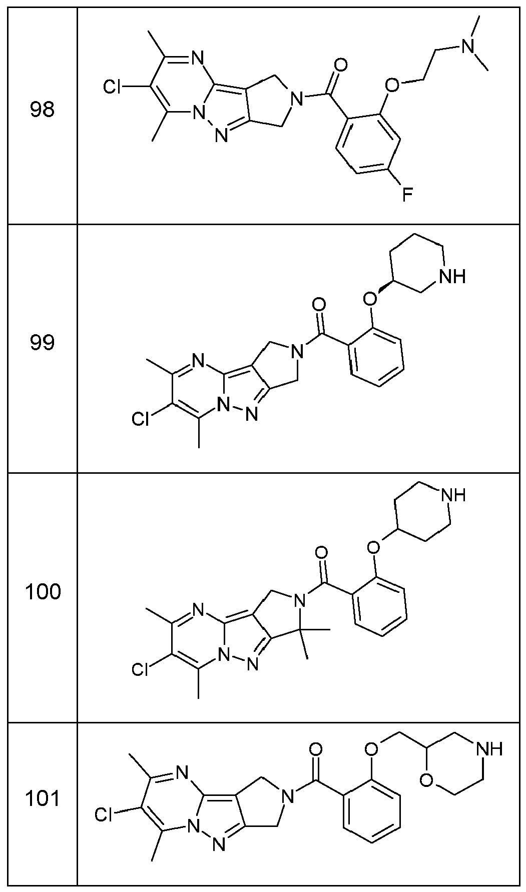

The following abbreviations refer to the abbreviations used below:
ACN (acetonitrile), AcOH (acetic acid), aq. (aqueous), DCC (dicyclohexylcarbodiimide), DCM (dichloromethane), DIC (diisopropylcarbodiimide), DIEA (di-isopropyl ethylamine), DMSO (dimethyl sulfoxide), DMF (N,N-dimethylformamide), EA (ethyl acetate), EDC (1-ethyl-3-(3-dimethylaminopropyl)carbodiimide hydrochloride), eq. (equivalent), EtOH (ethanol), g (gram), HATU (2-(1H-7-azabenzotriazol-1-yl)--1,1,3,3-tetramethyl uronium hexafluorophosphate methanaminium), cHex (cyclohexane), HPLC (high performance liquid chromatography), LG (leaving group), MeOH (methanol), MHz (Megahertz), MIBK (methyl isobutyl ketone), min (minute), mL (milliliter), mmol (millimole), MS (mass spectrometry), MTBE (tert-butyl methyl ether), MW (microwave), NMR (nuclear magnetic resonance), ppm (part per million), sat. (saturated), SFC (supercritical fluid chromatography), T3P (2,4,6-Tripropyl-[1,3,5,2,4,6]trioxatriphosphinane 2,4,6-trioxide), TEA (triethylamine), TFA (trifluoroacetic acid), THF (tetrahydrofurane), UV (ultraviolet).

In general, the compounds according to Formula (I) and related formulae of this invention can be prepared from readily available starting materials. If such starting materials are not commercially available, they may be prepared by standard synthetic techniques. In general, the synthesis pathways for any individual compound of Formula (I) and related formulae will depend on the specific substituent of each molecule, such factors being appreciated by those of ordinary skilled in the art. The following general methods and procedures described hereinafter in the examples may be employed to prepare compounds of Formula (I) and related formulae. Reaction conditions depicted in the following schemes, such as temperatures, solvents, or co-reagents, are given as examples only and are not restrictive. It will be appreciated that where typical or preferred experimental conditions (i.e. reaction temperatures, time, moles of reagents, solvents etc.) are given, other experimental conditions can also be used unless otherwise stated. Optimum reaction conditions may vary with the particular reactants or solvents used, but such conditions can be determined by the person skilled in the art, using routine optimisation procedures. For all the protection and deprotection methods, see Philip J. Kocienski, in "Protecting Groups", Georg Thieme Verlag Stuttgart, New York, 1994 and, Theodora W. Greene and Peter G. M. Wuts in "Protective Groups in Organic Synthesis", Wiley Interscience, 3rd Edition 1999.

A "leaving group" denotes a chemical moiety which can be removed or replaced by another chemical group.

Throughout the specification, the term leaving group preferably denotes Cl, Br, I or a reactively modified OH group, such as, for example, an activated ester, an imidazolide or alkylsulfonyloxy having 1 to 6 carbon atoms (preferably methylsulfonyloxy or trifluoromethylsulfonyloxy) or arylsulfonyloxy having 6 to 10 carbon atoms (preferably phenyl- or p tolylsulfonyloxy).

Radicals of this type for activation of the carboxyl group in typical acylation reactions are described in the literature (for example in the standard works, such as Houben-Weyl, Methoden der organischen Chemie [Methods of Organic Chemistry], Georg-Thieme-Verlag, Stuttgart).

Activated esters are advantageously formed in situ, for example through addition of HOBt or N hydroxysuccinimide.

Depending on the nature of R¹, R², R³, R⁴, R^{a}, R^{b}, and Q, different synthetic strategies may be selected for the synthesis of compounds of Formula (I). In the process illustrated in the following schemes, R¹, R², R³, R⁴, R^{a}, R^{b}, and Q are as above defined in the description unless otherwise mentioned.

Generally, tetraaza-cyclopenta[a]indenyl compounds of Formula (I) wherein R¹, R², R³, R⁴, R^{a}, R^{b}, and Q are defined as above can be prepared following the synthetic pathway described in the general scheme 1.

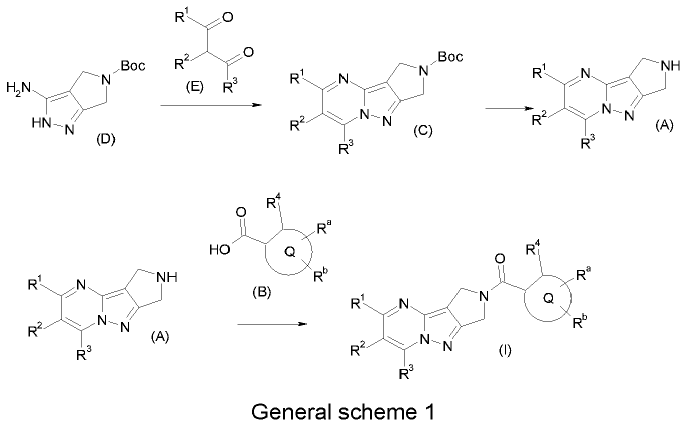

According to a preferred synthetic pathway, compounds of Formula (I) wherein R¹, R², R³, R⁴, R^{a}, R^{b}, and Q are as above defined, may be prepared by reaction between an amine of Formula (A) and a carboxylic acid of Formula (B) following usual conditions for the formation of an amide starting from a carboxylic acid and an amine by using coupling agents such as EDC, HATU, DCC, DIC or via the formation of an acid chloride or an activated ester. Preferred conditions consist in the treatment of compounds of Formula (A) wherein R¹, R² and R³ are as above defined with HATU or EDC followed by the addition of the amine of Formula (B) wherein R⁴, R^{a}, R^{b}, and Q are as above defined, in the presence of a base such as TEA or DIEA in a suitable solvent such as DMF or DCM at room temperature.

Compounds of Formula (A) wherein R¹, R² and R³ are as above defined may be prepared from the corresponding Boc protected amines of Formula (C), by treatment with an acid such TFA in DCM or HCl in dioxane or HCl in AcOH.

Compounds of Formula (C) wherein R¹, R² and R³ are as above defined may be prepared by reacting compounds of Formula (D) and compounds of Formula (E) in a suitable solvent such as AcOH at a temperature ranging from 25°C to 75°C, for 30 minutes to 48 hours.

Compound of Formula (D) may be prepared as described in Bioorg. Med. Chem. Lett. 2010, 20(14), 4273-4278.

Alternatively, compounds of general Formula (I) wherein R¹, R², R³, R^{a}, R^{b}, and Q are as above defined and R⁴ is OG, may be prepared as depicted in general scheme 2.

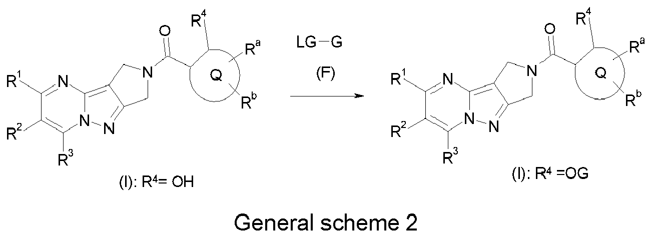

Compounds of Formula (I) wherein R¹, R², R³, R^{a}, R^{b}, and Q are as above defined and R⁴ is OG, may be prepared by reaction between a compound of Formula (I) wherein R⁴ is OH and a compound of Formula (F) wherein LG is a leaving group, preferably selected from Hal or an activated ester, in the presence of a base such as K₂CO₃, Cs₂CO₃, Na₂CO₃, NaH, in a solvent such as DMF, DMA, THF, 1,4-dioxane, acetone, ACN at a temperature ranging from 20°C to 200°C for few minutes to several hours. Preferred conditions consist in the treatment compound of Formula (I) wherein R⁴ is OH by a compound of Formula (F) in the presence of K₂CO₃, in a solvent such as DMF at a temperature of about 150°C using microwave heating for 10 minutes to 1 hour.

Compounds of Formula (B) wherein R^{a}, R^{b}, and Q are as above defined and R⁴ is OG may be prepared according to general scheme 3.

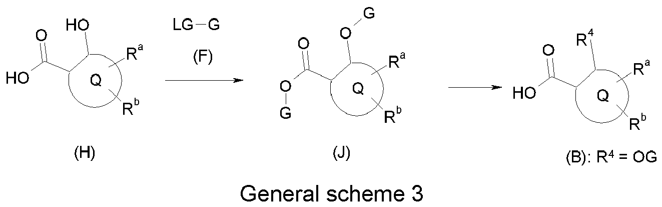

Compounds of Formula (B) wherein R⁴ is OG may be prepared by saponification of esters of Formula (J) wherein R^{a}, R^{b}, G and Q are as above defined, using LiOH, NaOH or KOH in water, water/THF, water/THF/ethanol orwater/1,4-dioxane, at temperatures between 0 and 100°C. Furthermore, ester can be hydrolyzed, for example, using acetic acid, TFA or HCl.

Compounds of Formula (J) wherein R^{a}, R^{b}, G and Q are as above defined may be prepared by reacting compounds of Formula (H) with compounds of Formula (F) in the presence of a base such as K₂CO₃, Cs₂CO₃, Na₂CO₃, NaH, in a solvent such as DMF, DMA, THF, 1,4-dioxane, acetone, ACN or mixtures thereof at a temperature ranging from 20°C to 200°C for few minutes to several hours.

Compounds of this invention can be isolated in association with solvent molecules by crystallization from an appropriate solvent or by evaporation of an appropriate solvent. The pharmaceutically acceptable anionic salts of the compounds of Formula (I), which contain a basic center, may be prepared in a conventional manner. For example, a solution of the free base may be treated with a suitable acid, either neat or in a suitable solution, and the resulting salt isolated either by filtration or by evaporation under vacuum of the reaction solvent.

The pharmaceutically acceptable cationic salts of the compounds of Formula (I), which contain an acidic center, may be prepared in a conventional manner. For example, a solution of the free acid may be treated with a suitable base, either neat or in a suitable solution, and the resulting salt isolated either by filtration or by evaporation under vacuum of the reaction solvent. In some cases, salts can be prepared by mixing a solution of the acid with a solution of an alkali or earth alkali salt (such as sodium ethylhexanoate, magnesium oleate), employing a solvent in which the desired alkali or earth alkali salt of the compounds of formula (I) precipitates, or can be otherwise isolated by concentration and addition of a non-solvent.

Both types of salts may be formed or interconverted using ion-exchange resin techniques.

Depending on the conditions used, the reaction times are generally between a few minutes and 14 days. The reaction temperature is between about -30°C and about 140°C, normally between -10°C and 90°C, in particular between about 0°C and 70°C. Compounds of the formula (I) and related formulae can furthermore be obtained by liberating compounds of the formula (I) from one of their functional derivatives by treatment with a solvolysing or hydrogenolysing agent.

Preferred starting materials for the solvolysis or hydrogenolysis are those which conform to the formula I and related formulae, but contain corresponding protected amino and/or hydroxyl groups instead of one or more free amino and/or hydroxyl groups, preferably those which carry an amino-protecting group instead of an H atom bonded to an N atom, in particular those which carry an R*-N group, in which R* denotes an amino-protecting group, instead of an HN group, and/or those which carry a hydroxyl-protecting group instead of the H atom of a hydroxyl group, for example those which conform to the formula I, but carry a -COOR** group, in which R** denotes a hydroxyl-protecting group, instead of a -COOH group.

It is also possible for a plurality of - identical or different - protected amino and/or hydroxyl groups to be present in the molecule of the starting material. If the protecting groups present are different from one another, they can in many cases be cleaved off selectively.

The term "amino-protecting group" is known in general terms and relates to groups which are suitable for protecting (blocking) an amino group against chemical reactions, but which are easy to remove after the desired chemical reaction has been carried out elsewhere in the molecule. Typical of such groups are, in particular, unsubstituted or substituted acyl, aryl, aralkoxymethyl or aralkyl groups. Since the amino-protecting groups are removed after the desired reaction (or reaction sequence), their type and size are furthermore not crucial; however, preference is given to those having 1-20, in particular 1-8, carbon atoms. The term "acyl group" is to be understood in the broadest sense in connection with the present process. It includes acyl groups derived from aliphatic, araliphatic, aromatic or heterocyclic carboxylic acids or sulfonic acids, and, in particular, alkoxy¬carbonyl, aryloxycarbonyl and especially aralkoxycarbonyl groups. Examples of such acyl groups are alkanoyl, such as acetyl, propionyl and butyryl; aralkanoyl, such as phenylacetyl; aroyl, such as benzoyl and tolyl; aryloxyalkanoyl, such as POA; alkoxycarbonyl, such as methoxy¬carbonyl, ethoxycarbonyl, 2,2,2-trichloroethoxycarbonyl, BOC (tert-butoxycarbonyl) and 2-iodoethoxycarbonyl; aralkoxycarbonyl, such as CBZ ("carbobenzoxy"), 4-methoxybenzyloxycarbonyl and FMOC; and aryl¬sulfonyl, such as Mtr. Preferred amino-protecting groups are BOC and Mtr, further¬more CBZ, Fmoc, benzyl and acetyl.

The term "hydroxyl-protecting group" is likewise known in general terms and relates to groups which are suitable for protecting a hydroxyl group against chemical reactions, but are easy to remove after the desired chemical reaction has been carried out elsewhere in the molecule. Typical of such groups are the above-mentioned unsubstituted or substituted aryl, aralkyl or acyl groups, furthermore also alkyl groups. The nature and size of the hydroxyl-protecting groups are not crucial since they are removed again after the desired chemical reaction or reaction sequence; preference is given to groups having 1-20, in particular 1-10, carbon atoms. Examples of hydroxyl-protecting groups are, inter alia, benzyl, 4-methoxybenzyl, p-nitrobenzoyl, p-toluenesulfonyl, tert-butyl and acetyl, where benzyl and tert-butyl are particularly preferred.

The compounds of the formula I and related formulae are liberated from their functional derivatives - depending on the protecting group used - for example strong inorganic acids, such as hydrochloric acid, perchloric acid or sulfuric acid, strong organic carboxylic acids, such as trichloroacetic acid, TFA or sulfonic acids, such as benzene- or p-toluenesulfonic acid. The presence of an additional inert solvent is possible, but is not always necessary. Suitable inert solvents are preferably organic, for example carboxylic acids, such as acetic acid, ethers, such as tetrahydrofuran or dioxane, amides, such as DMF, halogenated hydrocarbons, such as dichloromethane, furthermore also alcohols, such as methanol, ethanol or isopropanol, and water. Mixtures of the above-mentioned solvents are furthermore suitable. TFA is preferably used in excess without addition of a further solvent, and perchloric acid is preferably used in the form of a mixture of acetic acid and 70% perchloric acid in the ratio 9:1. The reaction temperatures for the cleavage are advantageously between about 0 and about 50°C, preferably between 15 and 30°C (room temperature).

The BOC, OtBut and Mtr groups can, for example, preferably be cleaved off using TFA in dichloromethane or using approximately 3 to 5N HCl in dioxane at 15-30°C, and the FMOC group can be cleaved off using an approximately 5 to 50% solution of dimethylamine, diethylamine or piperidine in DMF at 15-30°C.

Protecting groups which can be removed hydrogenolytically (for example CBZ, benzyl or the liberation of the amidino group from the oxadiazole derivative thereof) can be cleaved off, for example, by treatment with hydrogen in the presence of a catalyst (for example a noble-metal catalyst, such as palladium, advantageously on a support, such as carbon). Suitable solvents here are those indicated above, in particular, for example, alcohols, such as methanol or ethanol, or amides, such as DMF. The hydrogenolysis is generally carried out at temperatures between about 0 and 100°C and pressures between about 1 and 200 bar, preferably at 20-30°C and 1-10 bar. Hydrogenolysis of the CBZ group succeeds well, for example, on 5 to 10% Pd/C in methanol or using ammonium formate (instead of hydrogen) on Pd/C in methanol/DMF at 20-30°C. Examples of suitable inert solvents are hydrocarbons, such as hexane, petroleum ether, benzene, toluene or xylene; chlorinated hydrocarbons, such as trichloroethylene, 1,2-dichloroethane, tetrachloromethane, trifluoromethylbenzene, chloroform or dichloromethane; alcohols, such as methanol, ethanol, isopropanol, n-propanol, n-butanol or tert-butanol; ethers, such as diethyl ether, diisopropyl ether, tetrahydrofuran (THF) or dioxane; glycol ethers, such as ethylene glycol monomethyl or monoethyl ether or ethylene glycol dimethyl ether (diglyme); ketones, such as acetone or butanone; amides, such as acetamide, dimethylacetamide, N-methylpyrrolidone (NMP) or dimethyl¬formamide (DMF); nitriles, such as acetonitrile; sulfoxides, such as dimethyl sulfoxide (DMSO); carbon disulfide; carboxylic acids, such as formic acid or acetic acid; nitro compounds, such as nitromethane or nitrobenzene; esters, such as ethyl acetate, or mixtures of the said solvents.

Esters can be hydrolysed, for example, using HCl, H₂SO₄, or using LiOH, NaOH or KOH in water, water/THF, water/THF/ethanol or water/dioxane, at temperatures between 0 and 100°C.

Free amino groups can furthermore be acylated in a conventional manner using an acyl chloride or anhydride or alkylated using an unsubstituted or substituted alkyl halide, advantageously in an inert solvent, such as dichloromethane or THF and/or in the presence of a base, such as triethylamine or pyridine, at temperatures between -60°C and +30°C.

The formula (I) and related formulae also encompasses the optically active forms (stereoisomers), the enantiomers, the racemates, the diastereomers and the hydrates and solvates of these compounds. The term "solvates of the compounds" is taken to mean adductions of inert solvent molecules onto the compounds which form owing to their mutual attractive force. Solvates are, for example, mono- or dihydrates or alcoholates.

The term "pharmaceutically usable derivatives" is taken to mean, for example, the salts of the compounds of the formula I and so-called pro¬drug compounds.

The term "prodrug derivatives" is taken to mean compounds of the formula I which have been modified with, for example, alkyl or acyl groups, sugars or oligopeptides and which are rapidly cleaved in the organism to form the active compounds. Preferably "prodrug", as of the compounds of formula I, refers to derivative compounds that are rapidly transformed in vivo to yield the parent compound of the formula I, as for example by hydrolysis in blood. T. Higuchi and V. Stella provide a thorough discussion of the prodrug concept in "Pro-drugs as Novel Delivery Systems", Vol 14 of the A.C.S. Symposium Series, American Chemical Society (1975). Examples of esters useful as prodrugs for compounds containing carboxyl groups can be found on pages 14-21 of "Bioreversible Carriers in Drug Design: Theory and Application", edited by E. B. Roche, Pergamon Press: New York (1987). It is intended that these references, and any others cited throughout this specification, are incorporated herein by reference.

These also include biodegradable polymer derivatives of the compounds according to the invention, as described, for example, in Int. J. Pharm. 115, 61-67 (1995).

The formula (I) and related formulae also encompasses mixtures of the compounds of the formula I, for example mixtures of two diastereomers, for example in the ratio 1:1, 1:2, 1:3, 1:4, 1:5, 1:10, 1:100 or 1:1000.

These are particularly preferably mixtures of stereoisomeric compounds. Pharmaceutical formulations can be administered in the form of dosage units, which comprise a predetermined amount of active ingredient per dosage unit. Such a unit can comprise, for example, 0.5 mg to 1 g, preferably 1 mg to 700 mg, particularly preferably 5 mg to 100 mg, of a compound according to the invention, depending on the disease condition treated, the method of administration and the age, weight and condition of the patient, or pharmaceutical formulations can be administered in the form of dosage units which comprise a predetermined amount of active ingredient per dosage unit. Preferred dosage unit formulations are those which comprise a daily dose or part-dose, as indicated above, or a corresponding fraction thereof of an active ingredient. Furthermore, pharmaceutical formulations of this type can be prepared using a process, which is generally known in the pharmaceutical art.

Pharmaceutical formulations can be adapted for administration via any desired suitable method, for example by oral (including buccal or sublingual), rectal, nasal, topical (including buccal, sublingual or transdermal), vaginal or parenteral (including subcutaneous, intramuscular, intravenous or intradermal) methods. Such formulations can be prepared using all processes known in the pharmaceutical art by, for example, combining the active ingredient with the excipient(s) or adjuvant(s).

Pharmaceutical formulations adapted for oral administration can be administered as separate units, such as, for example, capsules or tablets; powders or granules; solutions or suspensions in aqueous or non-aqueous liquids; edible foams or foam foods; or oil-in-water liquid emulsions or water-in-oil liquid emulsions.

Thus, for example, in the case of oral administration in the form of a tablet or capsule, the active-ingredient component can be combined with an oral, non-toxic and pharmaceutically acceptable inert excipient, such as, for example, ethanol, glycerol, water and the like. Powders are prepared by comminuting the compound to a suitable fine size and mixing it with a pharmaceutical excipient comminuted in a similar manner, such as, for example, an edible carbohydrate, such as, for example, starch or mannitol. A flavour, preservative, dispersant and dye may likewise be present.

Capsules are produced by preparing a powder mixture as described above and filling shaped gelatine shells therewith. Glidants and lubricants, such as, for example, highly disperse silicic acid, talc, magnesium stearate, calcium stearate or polyethylene glycol in solid form, can be added to the powder mixture before the filling operation. A disintegrant or solubiliser, such as, for example, agar-agar, calcium carbonate or sodium carbonate, may likewise be added in order to improve the availability of the medica-ment after the capsule has been taken.

In addition, if desired or necessary, suitable binders, lubricants and disintegrants as well as dyes can likewise be incorporated into the mixture. Suitable binders include starch, gelatine, natural sugars, such as, for example, glucose or beta-lactose, sweeteners made from maize, natural and synthetic rubber, such as, for example, acacia, tragacanth or sodium alginate, carboxymethylcellulose, polyethylene glycol, waxes, and the like. The lubricants used in these dosage forms include sodium oleate, sodium stearate, magnesium stearate, sodium benzoate, sodium acetate, sodium chloride and the like. The disintegrants include, without being restricted thereto, starch, methylcellulose, agar, bentonite, xanthan gum and the like. The tablets are formulated by, for example, preparing a powder mixture, granulating or dry-pressing the mixture, adding a lubricant and a disintegrant and pressing the entire mixture to give tablets. A powder mixture is prepared by mixing the compound comminuted in a suitable manner with a diluent or a base, as described above, and optionally with a binder, such as, for example, carboxymethylcellulose, an alginate, gelatine or polyvinyl-pyrrolidone, a dissolution retardant, such as, for example, paraffin, an absorption accelerator, such as, for example, a quaternary salt, and/or an absorbant, such as, for example, bentonite, kaolin or dicalcium phosphate. The powder mixture can be granulated by wetting it with a binder, such as, for example, syrup, starch paste, acadia mucilage or solutions of cellulose or polymer materials and pressing it through a sieve. As an alternative to granulation, the powder mixture can be run through a tableting machine, giving lumps of non-uniform shape which are broken up to form granules. The granules can be lubricated by addition of stearic acid, a stearate salt, talc or mineral oil in order to prevent sticking to the tablet casting moulds. The lubricated mixture is then pressed to give tablets. The active ingredients can also be combined with a free-flowing inert excipient and then pressed directly to give tablets without carrying out the granulation or dry-pressing steps. A transparent or opaque protective layer consisting of a shellac sealing layer, a layer of sugar or polymer material and a gloss layer of wax may be present. Dyes can be added to these coatings in order to be able to differentiate between different dosage units.

Oral liquids, such as, for example, solution, syrups and elixirs, can be prepared in the form of dosage units so that a given quantity comprises a pre-specified amount of the compounds. Syrups can be prepared by dissolving the compounds in an aqueous solution with a suitable flavour, while elixirs are prepared using a non-toxic alcoholic vehicle. Suspensions can be for-mulated by dispersion of the compounds in a non-toxic vehicle. Solubilisers and emulsifiers, such as, for example, ethoxylated isostearyl alcohols and polyoxyethylene sorbitol ethers, preservatives, flavour additives, such as, for example, peppermint oil or natural sweeteners or saccharin, or other artificial sweeteners and the like, can likewise be added.

The dosage unit formulations for oral administration can, if desired, be encapsulated in microcapsules. The formulation can also be prepared in such a way that the release is extended or retarded, such as, for example, by coating or embedding of particulate material in polymers, wax and the like.

The compounds of the formula (I), and related formulae and salts, solvates and physiologically functional derivatives thereof and the other active ingredients can also be administered in the form of liposome delivery systems, such as, for example, small unilamellar vesicles, large unilamellar vesicles and multilamellar vesicles. Liposomes can be formed from various phospholipids, such as, for example, cholesterol, stearylamine or phosphatidylcholines.

The compounds of the formula (I), and related formulae and the salts, solvates and physiologically functional derivatives thereof and the other active ingredients can also be delivered using monoclonal antibodies as individual carriers to which the compound molecules are coupled. The compounds can also be coupled to soluble polymers as targeted medicament carriers. Such polymers may encompass polyvinylpyrrolidone, pyran copolymer, polyhydroxypropyl-methacrylamidophenol, polyhydroxyethylaspartamido-phenol or polyethylene oxide polylysine, substituted by palmitoyl radicals. The compounds may furthermore be coupled to a class of biodegradable polymers which are suitable for achieving controlled release of a medicament, for example polylactic acid, poly-epsilon-caprolactone, polyhydroxybutyric acid, poly-orthoesters, polyacetals, polydihydroxypyrans, polycyanoacrylates and crosslinked or amphipathic block copolymers of hydrogels.

Pharmaceutical formulations adapted for transdermal administration can be administered as independent plasters for extended, close contact with the epidermis of the recipient. Thus, for example, the active ingredient can be delivered from the plaster by iontophoresis, as described in general terms in Pharmaceutical Research, 3(6), 318 (1986).

Pharmaceutical compounds adapted for topical administration can be formulated as ointments, creams, suspensions, lotions, powders, solutions, pastes, gels, sprays, aerosols or oils.

For the treatment of the eye or other external tissue, for example mouth and skin, the formulations are preferably applied as topical ointment or cream. In the case of formulation to give an ointment, the active ingredient can be employed either with a paraffinic or a water-miscible cream base. Alternatively, the active ingredient can be formulated to give a cream with an oil-in-water cream base or a water-in-oil base. Pharmaceutical formulations adapted for topical application to the eye include eye drops, in which the active ingredient is dissolved or sus-pended in a suitable carrier, in particular an aqueous solvent.

Pharmaceutical formulations adapted for topical application in the mouth encompass lozenges, pastilles and mouthwashes.

Pharmaceutical formulations adapted for rectal administration can be administered in the form of suppositories or enemas.

Pharmaceutical formulations adapted for nasal administration in which the carrier substance is a solid comprise a coarse powder having a particle size, for example, in the range 20-500 microns, which is administered in the manner in which snuff is taken, i.e. by rapid inhalation via the nasal passages from a container containing the powder held close to the nose. Suitable formulations for administration as nasal spray or nose drops with a liquid as carrier substance encompass active-ingredient solutions in water or oil. Pharmaceutical formulations adapted for administration by inhalation encompass finely particulate dusts or mists, which can be generated by various types of pressurised dispensers with aerosols, nebulisers or insuf-flators.

Pharmaceutical formulations adapted for vaginal administration can be administered as pessaries, tampons, creams, gels, pastes, foams or spray formulations.

Pharmaceutical formulations adapted for parenteral administration include aqueous and non-aqueous sterile injection solutions comprising antioxidants, buffers, bacteriostatics and solutes, by means of which the formulation is rendered isotonic with the blood of the recipient to be treated; and aqueous and non-aqueous sterile suspensions, which may comprise suspension media and thickeners. The formulations can be administered in single-dose or multidose containers, for example sealed ampoules and vials, and stored in freeze-dried (lyophilised) state, so that only the addition of the sterile carrier liquid, for example water for injection purposes, immediately before use is necessary. injection solutions and suspensions prepared in accordance with the recipe can be prepared from sterile powders, granules and tablets.

It goes without saying that, in addition to the above particularly mentioned constituents, the formulations may also comprise other agents usual in the art with respect to the particular type of formulation; thus, for example, formulations which are suitable for oral administration may comprise flavours.

A therapeutically effective amount of a compound of the formula (I), and related formulae and of the other active ingredient depends on a number of factors, including, for example, the age and weight of the animal, the precise disease condition which requires treatment, and its severity, the nature of the formulation and the method of administration, and is ultimately determined by the treating doctor or vet. However, an effective amount of a compound is generally in the range from 0.1 to 100 mg/kg of body weight of the recipient (mammal) per day and particularly typically in the range from 1 to 10 mg/kg of body weight per day. Thus, the actual amount per day for an adult mammal weighing 70 kg is usually between 70 and 700 mg, where this amount can be administered as an individual dose per day or usually in a series of part-doses (such as, for example, two, three, four, five or six) per day, so that the total daily dose is the same. An effective amount of a salt or solvate or of a physiologically functional derivative thereof can be determined as the fraction of the effective amount of the compound per se.

The present invention furthermore relates to a method for treating a subject suffering from a M1 related disorder, comprising administering to said subject an effective amount of a compound of formula (I) and related formulae. The present invention preferably relates to a method, wherein the M1 associated disorder is Alzheimer's disease, Parkinson disease, schizophrenia, movement disorders, memory disorders, chronic neuropathic pain, sleep disorders, epilepsy. Nociception disorder, dementia, hallucination, delusion, paranoia.

### EXPERIMENTAL PART

The compounds of invention have been named according to the standards used in the program AutoNom (v1.0.1.1).

The compounds according to formula (I) can be prepared from readily available starting materials by several synthetic approaches, using both solution-phase and solid-phase chemistry protocols or mixed solution and solid phase protocols. Examples of synthetic pathways are described below in the examples.

The commercially available starting materials used in the following experimental description were purchased from Aldrich, Sigma, ACROS or ABCR unless otherwise reported.

**¹H NMR analyses** were carried out using BRUKER NMR, model DPX-300 MHz FT-NMR. Residual signal of deuterated solvent was used as internal reference. Chemical shifts (δ) are reported in ppm in relative to the residual solvent signal (δ = 2.50 for ¹H NMR in DMSO-d₆, and 7.26 in CDCl₃). s (singlet), d (doublet), t (triplet), q (quadruplet), br (broad). Some compounds in the experimental part exist as mixture of rotamers in different ratios as described in the ¹H NMR descriptions.

**The MS data** provided in the examples described below were obtained as followed: Mass spectrum: LC/MS Waters ZMD (ESI).

**HPLC analyses were** obtained as followed using a Waters XbridgeTM C8 50 mm x 4.6 mm column at a flow of 2 mL/min; 8 min gradient H₂O:CH₃CN:TFA from 100:0:0.1 % to 0:100:0.05 % with UV detection (maxplot).

**The SFC purifications** were performed with a Prep SFC 100 UV from Thar-Waters.

**The mass directed preparative HPLC** purifications were performed with a mass directed autopurification Fractionlynx from Waters equipped with a Sunfire Prep C18 OBD column 19x100 mm 5 µm, unless otherwise reported. All purifications were performed with a gradient of ACN/H₂O or ACN/H₂O/HCOOH (0.1 %).

**The microwave chemistry** was performed on a single mode microwave reactor Emrys™ Optimiser or Initiator™ Sixty from Biotage.

### Method A (amide formation using HATU) :

HATU (1.0-1.2 eq.) was added to a solution of the carboxylic acid (1.0-1.2 eq.) and DIEA (2-4 eq.) in DMF and the reaction mixture was stirred at room temperature for 15 min to 1 hour whereupon the amine (1 eq.) was added. The resulting mixture was stirred at room temperature until completion.

### Method B (amide formation using EDC) :

EDC (1.0-1.2 eq.) was added to a solution of the carboxylic acid (1.0-1.2 eq.) and TEA (2-4 eq.) in DCM and the reaction mixture was stirred at room temperature for 15 min to 1 hour whereupon the amine (1 eq.) was added. The resulting mixture was stirred at room temperature until completion.

### Preparation of Intermediates

### Intermediate A1 : 5,6,7-trimethyl-2,3-dihydro-1H-2,4,7a,8-tetraaza-cyclopenta[a]indene hydrochloride

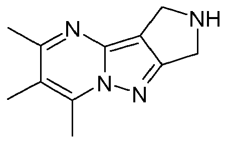

### Step 1 : 5,6,7-trimethyl-1H,3H-2,4,7a,8-tetraaza-cyclopenta[a]indene-2-carboxylic acid tert-butyl ester

A mixture of 3-amino-2,6-dihydro-4H-pyrrolo[3,4-c]pyrazole-5-carboxylic acid tert-butyl ester (2.4 g; 10.7 mmol; 1 eq.) and 3-methyl-2,4-pentanedione (1.75 mL; 15 mmol; 1.4 eq.) in AcOH (25 mL) was stirred at room temperature for 18 hours. The solvent was evaporated in vacuo and the residue partitioned between ethyl acetate and sat. aq. NaHCO₃. The organic layer was dried over sodium sulfate and concentrated in vacuo. The residue was triturated in ACN and the precipitate filtered off to afford the title compound (1.7 g, 52%) as a white solid. The mother liquor was concentrated in vacuo and the residue purified by column chromatography (DCM/EtOH, 95/5) to afford the title compound (1.5 g, 46%) as a white solid. ¹H NMR (DMSO-d₆) δ 4.59-4.53 (m, 4H), 3.34 (s, 6H), 2.27 (s, 3H), 1.47 (s, 9H). HPLC (max plot) 96.1%; Rt 3.92 min. UPLC/MS:
(MS+) 303.1 ([M+H]⁺).

### Step 2 : 5,6,7-trimethyl-2,3-dihydro-1 H-2,4,7a,8-tetraaza-cyclopentaralindene hydrochloride

A 4M solution of HCl in 1,4-dioxane (1.24 mL; 4.96 mmol; 15 eq.) was added to a solution of 5,6,7-trimethyl-1H,3H-2,4,7a,8-tetraaza-cyclopenta[a]indene-2-carboxylic acid tert-butyl ester (100 mg; 0.33 mmol; 1 eq.) in 1,4-dioxane (5 mL) and the resulting mixture was stirred at room temperature for 18 hours. The solvent was evaporated in vacuo and the residue was triturated in 1,4-dioxane. Concentration to dryness afforded the title compound (65 mg, 82%) as a white solid. ¹H NMR (DMSO-d₆) δ 10.40-10.30 (m, 1H), 4.57-4.50 (m, 4H), 2.73 (s, 3H), 2.54 (s, 3H), 2.28 (s, 3H). HPLC (max plot) 100.0%; Rt 1.26 min. UPLC/MS: (MS+) 203.1 ([M+H]⁺).

### Intermediate A2 : 6-ethyl-5,7-dimethyl-2,3-dihydro-1H-2,4,7a,8-tetraaza-cyclopenta[alindene

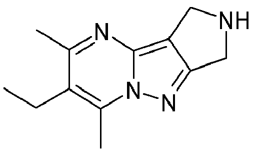

A mixture of 3-amino-2,6-dihydro-4H-pyrrolo[3,4-c]pyrazole-5-carboxylic acid tert-butyl ester (0.6 g; 2.68 mmol; 1 eq.) and 3-ethyl-2,4-pentanedione (0.36 mL; 2.68 mmol; 1 eq.) in AcOH (8 mL) was stirred at room temperature for 32 hours. Aq. 32% HCl (1.05 mL; 10.7 mmol; 4 eq.) was added and the resulting mixture stirred for a further 16 hours. After concentration in vacuo, the residue was triturated in MTBE and the precipitate filtered off and dried. The solid was taken up in ethyl acetate and washed with 1 M aq. NaOH (2x), dried over magnesium sulfate and concentrated in vacuo to afford the title compound (450 mg, 94%) as a beige solid. ¹H NMR (DMSO-d₆) δ 4.05-4.00 (m, 4H), 2.73-2.63 (m, 5H), 2.51 (s, 3H), 1.12 (t, *J* = 7.4 Hz, 3H). UPLC/MS: (MS+) 217.3
([M+H]⁺).

### Intermediate A3 : 6-chloro-5,7-dimethyl-2,3-dihydro-1H-2,4,7a,8-tetraazacyclopenta[a]indene hydrochloride

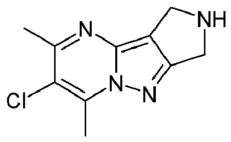

### Step 1 : 6-chloro-5,7-dimethyl-1H,3H-2,4,7a,8-tetraaza-cyclopentaralindene-2-carboxylic acid tert-butyl ester

A solution of 3-amino-2,6-dihydro-4H-pyrrolo[3,4-c]pyrazole-5-carboxylic acid tert-butyl ester (1 g; 4.46 mmol; 1 eq.) and 3-chloroacetylacetone (0.71 mL; 6.24 mmol; 1.4 eq.) in AcOH (10 mL) was stirred at room temperature for 18 hours. The reaction was poured into water (100 mL) under vigorous stirring and the precipitate filtered off, washed with water (3x) and dried to afford the title compound (1298 mg, 90%) as an off-white solid. ¹ H NMR (DMSO-d₆) δ 4.60-4.52 (m, 4H), 2.81 (br s, 3H), 2.60-2.57 (m, 3H), 1.48 (s, 9H). HPLC (max plot) 98.4%; Rt 3.96 min. UPLC/MS: (MS+) 323 ([M+H]⁺).

### Step 2 : 6-chloro-5,7-dimethyl-2,3-dihydro-1 H-2,4,7a,8-tetraaza-cyclopentaralindene hydrochloride

Aq. 32% HCl (1.14 mL; 11.62 mmol; 3 eq.) was added to a suspension of 6-chloro-5,7-dimethyl-1H,3H-2,4,7a,8-tetraaza-cyclopenta[a]indene-2-carboxylic acid tert-butyl ester (1.25 g; 3.87 mmol; 1 eq.) in AcOH (6.25 mL) and the resulting mixture was stirred at room temperature for 2 hours then poured into MTBE (40 mL). The precipitate was filtered off, washed with MTBE (3x) and dried to afford the title compound (1 g, 100%) as an off-white solid. ¹H NMR (DMSO-d₆) δ 10.55 (s, 2H), 4.57-4.52 (m, 4H), 2.83 (s, 3H), 2.61 (s, 3H). HPLC (max plot) 100.0%; Rt 1.38 min. UPLC/MS: (MS+) 222.9 ([M+H]⁺).

### Intermediate A4 : 6-chloro-2,3-dihydro-1H-2,4,7a,8-tetraaza-cyclopenta[a]indene hydrochloride

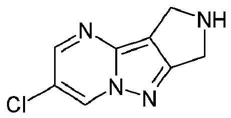

### Step 1 : 6-chloro-1H,3H-2,4,7a,8-tetraaza-cyclopenta[a]lindene-2-carboxylic acid tert-butyl ester

A mixture of 3-amino-2,6-dihydro-4H-pyrrolo[3,4-c]pyrazole-5-carboxylic acid tert-butyl ester (2 g; 8.92 mmol; 1 eq.) and 2-chloromalonaldehyde (1.04 g; 9.81 mmol; 1.1 eq.) in AcOH was stirred at room temperature for 18 hours then diluted with water (30 mL). The precipitate was filtered off and purified by column chromatography (DCM/EA, from 95/5 to 80/20) to afford the title compound (1.24 g, 47%) as a white solid. ¹H NMR (DMSO-d₆) δ 9.57 (d, *J* = 2.3 Hz, 1 H), 8.59 (d, *J* = 2.3 Hz, 1H), 4.63-4.57 (m, 4H), 1.47 (s, 9H). HPLC (max plot) 99.1%; Rt 3.75 min.

### Step 2 : 6-chloro-2,3-dihydro-1 H-2,4,7a,8-tetraaza-cyclopenta[a]indene hydrochloride

Aq. 32% HCl (1.2 mL; 12.2 mmol; 3 eq.) was added to a suspension 6-chloro-1H,3H-2,4,7a,8-tetraaza-cyclopenta[a]indene-2-carboxylic acid tert-butyl ester (1.2 g; 4.07 mmol; 1 eq.) in AcOH (6 mL) and the resulting mixture was stirred at room temperature for 2 hours then poured dropwise into ACN (40 mL) under vigorous stirring. The precipitate was filtered off, washed with ACN (2x) and dried to afford the title compound (0.85 g, 78%) as a white solid. ¹H NMR (DMSO) δ 10.49 (s, 2H), 9.65 (d, *J* = 2.3 Hz, 1H), 8.68 (d, *J* = 2.3 Hz, 1H), 4.58 (s, 4H). HPLC (max plot) 100.0%; Rt 3.76 min. UPLC/MS: (MS+) 195 ([M+H]⁺).

### Intermediate A5 : 6-methyl-2,3-dihydro-1H-2,4,7a,8-tetraaza-cyclopenta[a]indene hydrochloride

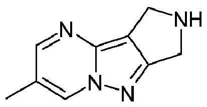

### Step 1 : 6-methyl-1H,3H-2,4,7a,8-tetraaza-cyclopentaralindene-2-carboxylic acid tert-butyl ester

A mixture of 3-amino-2,6-dihydro-4H-pyrrolo[3,4-c]pyrazole-5-carboxylic acid tert-butyl ester (5 g; 22.3 mmol; 1 eq.) and 1,1,3,3-tetraethoxy-2-methylpropane (4.32 mL; 22.3 mmol; 1 eq.) in AcOH (50 mL) was stirred at 50°C for 20 hours then diluted with water (250 mL). The precipitate was filtered off and dried to afford the title compound (3.5 g, 57%) as a pale beige solid. ¹H NMR (DMSO-d₆) δ 8.98-8.95 (m, 1 H), 8.43 (d, *J* = 2.1 Hz, 1H), 4.61-4.54 (m, 4H), 2.31 (s, 3H), 1.47 (s, 9H). HPLC (max plot) 99.7%; Rt 3.27 min. UPLC/MS: (MS+) 275.0 ([M+H]⁺).

### Step 2 : 6-methyl-2,3-dihvdro-1H-2,4,7a,8-tetraaza-cyclopentaralindene hydrochloride

Aq. 32% HCl (1.61 mL; 16.4 mmol; 3 eq.) was added to a solution of 6-methyl-1H,3H-2,4,7a,8-tetraaza-cyclopenta[a]indene-2-carboxylic acid tert-butyl ester (1.5 g; 5.47 mmol; 1 eq.) in AcOH (7.5 mL) and the resulting mixture was stirred at room temperature for 28 hours then poured dropwise into EtOH (40 mL) under vigorous stirring. The precipitate was filtered off, washed with EtOH then MTBE and dried to afford the title compound (0.75 g, 65%) as a white solid. ¹H NMR (DMSO-d₆) δ 10.50 (s, 2H), 9.06-9.02 (m, 1H), 8.51 (d, *J* = 2.1 Hz, 1H), 4.55-4.52 (m, 4H), 2.33 (s, 3H). HPLC (max plot) 100.0%; Rt 4.36 min.

### Intermediate C1 : 6,7-dimethyl-1H,3H-2,4,7a,8-tetraaza-cyclopenta[a]indene-2-carboxylic acid tert-butyl ester

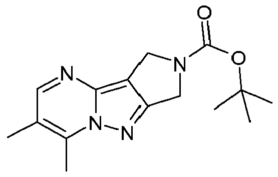

A mixture of 3-amino-2,6-dihydro-4H-pyrrolo[3,4-c]pyrazole-5-carboxylic acid tert-butyl ester (1 g; 4.46 mmol; 1 eq.) and (3E)-4-hydroxy-3-methylbut-3-en-2-one (prepared according to J. Het. Chem. 1980, 17(1), 33-37) (0.6 g; 4.91 mmol; 1.1 eq.) in AcOH (5 mL) was stirred at room temperature for 18 hours then concentrated in vacuo. The residue was partitioned between 1M NaOH and DCM, the organic layer was dried over magnesium sulfate and concentrated in vacuo. The resulting oil was triturated in Et₂O, the precipitate filtered off and the solution concentrated in vacuo. The residue was purified by SFC (column : Chiralpak IC, eluent 25% MeOH) to afford successively the title compound (140 mg, 11 %) as a white solid and Intermediate C2. ¹H NMR (CDCl₃) δ 8.33 (s, 1H), 4.72-4.62 (m, 4H), 2.54 (d, *J* = 3.1 Hz, 3H), 2.29 (d, *J* = 0.7 Hz, 3H), 1.52 (d, *J* = 3.3 Hz, 9H). HPLC (max plot) 87.3%; Rt 3.27 min. UPLC/MS: (MS+) 289.1
([M+H]⁺).

### Intermediate C2 : 5,6-dimethyl-1H,3H-2,4,7a,8-tetraaza-cyclopenta[a]indene-2-carboxylic acid tert-butyl ester

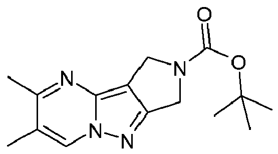

Second eluting compound isolated (170 mg, 13%) as a white solid during the preparation of Intermediate C1. ¹H NMR (CDCl₃) δ 8.27 (d, *J* = 3.0 Hz, 1H), 4.75-4.66 (m, 4H), 2.74 (s, 3H), 2.36 (s, 3H), 1.53 (s, 9H). UPLC/MS: (MS+) 289.1 ([M+H]⁺).

### Intermediate A6 : 6,7-dimethyl-2,3-dihydro-1H-2,4,7a,8-tetraaza-cyclopenta[a]indene bis hydrochloride

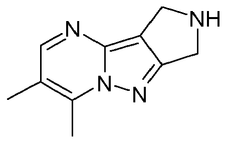

A 4M solution of HCl in 1,4-dioxane (10 mL; 40 mmol; 82.4 eq.) was added to a solution 6,7-dimethyl-1H,3H-2,4,7a,8-tetraaza-cyclopenta[a]indene-2-carboxylic acid tert-butyl ester (140 mg; 0.49 mmol; 1 eq.) in MeOH (2 mL) and the resulting mixture was stirred at room temperature for 18 hours then concentrated in vacuo. The residue was triturated in EtOH and the precipitate filtered off and dried to afford the title compound (78 mg, 62%) as a pale yellow solid. ¹H NMR (DMSO-d₆) δ 10.39 (br s, 2H), 8.91 (d, *J* = 0.9 Hz, 1H), 6.24 (br s, 1H), 4.51-4.48 (m, 4H), 2.49 (s, 3H), 2.26 (s, 3H). HPLC (max plot) 99.9%; Rt 1.03 min. UPLC/MS: (MS+) 189.0 ([M+H]⁺).

### Intermediate A7 : 5,6-dimethyl-2,3-dihydro-1H-2,4,7a,8-tetraazacyclopentaralindene bis hydrochloride

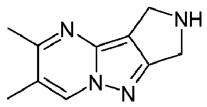

A 4M solution of HCl in 1,4-dioxane (10 mL; 40 mmol; 67.8 eq.) was added to a solution of 5,6-dimethyl-1H,3H-2,4,7a,8-tetraaza-cyclopenta[a]indene-2-carboxylic acid tert-butyl ester (170 mg; 0.59 mmol; 1 eq.) in MeOH (2 mL) and the resulting mixture was stirred at room temperature for 18 hours then concentrated in vacuo. The residue was triturated in EtOH and the precipitate filtered off and dried to afford the title compound (90 mg, 58%) as a pale yellow solid. ¹H NMR (DMSO-d₆) δ 10.41 (br s, 2H), 8.66-8.17 (br s, 1H), 8.45 (s, 1H), 4.57-4.53 (m, 4H), 2.70 (s, 3H), 2.35 (s, 3H). HPLC (max plot) 99.6%; Rt 1.03 min. UPLC/MS: (MS+) 189.0 ([M+H]⁺).

### Intermediate Z1 : (2-hydroxy-phenyl)-(5,6,7-trimethyl-1 H,3H-2,4,7a,8-tetraazacyclopenta[a]inden-2-yl)-methanone

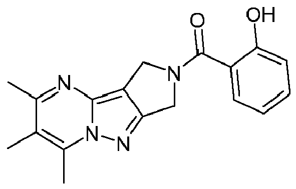

The title compound was prepared following procedure described in Method B starting Intermediate A1 and salicylic acid. After purification by recrystallization (ACN), the title compound was obtained as a white solid (463 mg, 34%). ¹H NMR (CDCl₃) δ 10.90 (s, 1H), 7.65-7.62 (m, 1H), 7.41-7.35 (m, 1H), 7.04-7.01 (m, 1H), 6.92-6.87 (m, 1H), 5.14-5.09 (m, 4H), 2.77 (s, 3H), 2.56 (s, 3H), 2.31 (s, 3H). HPLC (max plot) 94.4%; Rt 2.44 min. UPLC/MS: (MS+) 323.0 ([M+H]⁺), (MS-) 321.2 ([M-H]⁻).

### Intermediate A8 : 6-bromo-2,3-dihydro-1H-2,4,7a,8-tetraaza-cyclopenta[a]indene hydrochloride

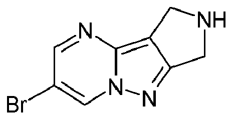

### Step 1 : 6-bromo-1H,3H-2,4,7a,8-tetraaza-cyclopenta[a]lindene-2-carboxylic acid tert-butyl ester

A mixture of bromomalonaldehyde (2.02 g; 13.38 mmol; 1 eq.) and 3-amino-2,6-dihydro-4H-pyrrolo[3,4-c]pyrazole-5-carboxylic acid tert-butyl ester (3 g; 13.38 mmol; 1 eq.) in AcOH (21 mL) was stirred at room temperature for 40 minutes. The insoluble material was removed by filtration and the solution diluted with water (60 mL). The precipitate was filtered off, washed with water (3x) and dried. Recrystallization from iPrOH afforded the title compound (2.02 g, 45%) as beige solid. ¹H NMR (DMSO-d₆) δ 9.60 (d, *J* = 2.2 Hz, 1H), 8.60 (d, *J* = 2.2 Hz, 1H), 4.62-4.55 (m, 4H), 1.47 (s, 9H). HPLC (max plot) 99.8%; Rt 3.54 min. UPLC/MS: (MS+) 339.3 and 341.3 ([M+H]⁺).

### Step 2 : 6-bromo-2,3-dihydro-1H-2,4,7a,8-tetraaza-cyclopenta[a]indene hydrochloride

A 4M solution of HCl in 1,4-dioxane (35 mL; 140 mmol; 23.7 eq.) was added to 6-bromo-1 H,3H-2,4,7a,8-tetraaza-cyclopenta[a]indene-2-carboxylic acid tert-butyl ester (2 g; 5.9 mmol; 1 eq.) and the resulting mixture was stirred at room temperature for 18 hours. The precipitate was filtered off, washed with Et₂O and dried to afford the title compound (1.56 g, 96%) as beige solid. ¹H NMR (DMSO-d₆) δ 10.61 (br s, 2H), 9.68 (d, *J* = 2.2 Hz, 1 H), 8.69 (d, J = 2.2 Hz, 1 H), 4.56 (s, 4H). UPLC/MS: (MS+) 239.1 and 241.1 ([M+H]⁺).

### Intermediate B1 : 2-ethoxy-4-fluoro-benzoic acid

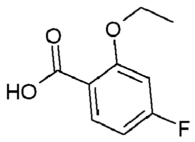

### Step 1 : 2-ethoxy-4-fluoro-benzoic acid ethyl ester

lodoethane (2.56 mL; 32.03 mmol; 2.5 eq.) was added to a suspension of 4-fluoro-2-hydroxybenzoic acid (2 g; 12.81 mmol; 1 eq.) and potassium carbonate (5.31 g; 38.43 mmol; 3 eq.) in DMF (20 mL) and the resulting mixture was stirred at 80°C for 3 hours then partitioned between MTBE (100 mL) and water (100 mL). The organic layer was washed with water then brine, dried over sodium sulfate and concentrated in vacuo to afford the title compound (2.3 g, 85%) as a pale yellow oil. ¹H NMR (DMSO-d₆) δ 7.71 (dd, *J* = 7.0, 8.6 Hz, 1 H), 7.03 (dd, *J* = 2.4, 11.7 Hz, 1H), 6.83 (dt, *J* = 2.4, 8.5 Hz, 1H), 4.23 (q, *J* = 7.1 Hz, 2H), 4.09 (q, *J* = 7.0 Hz, 2H), 1.33 (t, *J* = 7.0 Hz, 3H), 1.27 (t, *J* = 7.1 Hz, 3H). HPLC (max plot) 94.3%; Rt 4.04 min. UPLC/MS: (MS+) 213.2 ([M+H]⁺)

### Step 2 : 2-ethoxy-4-fluoro-benzoic acid

A 5M solution of sodium hydroxide (6.5 mL; 32.5 mmol; 3 eq.) was added to a solution of 2-ethoxy-4-fluoro-benzoic acid ethyl ester (2.3 g; 10.8 mmol; 1 eq.) in EtOH (46 mL) and the resulting mixture was stirred at 40°C for 18 hours then concentrated in vacuo. The residue was taken up in water and the pH made acidic with 5M HCl. The precipitate was filtered off, washed with water and dried to afford the title compound (1.8 g, 90%) as a white solid. ¹H NMR (DMSO-d₆) δ 12.56 (s, 1H), 7.71 (dd, *J* = 7.0, 8.6 Hz, 1H), 7.01 (dd, *J* = 2.4, 11.7 Hz, 1H), 6.80 (dt, *J* = 2.4, 8.5 Hz, 1H), 4.09 (q, *J* = 7.0 Hz, 2H), 1.32 (t, *J* = 7.0 Hz, 3H). HPLC (max plot) 99.3%; Rt 2.71 min. UPLC/MS: (MS+) 185.2 ([M+H]⁺).

### Intermediate Z2 : (6-chloro-5,7-dimethyl-1H,3H-2,4,7a,8-tetraazacyclopenta[a]inden-2-yl)-(4-fluoro-2-hydroxy-phenyl)-methanone

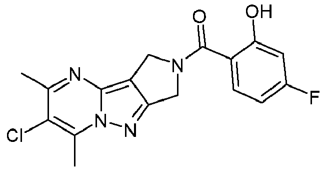

The title compound was prepared following procedure described in Method A starting from Intermediate A3 and 4-fluoro-2-hydroxybenzoic acid and the compound has precipitated out. After filtration, washing with DMF and drying the title compound was obtained as a white solid (1.14 g, 82%). ¹H NMR (CDCl₃) δ 11.47 (s, 1 H), 7.68-7.63 (m, 1H), 6.76-6.71 (m, 1H), 6.69-6.59 (m, 1H), 5.15-5.09 (m, 4H), 2.91 (s, 3H), 2.68 (s, 3H). HPLC (max plot) 91.9%; Rt 3.16 min. UPLC/MS: (MS+) 361.4 ([M+H]⁺), (MS-) 359.4 ([M-H]⁻).

### Intermediate B2 : 2-Ethoxy-3,5-difluoro-benzoic acid

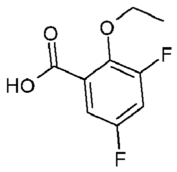

### Step 1 : 2-ethoxy-3,5-difluoro-benzoic acid ethyl ester

lodoethane (1.15 mL; 14.36 mmol; 2.5 eq.) was added to a suspension of 3,5-difluoro-2-hydroxybenzoic acid (1 g; 5.74 mmol; 1 eq.) and potassium carbonate (2.38 g; 17.23 mmol; 3 eq.) in DMF (20 mL) and the resulting mixture was stirred at 80°C for 20 hours then partitioned between MTBE (100 mL) and water (50 mL). The organic layer was washed with water then brine, dried over sodium sulfate and concentrated in vacuo to afford the title compound (1.16 g, 88%) as a colorless liquid. ¹H NMR (DMSO-d₆) δ 7.67-7.58 (m, 1 H), 7.38-7.32 (m, 1 H), 4.31 (q, *J* = 7.2 Hz, 2H), 4.05 (dq, *J* = 7.0, 0.6 Hz, 2H), 1.35-1.26 (m, 6H). HPLC (max plot) 99.8%; Rt 4.47 min. UPLC/MS: (MS+) 231.2 ([M+H]⁺).

### Step 2 : 2-ethoxy-3,5-difluoro-benzoic acid

A 5M solution of sodium hydroxide (3.2 mL; 16 mmol; 3.2 eq.) was added to a solution of 2-ethoxy-3,5-difluoro-benzoic acid ethyl ester (1.16 g; 5.04 mmol; 1 eq.) in EtOH (23 mL) and the resulting mixture was stirred at 40°C for 18 hours then concentrated in vacuo. The residue was taken up in water and the pH made acidic with 5M HCl. The precipitate was filtered off, washed with water and dried to afford the title compound (1.8 g, 90%) as a white solid. ¹H NMR (DMSO-d₆) δ 13.44 (s, 1 H), 7.61-7.52 (m, 1H), 7.35-7.28 (m, 1H), 4.04 (q, *J* = 7.0 Hz, 2H), 1.28 (t, *J* = 7.0 Hz, 3H). HPLC (max plot) 79.5%; Rt 3.13 min.

### Intermediate B3 : 4-(2-carboxy-phenoxy)-piperidine-1-carboxylic acid tert-butyl ester

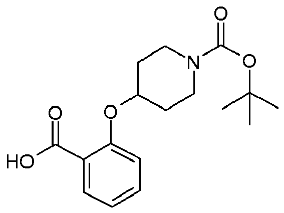

Di-tert-butyldicarbonate (2.03 g; 9.31 mmol; 1.2 eq.) was added to a solution of 2-(piperidin-4-yloxy)-benzoic acid hydrochloride (2 g; 7.76 mmol; 1 eq.) and TEA (2.16 mL; 15.5 mmol; 2 eq.) in DCM (20 mL) and the resulting mixture was stirred at room temperature for 16 hours. Water (150 mL) was added and the pH made acidic. The two phases were separated and the aqueous layer extracted with DCM. The combined organic phase was washed with brine, dried over magnesium sulfate and concentrated in vacuo to afford the title compound (1.03 g, 41%) as an off-white solid. ¹H NMR (DMSO-d₆) δ 12.62 (s, 1H), 7.49-7.44 (m, 1H), 7.18 (dd, *J* = 7.7, 1.8 Hz, 1H), 7.18 (d, *J* = 8.2 Hz, 1H), 7.02-6.97 (m, 1 H), 4.74-4.67 (m, 1H), 3.55-3.45 (m, 2H), 3.39-3.31 (m, 2H), 1.86-1.77 (m, 2H), 1.67-1.57 (m, 2H), 1.40 (s, 9H). HPLC (max plot) 99.8%; Rt 3.57 min. UPLC/MS: (MS-) 320.4 ([M-H]⁻).

### Preparation of Compounds of Formula (I)

### Example 1 : (6-chloro-5,7-dimethyl-1H,3H-2,4,7a,8-tetraaza-cyclopenta[a]inden-2-yl)-[2-(1-methyl-piperidin-4-yloxy)-phenyl]-methanone

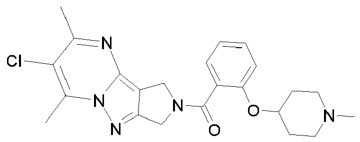

The title compound was prepared following procedure described in Method A starting Intermediate A3 and 2-(1-methyl-piperidin-4-yloxy)-benzoic acid hydrochloride. After purification by recrystallization from ACN, the title compound was obtained as a white solid (72 mg, 42%). ¹H NMR (CDCl₃) δ 7.39-7.32 (m, 2H), 7.07-6.95 (m, 2H), 5.02-5.01 (m, 2H), 4.67 (br s, 2H), 4.39 (br s, 1H), 2.91 (s, 2H), 2.85 (s, 1H), 2.69 (s, 1H), 2.62 (s, 2H), 2.57-2.47 (m, 2H), 2.31-2.21 (m, 2H), 2.19 (s, 3H), 2.01-1.75 (m, 4H). HPLC (max plot) 99.2%; Rt 2.56 min. UPLC/MS: (MS+) 440.4 ([M+H]+).

### Example 2 : (6-chloro-5,7-dimethyl-1H,3H-2,4,7a,8-tetraaza-cyclopenta[a]inden-2-yi)-[2-(1,1,2,2-tetrafluoro-ethoxy)-phenyl]-methanone

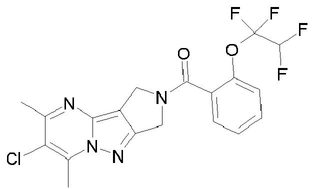

The title compound was prepared following procedure described in Method A starting from Intermediate A3 and 2-(1,1,2,2-tetrafluoroethoxy)benzoic acid. After purification by crystallization (ACN), the title compound was obtained as a white powder (59 mg, 23%). ¹H NMR (DMSO-d₆) δ 7.66-7.58 (m, 2H), 7.54-7.43 (m, 2H), 6.73 (t, *J* = 51.6 Hz, 1H), 4.84 (s, 1H), 4.82 (s, 1H), 4.55 (s, 1H), 4.51 (s, 1 H), 2.84 (s, 1.5H), 2.80 (s, 1.5H), 2.62 (s, 1.5H), 2.55 (s, 1.5H). HPLC (max plot) 99.9%, Rt 4.31 min. UPLC/MS: (MS+) 443.4 ([M+H]⁺), (MS-) 441.4 ([M-H]⁻). Melting point: 184-186°C (ACN).

### Example 3 : (6-chloro-5,7-dimethyl-1H,3H-2,4,7a,8-tetraaza-cyclopenta[a]inden-2-yl)-[2-(piperidin-4-yloxy)-phenyl]-methanone

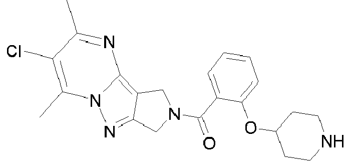

### Step 1 :4-[2-(6-chloro-5,7-dimethyl-1H,3H-2,4,7a,8-tetraaza-cyclopenta[a]indene-2-carbonyl)-phenoxy]-piperidine-1-carboxylic acid tert-butyl ester

The title compound was prepared following procedure described in Method A starting from Intermediate A3 and Intermediate B3. After aqueous work-up the crude title compound was obtained (2.78 g) as a pale yellow solid and was used in the next step without further purification. ¹H NMR (CDCl₃) δ 7.40-7.33 (m, 2H), 7.09-6.96 (m, 2H), 5.00-4.99 (m, 2H), 4.65 (br s, 2H), 4.56-4.49 (m, 1H), 3.56-3.46 (m, 2H), 3.38-3.28 (m, 2H), 2.90 (s, 2H), 2.85 (s, 1H), 2.69 (s, 1H), 2.61 (s, 2H), 1.90-1.65 (m, 4H), 1.41 (s, 9H). HPLC (max plot) 95.6%, Rt 4.39 min. UPLC/MS: (MS+) 426.4 ([M-Boc+2H]⁺).

### Step 2 : (6-chloro-5,7-dimethyl-1H,3H-2,4,7a,8-tetraaza-cyclopenta[a]inden-2-yl)-[2-(piperidin-4-yloxy)-phenyl]-methanone

A 4M solution of HCl in 1,4-dioxane (6.6 mL; 26.4 mmol; 5 eq.) was added to a solution of 4-[2-(6-chloro-5,7-dimethyl-1H,3H-2,4,7a,8-tetraaza-cyclopenta[a]indene-2-carbonyl)-phenoxy]-piperidine-1-carboxylic acid tert-butyl ester (2.78 g; 5.28 mmol; 1 eq.) in 1,4-dioxane (30 mL) and the reaction mixture was stirred at room temperature for 18 hours then concentrated in vacuo. The residue was partitioned between DCM and sat. aq. Na₂CO₃ and the two phases separated. The organic layer was dried over magnesium sulfate and concentrated in vacuo to afford the title compound (1.78 g, 79%) as a yellow solid. ¹H NMR (DMSO-d₆) δ 7.46-7.39 (m, 1H), 7.33-7.29 (m, 1H), 7.21-7.19 (m, 1H), 7.07-7.01 (m, 1H), 4.85 (s, 1H), 4.82 (s, 1H), 4.58-4.49 (m, 3H), 2.92-2.76 (m, 2H), 2.84 (s, 1.5H), 2.80 (s, 1.5H), 2.69-2.58 (m, 2H), 2.62 (s, 1.5H), 2.55 (s, 1.5H), 1.93-1.68 (m, 2H), 1.54-1.43 (m, 2H). HPLC (max plot) 92.5%; Rt 2.69 min. UPLC/MS: (MS+) 426.4 ([M+H]⁺).

### Example 4 : [2-(1-methyl-piperidin-4-yloxy)-phenyl]-(5,6,7-trimethyl-1H,3H-2,4,7a,8-tetraaza-cyclopenta[a]inden-2-yl)-methanone

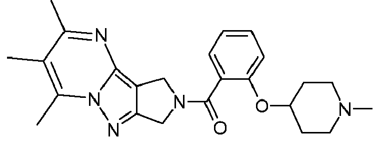

The title compound was prepared following procedure described in Method A starting from Intermediate A1 and 2-(1-methyl-piperidin-4-yloxy)-benzoic acid hydrochloride. After purification by mass directed preparative HPLC, the title compound was obtained as a white solid (65 mg, 31%). ¹H NMR (CDCl₃) δ 7.45-7.34 (m, 2H), 7.13-6.99 (m, 2H), 5.00 (s, 0.7H), 4.97 (s, 1.3H), 4.76 (s, 1H), 4.62 (s, 1.3H), 4.59 (s, 0.7H), 3.32-3.24 (m, 2H), 3.14-3.10 (m, 2H), 2.78-2.77 (m, 5H), 2.72 (s, 1H), 2.59 (s, 1H), 2.51 (s, 2H), 2.31 (s, 1H), 2.30 (s, 2H), 2.27-2.23 (m, 2H), 2.13-2.08 (m, 2H). HPLC (max plot) 99.5%; Rt 2.25 min. UPLC/MS: (MS+) 420.5 ([M+H]⁺).

### Example 5 : (6-chloro-5,7-dimethyl-1H,3H-2,4,7a,8-tetraaza-cyclopenta[a]inden-2-yl)-(2-ethoxy-4-fluoro-phenyl)-methanone

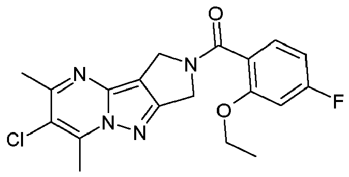

The title compound was prepared following procedure described in Method A starting from Intermediate A3 and Intermediate B1. After purification by crystallization (ACN), the title compound was obtained as a white powder (135 mg, 60%). ¹H NMR (DMSO-d₆) δ 7.39-7.31 (m, 1 H), 7.06 (d, *J* = 11.2 Hz, 1H), 6.91-6.81 (m, 1H), 4.82 (s, 1H), 4.79 (s, 1H), 4.56 (s, 1H), 4.51 (s, 1H), 4.14 (q, *J* = 6.9 Hz, 1 H), 4.13 (q, *J* = 6.9 Hz, 1H), 2.83 (s, 1.5H), 2.80 (s, 1.5H), 2.61 (s, 1.5H), 2.55 (s, 1.5H), 1.24 (t, *J* = 6.9 Hz, 1.5H), 1.23 (t, *J* = 6.9 Hz, 1.5H). HPLC (max plot) 99.2%, Rt 4.05 min. UPLC/MS: (MS+) 389.4 ([M+H]⁺).

### Example 6 : (6-chloro-5,7-dimethyl-1H,3H-2,4,7a,8-tetraaza-cyclopenta[a]inden-2-v)-[2-(4-methyl-[1,4]diazepan-1-yl)-phenyl]-methanone

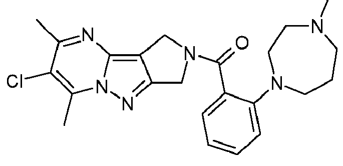

The title compound was prepared following procedure described in Method A starting from Intermediate A3 and 2-(4-methylperhydro-1,4-diazepin-1-yl)benzoic acid hydrochloride hemihydrate. After purification by crystallization (ACN), the title compound was obtained as a white powder (45 mg, 31 %). HPLC (max plot) 95.6%, Rt 2.74 min. UPLC/MS: (MS+) 439.4 ([M+H]⁺), (MS-) 437.5 ([M-H]⁻).

### Example 7 : (6-chloro-5,7-dimethyl-1H,3H-2,4,7a,8-tetraaza-cyclopenta[a]inden-2-yl)-(2-pyrrolidin-1-yl-phenyl)-methanone

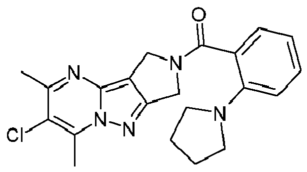

The title compound was prepared following procedure described in Method A starting from Intermediate A3 and 2-(pyrrolidin-1-yl)benzoic acid (Ukrorgsynthesis Ltd.), and has precipitated out from the reaction mixture. After filtration, washing twice with DMF and purification by crystallization (ACN), the title compound was obtained as a white powder (15 mg, 7%). ¹H NMR (DMSO-d₆) δ 7.31-7.16 (m, 2H), 6.80-6.68 (m, 2H), 4.84 (s, 1H), 4.81 (s, 1H), 4.78-4.63 (m, 1H), 4.55-4.38 (m, 1H), 3.31-3.20 (m, 2H), 3.19-3.06 (m, 2H), 2.83 (s, 1.5H), 2.80 (s, 1.5H), 2.61 (s, 1.5H), 2.55 (s, 1.5H), 1.90-1.75 (m, 4H). HPLC (max plot) 99.3%, Rt 3.65 min. UPLC/MS: (MS+) 396.4 ([M+H]⁺).

### Example 8 : (6-chloro-5,7-dimethyl-1H,3H-2,4,7a,8-tetraaza-cyclopenta[a]inden-2-yl)-[2-(2-dimethylamino-ethoxy)-phenyl]-methanone

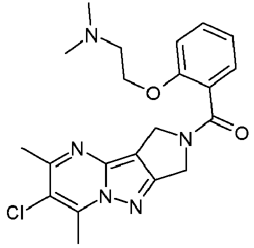

The title compound was prepared following procedure described in Method A starting from Intermediate A3 and 2-[2-(dimethylamino)ethoxy]benzoic acid hydrochloride (Enamine). After purification by flash chromatography (silica, DCM/EtOH/aqueous NH₃), followed by crystallization (MIBK), the title compound was obtained as a white powder (542 mg, 34%).¹H NMR (CDCl₃) δ 7.42-7.31 (m, 2H), 7.10-7.00 (m, 1H), 7.00-6.91 (m, 1H), 5.01-4.96 (m, 2H), 4.65 (s, 2H), 4.17 (t, *J* = 5.8 Hz, 2H), 2.91 (s, 1.8H), 2.85 (s, 1.2H), 2.72 (t, *J* = 5.8 Hz, 2H), 2.69 (s, 1.2H), 2.61 (s, 1.8H), 2.26 (s, 6H). HPLC (max plot) 99.4%, Rt 2.69 min. UPLC/MS: (MS+) 414.4 ([M+H]⁺). Melting point: 156-159°C (MIBK). Elemental analysis (C₂₁H₂₄ClN₅O₂): calculated: C 60.94%, H 5.84%, N 16.92%; found: C 60.85%, H 5.94%, N 16.75%.

### Example 9 : (6-chloro-5,7-dimethyl-1H,3H-2,4,7a,8-tetraaza-cyclopenta[a]inden-2-yl)-(2-isopropoxy-phenyl)-methanone

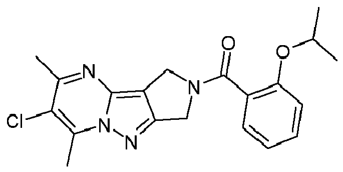

The title compound was prepared following procedure described in Method A starting from Intermediate A3 and 2-isopropoxybenzoic acid (Ukrorgsynthesis Ltd.). After purification by crystallization (ACN), the title compound was obtained as a pale beige powder (44 mg, 35%). ¹H NMR (DMSO-d₆) δ 7.46-7.38 (m, 1H), 7.31-7.26 (m, 1H), 7.14 (d, *J* = 8.3 Hz, 1H), 7.05-6.99 (m, 1H), 4.83 (s, 1H), 4.80 (s, 1H), 4.73-4.60 (m, 1H), 4.55 (s, 1H), 4.49 (s, 1H), 2.83 (s, 1.5H), 2.79 (s, 1.5H), 2.61 (s, 1.5H), 2.54 (s, 1.5H), 1.21 (d, *J* = 6.0 Hz, 3H), 1.20 (d, *J* = 6.0 Hz, 3H). HPLC (max plot) 98.0%, Rt 4.17 min. UPLC/MS: (MS+) 385.1 ([M+H]⁺), (MS-) 383.1 ([M-H]⁻).

### Example 10 : (6-chloro-5,7-dimethyl-1H,3H-2,4,7a,8-tetraaza-cyclopenta[a]inden-2-yl)-(2-ethoxy-phenyl)-methanone

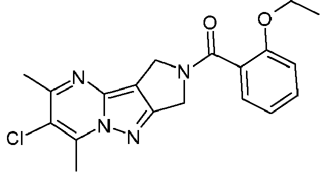

The title compound was prepared following procedure described in Method A starting from Intermediate A3 and 2-ethoxybenzoic acid. After purification by crystallization (ACN), the title compound was obtained as a white powder (184 mg, 66%).¹H NMR (DMSO-d₆) δ 7.47-7.39 (m, 1H), 7.32-7.27 (m, 1H), 7.13 (d, *J* = 8.4 Hz, 1H), 7.06-6.99 (m, 1H), 4.83(s, 1H), 4.80(s, 1H), 4.55(s, 1H), 4.50(s, 1H), 4.13(q, *J* = 6.9 Hz, 1H), 4.12 (q, *J* = 6.9 Hz, 1H), 2.82 (s, 1.5H), 2.78 (s, 1.5H), 2.61 (s, 1.5H), 2.54 (s, 1.5H), 1.24 (t, *J* = 6.9 Hz, 1.5H), 1.23 (t, *J* = 6.9 Hz, 1.5H). HPLC (max plot) 98.6%, Rt 3.87 min. UPLC/MS: (MS+) 371.0 ([M+H]⁺). Melting point: 164-167°C (ACN).

### Example 11 : (2-butoxy-phenyl)-(6-chloro-5,7-dimethyl-1H,3H-2,4,7a,8-tetraaza-cyclopenta[a]inden-2-yl)-methanone

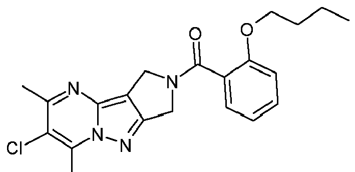

The title compound was prepared following procedure described in Method A starting from Intermediate A3 and 2-butoxybenzoic acid. After purification by crystallization (ACN), the title compound was obtained as a white powder (142 mg, 61%). ¹H NMR (DMSO-d₆) δ 7.47-7.39 (m, 1H), 7.32-7.27 (m, 1H), 7.14 (d, *J* = 8.4 Hz, 1H), 7.07-7.00 (m, 1H), 4.82 (s, 1H), 4.79 (s, 1H), 4.53 (s, 1H), 4.48 (s, 1H), 4.05 (t, *J* = 6.4 Hz, 1H), 4.04 (t, *J* = 6.4 Hz, 1H), 2.83 (s, 1.5H), 2.79 (s, 1.5H), 2.61 (s, 1.5H), 2.54 (s, 1.5H), 1.65-1.53 (m, 2H), 1.35-1.20 (m, 2H), 0.77 (t, *J* = 7.3 Hz, 1.5H), 0.75 (t, *J* = 7.3 Hz, 1.5H). HPLC (max plot) 99.1 %, Rt 4.55 min. UPLC/MS: (MS+) 399.4 ([M+H]⁺). Melting point: 167-169°C (ACN).

### Example 12 : (2-ethoxy-phenyl)-(5,6,7-trimethyl-1H,3H-2,4,7a,8-tetraaza-cyclopentafalinden-2-yl)-methanone

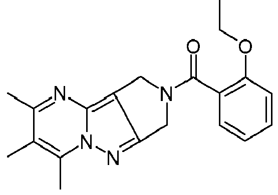

The title compound was prepared following procedure described in Method B starting from Intermediate A1 and 2-ethoxybenzoic acid. After purification by flash chromatography (petroleum ether/EA, 50/50 to EA) the title compound was obtained as an white solid (29 mg, 38%). ¹H NMR (CDCl₃) δ 7.39-7.28 (m, 2H), 7.01-6.88 (m, 2H), 5.10-4.95 (m, 2H), 4.68-4.60 (m, 2H), 4.16-4.06 (m, 2H), 2.74-2.68 (m, 3H), 2.54-2.48 (m, 3H), 2.26 (m, 3H), 1.35-1.19 (m, 3H). HPLC (max plot) 95.3%; Rt 3.22 min. UPLC/MS: (MS+) 351.0 ([M+H]⁺).

### Example 13 : (2-ethoxy-5-fluoro-phenyl)-(5,6,7-trimethyl-1H,3H-2,4,7a,8-tetraaza-cyclopentar[a]inden-2-yl)-methanone

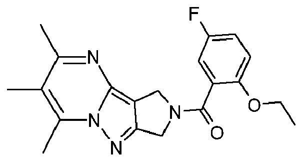

The title compound was prepared following procedure described in Method B starting from Intermediate A1 and 2-ethoxy-5-flurorobenzoic acid. After purification by flash chromatography (petroleum ether/EA, 50/50 to EA), the title compound was obtained as a white solid (82 mg, 70%). ¹H NMR (CDCl₃) δ 7.04-6.95 (m, 2H), 6.85-6.77 (m, 1H), 4.97-4.90 (m, 2H), 4.63-4.55 (m, 2H), 4.02-3.94 (m, 2H), 2.71-2.66 (m, 3H), 2.51-2.45 (m, 3H), 2.23 (s, 3H), 1.25-1.16 (m, 3H). HPLC (max plot) 96.7%; Rt 3.77 min. UPLC/MS: (MS+) 369.2 ([M+H]⁺).

### Example 14 : (6-chloro-5,7-dimethyl-1H,3H-2,4,7a,8-tetraaza-cyclopenta[a]inden-2-yl)-(2-trifluoromethoxy-phenyl)-methanone

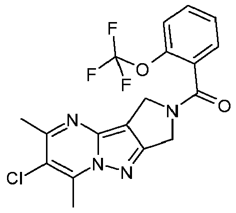

The title compound was prepared following procedure described in Method A starting from Intermediate A3 and 2-(trifluoromethoxy)benzoic acid. After purification by crystallization (ACN), the title compound was obtained as a white powder (97 mg, 41%). ¹H NMR (DMSO-d₆) δ 7.71-7.61 (m, 2H), 7.57-7.50 (m, 2H), 4.89 (s, 1H), 4.85 (s, 1H), 4.58 (s, 1H), 4.53 (s, 1H), 2.84 (s, 1.5H), 2.80 (s, 1.5H), 2.62 (s, 1.5H), 2.55 (s, 1.5H). HPLC (max plot) 100%, Rt 4.25 min. UPLC/MS: (MS+) 411.0 ([M+H]⁺). Melting point: 179-181°C (ACN).

### Example 15 : (6-chloro-5,7-dimethyl-1H,3H-2,4,7a,8-tetraaza-cyclopentaralinden-2-yl)-[2-(2-methylsulfanyl-ethoxy)-phenyl]-methanone

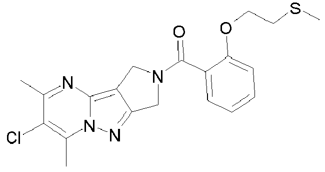

The title compound was prepared following procedure described in Method A starting from Intermediate A3 and 2-(2-methylsulfanyl-ethoxy)-benzoic acid (Ukrorgsynthesis Ltd.). After purification by crystallization (ACN), the title compound was obtained as a white powder (325 mg, 81%). ¹H NMR (DMSO-d₆) δ 7.48-7.41 (m, 1H), 7.33-7.28 (m, 1H), 7.16 (d, *J* = 8.3 Hz, 1H), 7.09-7.02 (m, 1H), 4.81 (s, 1H), 4.78 (s, 1H), 4.59 (s, 1H), 4.53 (s, 1H), 4.24 (t, *J* = 6.1 Hz, 2H), 2.83(s, 1.5H), 2.79 (s, 1.5H), 2.77 (t, *J* = 6.1 Hz, 1H), 2.76 (t, *J* = 6.1 Hz, 1H), 2.61 (s, 1.5H), 2.54 (s, 1.5H), 2.01 (s, 1.5H), 1.99 (s, 1.5H). HPLC (max plot) 99.4%, Rt 4.02 min. UPLC/MS: (MS+) 417.3 ([M+H]⁺), (MS-) 415.5 ([M-H]⁻). Melting point: 183-185°C (ACN).

### Example 16 : (6-chloro-5,7-dimethyl-1H,3H-2,4,7a,8-tetraaza-cyclopenta[a]inden-2-yl)-(3,5-difluoro-2-methoxy-phenyl)-methanone

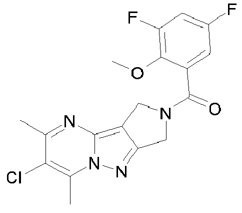

The title compound was prepared following procedure described in Method A starting from Intermediate A3 and 3,5-difluoro-2-methoxybenzoic acid (JRD Fluorochemicals Ltd.). After purification by crystallization (ACN), the title compound was obtained as a pale beige powder (80 mg, 35%). ¹H NMR (DMSO-d₆) δ 7.54-7.45 (m, 1H), 7.25-7.17 (m, 1H), 4.86 (s, 1H), 4.83 (s, 1H), 4.62 (s, 1H), 4.57 (s, 1H), 3.84 (t, *J* = 1.3 Hz, 3H), 2.84 (s, 1.5H), 2.81 (s, 1.5H), 2.62 (s, 1.5H), 2.56 (s, 1.5H). HPLC (max plot) 98.7%, Rt 3.99 min. UPLC/MS: (MS+) 393.4 ([M+H]⁺). Melting point: 185-187°C (ACN).

### Example 17 : (2-trifluoromethoxy-phenyl)-(5,6,7-trimethyl-1H,3H-2,4,7a,8-tetraaza-cyclopentafalinden-2-yl)-methanone

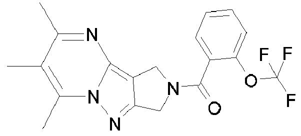

The title compound was prepared following procedure described in Method B starting from Intermediate A1 and 2-(trifluoromethoxy)benzoic acid. After purification by flash chromatography (petroleum ether/EA, 50/50 to EA) the title compound was obtained as a colourless oil (30 mg, 37%). ¹H NMR (CDCl₃) δ 7.53-7.46 (m, 2H), 7.43-7.33 (m, 2H), 5.06-5.01 (m, 2H), 4.65-4.58 (m, 2H), 2.79-2.77 (m, 3H), 2.59-2.58 (m, 3H), 2.32 (s, 3H). HPLC (max plot) 94.6%; Rt 4.02 min. UPLC/MS: (MS+) 391.1 ([M+H]⁺).

### Example 18 : (6-chloro-5,7-dimethyl-1H,3H-2,4,7a,8-tetraaza-cyclopenta[a]inden-2-yl)-[4-fluoro-2-(2-pyrazol-1-yl-ethoxy)-phenyl]-methanone

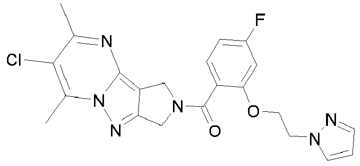

A mixture of Intermediate Z2 (100 mg; 0.28 mmol; 1 eq.), 1-(2-bromoethyl)-1H-pyrazole (97 mg; 0.55 mmol; 2 eq.) and K₂CO₃ (115 mg; 0.83 mmol; 3 eq.) in DMF (2.5 mL) was stirred at 150°C for 20 min (MW heating). The reaction mixture was diluted with water and extracted with DCM. The organic layer was washed with brine, dried over MgSO4 and concentrated in vacuo. Purification by mass directed preparative HPLC afforded the title compound (24 mg, 19%) as an off-white solid. ¹H NMR (CDCl₃) δ 7.33-7.19 (m, 3H), 6.78-6.71 (m, 1H), 6.65-6.60 (m, 1H), 5.63-5.61 (m, 1H), 4.96 (s, 0.7H), 4.94 (s, 1.3H), 4.49-4.45 (m, 2H), 4.38-4.34 (m, 2H), 4.23 (s, 1.3H), 4.19 (s, 0.7H), 2.93 (s, 2H), 2.88 (s, 1H), 2.71 (s, 1H), 2.63 (s, 2H). HPLC (max plot) 96.3%; Rt 3.31 min. UPLC/MS: (MS+) 455.4 ([M+H]⁺).

### Example 19 : [2-(4-methyl-[1,4]diazepan-1-yl)-phenyl]-(5,6,7-trimethyl-1H,3H-2,4,7a,8-tetraaza-cyclopenta[a]inden-2-yl)-methanone

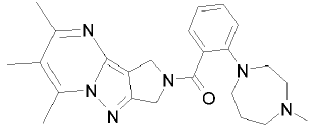

The title compound was prepared following procedure described in Method A starting from Intermediate A1 and 2-(4-methylperhydro-1,4-diazepin-1-yl)benzoic acid hydrochloride hemihydrate. After purification by mass directed preparative HPLC, the title compound was obtained as an off-white powder (80 mg, 46%).¹H NMR (CDCl₃) δ 7.35-7.27 (m, 2H), 6.99-6.89 (m, 2H), 5.10-4.77 (m, 3H), 4.52-4.28 (m, 1H), 3.57-3.39 (m, 4H), 2.79 (s, 2H), 2.73 (s, 1H), 2.70-2.58 (m, 4H), 2.59 (s, 1H), 2.52 (s, 2H), 2.34-2.26 (m, 6H), 1.97-1.87 (m, 2H). HPLC (max plot) 96.2%; Rt 2.25 min. UPLC/MS: (MS+) 419.2 ([M+H]⁺).

### Example 20 : [2-(2-piperidin-1-yl-ethoxy)-phenyl]-(5,6,7-trimethyl-1H,3H-2,4,7a,8-tetraaza-cyclopenta[a]inden-2-yl)-methanone

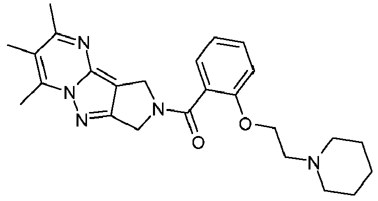

Intermediate Z1 (100 mg; 0.31 mmol; 1 eq.) was added to a suspension of NaH (60% in oil, 17.6 mg; 0.4 mmol; 1.3 eq.) in THF (3 mL) and DMF (2 mL) and the resulting mixture was stirred at room temperature for 10 minutes whereupon 1-(2-chloroethyl)piperidine hydrochloride (86 mg; 0.47 mmol; 1.5 eq.) was added. The reaction mixture was stirred at room temperature for 7 days, during which time several additions of NaH and 1-(2-chloroethyl)piperidine hydrochloride were made. The reaction mixture was partitioned between water and DCM and the two phases separated. The organic layer was washed with brine, dried over magnesium sulphate and concentrated in vacuo. Purification by mass directed preparative HPLC afforded the title compound (66 mg, 49%) as a white solid. ¹H NMR (CDCl₃) δ 7.40-7.31 (m, 2H), 7.05-6.92 (m, 2H), 5.01 (s, 0.7H), 4.98 (s, 1.3H), 4.65 (s, 2H), 4.17-4.14 (m, 2H), 2.78 (s, 2H), 2.75-2.69 (m, 3H), 2.59 (s, 1H), 2.51 (s, 2H), 2.42 (br s, 2H), 2.31 (s, 1H), 2.30 (s, 2H), 1.59-1.26 (m, 8H). HPLC (max plot) 98.4%; Rt 2.33 min. UPLC/MS: (MS+) 434.5 ([M+H]⁺).

### Example 21 : (2-butoxy-phenyl)-(5,6,7-trimethyl-1H,3H-2,4,7a,8-tetraaza-cyclopenta[a]inden-2-yl)-methanone

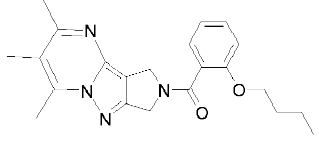

The title compound was prepared following procedure described in Method A starting from Intermediate A1 and 2-butoxybenzoic acid. After purification by mass directed preparative HPLC, the title compound was obtained as a yellow solid (77 mg, 49%). ¹H NMR (CDCl₃) δ 7.40-7.31 (m, 2H), 7.04-6.92 (m, 2H), 5.02 (s, 0.7H), 5.00 (s, 1.3H), 4.63 (br s, 2H), 4.04-3.98 (m, 2H), 2.79 (s, 2H), 2.73 (s, 1H), 2.59 (s, 1H), 2.52 (s, 2H), 2.31 (s, 1H), 2.30 (s, 2H), 1.73-1.64 (m, 2H), 1.42-1.30 (m, 2H), 0.86-0.81 (m, 3H). HPLC (max plot) 98.8%; Rt 3.74 min. UPLC/MS: (MS+) 379.1 ([M+H]⁺).

### Example 22 : [2-(2-dimethylamino-ethoxy)-phenyl]-(5,6,7-trimethyl-1H,3H-2,4,7a,8-tetraaza-cyclopenta[a]inden-2-yl)-methanone

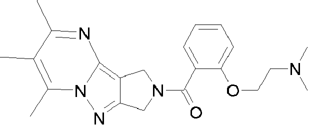

The title compound was prepared following procedure described in Method A starting from Intermediate A1 and 2-[2-(dimethylamino)ethoxy]benzoic acid. After purification by crystallization (DCM/Et₂O), the title compound was obtained as an off-white powder (110 mg, 21 %). ¹H NMR (DMSO-d₆) δ 7.47-7.39 (m, 1H), 7.32-7.27 (m, 1H), 7.14 (d, *J* = 8.1 Hz, 1H), 7.07-7.00 (m, 1H), 4.77 (s, 1H), 4.75 (s, 1H), 4.56 (br s, 1H), 4.52 (br s, 1H), 4.12 (t, *J* = 5.5 Hz, 2H), 2.72 (s, 1.5H), 2.68 (s, 1.5H), 2.55-2.50 (m, 3.5H), 2.45 (s, 1.5H), 2.26 (s, 1.5H), 2.25 (s, 1.5H), 2.05 (s, 3H), 2.04 (s, 3H). HPLC (max plot) 99.1%, Rt 2.22 min. UPLC/MS: (MS+) 394.2 ([M+H]⁺). Melting point: 148-150°C (DCM/Et₂O).

### Example 23 : acetic acid 2-(5,6,7-trimethyl-1H,3H-2,4,7a,8-tetraaza-cvclopenta[a]indene-2-carbonyl)-phenyl ester

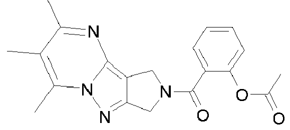

The title compound was prepared following procedure described in Method B starting from Intermediate A1 and acetylsalicylic acid. After purification by flash chromatography (petroleum ether/EA, 50/50 to EA) the title compound was obtained as a white solid (200 mg, 66%). ¹H NMR (DMSO) δ 7.66-7.49 (m, 2H), 7.41-7.37 (m, 1H), 7.28-7.25 (m, 1H), 4.85-4.78 (m, 2H), 4.58-4.54 (m, 2H), 2.71 (s, 1.5H), 2.68 (s, 1.5H), 2.52 (s, 1.5H), 2.45 (s, 1.5H), 2.27 (s, 1.5H), 2.25 (s, 1.5H), 2.17 (s, 3H). HPLC (max plot) 89.1%; Rt 3.27 min. UPLC/MS: (MS+) 365.15 ([M+H]⁺).

### Example 24 : [2-(2-pyrazol-1-yl-ethoxy)-phenyl]-(5,6,7-trimethyl-1H,3H-2,4,7a,8-tetraaza-cyclopentafalinden-2-yl)-methanone

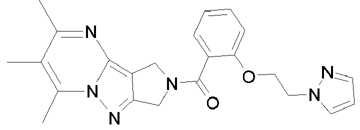

A mixture of Intermediate Z1 (100 mg; 0.31 mmol; 1 eq.), 1-(2-bromoethyl)-1H-pyrazole (81 mg; 0.47 mmol; 1.5 eq.) and K₂CO₃ (129 mg; 0.93 mmol; 3 eq.) in DMF (2.5 mL) was stirred at 140°C for 40 minutes (MW heating). 1-(2-bromoethyl)-1H-pyrazole (54.3 mg; 0.31 mmol; 1 eq.) was added and the reaction mixture was stirred at 140°C for a further 20 minutes (MW heating). The reaction mixture was partitioned between DCM and water and the two phases separated. The organic layer was washed with brine, dried over MgSO4 and concentrated in vacuo. Purification by mass directed preparative HPLC afforded the title compound (46 mg, 36%) as a white solid. ¹H NMR (CDCl₃) δ 7.38-7.27 (m, 3.3H), 7.20-7.19 (m, 0.7H), 7.06-6.99 (m, 1H), 6.90-6.87 (m, 1H), 5.63-5.62 (m, 0.7H), 5.61-5.59 (m, 0.3H), 5.00 (s, 0.7H), 4.97 (s, 1.3H), 4.48-4.45 (m, 2H), 4.37-4.34 (m, 2H), 4.26 (s, 1.3H), 4.22 (s, 0.7H), 2.80 (s, 2H), 2.74 (s, 1H), 2.61 (s, 1H), 2.52 (s, 2H), 2.33 (s, 1H), 2.32 (s, 2H). HPLC (max plot) 90.6%; Rt 2.69 min. UPLC/MS: (MS+) 417.4 ([M+H]⁺).

### Example 25 : (2-methylsulfanyl-phenyl)-(5,6,7-trimethyl-1H,3H-2,4,7a,8-tetraaza-cyclopenta[a]inden-2-yl)-methanone

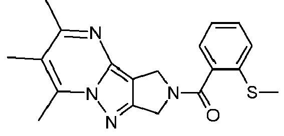

The title compound was prepared following procedure described in Method B starting from Intermediate A1 and 2-(methylthio)benzoic acid. After purification by flash chromatography (petroleum ether/EA, 50/50 to EA) the title compound was obtained as a white solid (31 mg, 42%). ¹H NMR (CDCl₃) δ 7.38-7.20 (m, 4H), 5.04-5.02 (m, 2H), 4.59-4.56 (m, 2H), 2.76 (s, 2H), 2.70 (s, 1H), 2.57 (s, 1H), 2.52-2.45 (m, 5H). HPLC (max plot) 94.0%; Rt 3.56 min. UPLC/MS: (MS+) 353.14 ([M+H]⁺).

### Example 26 : (2-isopropoxy-phenyl)-(5,6,7-trimethyl-1H,3H-2,4,7a,8-tetraaza-cyclopenta[a]inden-2-yl)-methanone

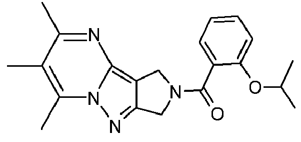

A mixture of Intermediate Z1 (50 mg; 0.15 mmol; 1 eq.), 2-iodopropane (77 mg; 0.45 mmol; 3 eq.) and K₂CO₃ (62 mg; 0.45 mmol; 3 eq.) in DMF (2.5 mL) was stirred at 140°C for 20 minutes (MW heating). The reaction mixture was partitioned between DCM and water and the two phases separated. The organic layer was washed with brine, dried over MgSO₄ and concentrated in vacuo. Purification by mass directed preparative HPLC afforded the title compound (3.7 mg, 7%) as a brown solid. HPLC (max plot) 99.0%; Rt 3.89 min. UPLC/MS: (MS+) 365.1 ([M+H]⁺).

### Example 27 : (2-ethoxy-phenyl)-(6-ethyl-5,7-dimethyl-1H,3H-2,4,7a,8-tetraaza-cyclopenta[a]inden-2-yl)-methanone

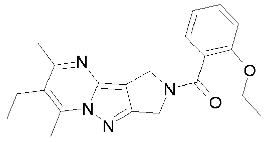

T3P (50% in DMF) (0.18 mL; 0.62 mmol; 1.3 eq.) was added to a solution of Intermediate A2 (100 mg; 0.46 mmol; 1 eq.), 2-ethoxy-benzoic acid (85 mg; 0.51 mmol; 1.1 eq.) and DIEA (243 µL; 1.43 mmol; 3.1 eq.) in DCE (10 mL) and the resulting mixture was stirred at room temperature for 16 hours. The solution was diluted with DCM and washed successively with sat. aq. NH₄Cl, sat. aq. NaHCO₃ and brine, dried over magnesium sulfate and concentrated in vacuo. Purification by column chromatography (EA) afforded the title compound (105 mg, 63%) as a white foam. ¹H NMR (DMSO-d₆) δ 7.47-7.39 (m, 1H), 7.32-7.26 (m, 1H), 7.13 (d, *J* = 8.3 Hz, 1H), 7.03 (t, *J* = 7.4 Hz, 1H), 4.82-4.77 (m, 2H), 4.52 (br s, 1H), 4.47 (br s, 1H), 4.17-4.07 (m, 2H), 2.76-2.65 (m, 5H), 2.57 (s, 1.5H), 2.51 (s, 1.5H), 1.26-1.19 (m, 3H), 1.16-1.08 (m, 3H). HPLC (max plot) 98.6%; Rt 3.59 min. UPLC/MS: (MS+) 365.4 ([M+H]⁺).

### Example 28 : (6-chloro-5,7-dimethYl-1H,3H-2,4,7a,8-tetraaza-cyclopenta[a]inden-2-yl)-(2-ethoxy-3,5-difluoro-phenyl)-methanone

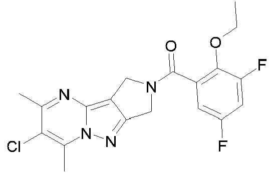

The title compound was prepared following procedure described in Method A starting from Intermediate A3 and Intermediate B2. After purification by crystallization (ACN/iPr₂O), the title compound was obtained as a pale beige powder (60 mg, 25%). ¹H NMR (DMSO-d₆) δ 7.54-7.44 (m, 1H), 7.23-7.16 (m, 1H), 4.86 (s, 1H), 4.83 (s, 1H), 4.62

(s, 1H), 4.58 (s, 1H), 4.07 (q, *J* = 7.0 Hz, 2H), 2.84 (s, 1.5H), 2.81 (s, 1.5H), 2.62 (s, 1.5H), 2.56 (s, 1.5H), 1.18 (t, *J* = 7.0 Hz, 3H). HPLC (max plot) 99.9%, Rt 4.32 min. UPLC/MS: (MS+) 407.4 ([M+H]⁺), (MS-) 405.4 ([M-H]⁻). Melting point: 153-155°C (ACN/iPr₂O).

### Example 29 : (2-benzyloxy-phenyl)-(5,6,7-trimethyl-1H,3H-2,4,7a,8-tetraaza-cyclopenta[a]inden-2-yl)-methanone

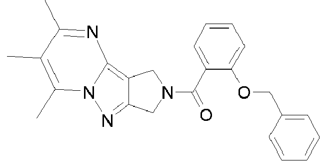

The title compound was prepared following procedure described in Method B starting from Intermediate A1 and 2-benzyloxybenzoic acid. After purification by mass directed preparative HPLC the title compound was obtained as an off-white powder (76 mg, 44%). ¹H NMR (CDCl₃) δ 7.39-7.32 (m, 4H), 7.29-7.21 (m, 3H), 7.07-6.99 (m, 2H), 5.15 (s, 0.7H), 5.14 (s, 1.3H), 5.04 (s, 0.7H), 5.01 (s, 1.3H), 4.64 (s, 1.3H), 4.63 (s, 0.7H), 2.77 (s, 2H), 2.71 (s, 1H), 2.58 (s, 1H), 2.50 (s, 2H), 2.30 (s, 1H), 2.29 (s, 2H). HPLC (max plot) 99.8%; Rt 3.69 min. UPLC/MS: (MS+) 419.2 ([M+H]⁺).

### Example 30 : (6-Chloro-5,7-dimethyl-1H,3H-2,4,7a,8-tetraaza-cyclopenta[a]inden-2-yl)-(2-isobutoxy-phenyl)-methanone

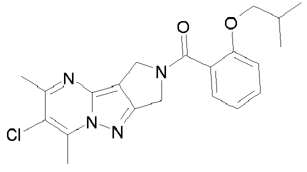

The title compound was prepared following procedure described in Method A starting from Intermediate A3 and 2-(2-methylpropoxy)benzoic acid (Enamine Ltd.). After purification by crystallization (ACN), the title compound was obtained as a white powder (192 mg, 83%). ¹H NMR (DMSO-d₆) δ 7.47-7.36 (m, 1H), 7.32-7.27 (m, 1H), 7.12 (d, *J* = 8.4 Hz, 1H), 7.07-7.00 (m, 1H), 4.83 (s, 1H), 4.80 (s, 1H), 4.53 (s, 1H), 4.48 (s, 1H), 3.82 (d, *J* = 6.2 Hz, 2H), 2.83 (s, 1.5H), 2.79 (s, 1.5H), 2.61 (s, 1.5H), 2.54 (s, 1.5H), 1.98-1.83 (m, 1H), 0.83 (d, *J* = 6.7 Hz, 3H), 0.82 (d, *J* = 6.7 Hz, 3H). HPLC (max plot) 99.7%, Rt 4.56 min. UPLC/MS: (MS+) 399.4 ([M+H]⁺). Melting point: 184-187°C (ACN).

### Example 31 : (5,6-Dimethyl-1H,3H-2,4,7a,8-tetraaza-cyclopenta[a]inden-2-yl)-(2-ethoxy-phenyl)-methanone

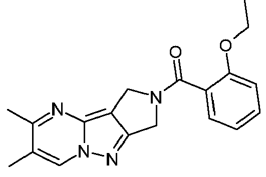

The title compound was prepared following procedure described in Method A starting from Intermediate A7 and 2-ethoxybenzoic acid. After purification by mass directed preparative HPLC the title compound was obtained as a white solid (28 mg, 36%). ¹H NMR (CDCl₃) δ 8.40 (s, 0.5H), 8.35 (s, 0.5H), 7.46-7.40 (m, 1H), 7.31-7.28 (m, 1H), 7.15-7.12 (m, 1H), 7.05-7.00 (m, 1H), 4.83-4.82 (m, 2H), 4.55-4.51 (m, 2H), 4.16-4.08 (m, 2H), 2.70 (s, 1.5H), 2.67 (s, 1.5H), 2.33 (s, 1.5H), 2.32 (s, 1.5H), 1.26-1.20 (m, 3H). HPLC (max plot) 96.2%; Rt 3.02 min. UPLC/MS: (MS+) 337.4 ([M+H]⁺).

### Example 32 : [2-(2-dimethylamino-ethoxy)-phenyl]-(5,6-dimethyl-1H,3H-2,4,7a,8-tetraaza-cyclopenta[a]inden-2-yl)-methanone

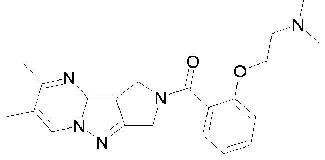

The title compound was prepared following procedure described in Method A starting from Intermediate A7 and 2-[2-(dimethylamino)ethoxy]benzoic acid. After purification by mass directed preparative HPLC the title compound was obtained as a yellow solid (80 mg, 61%). ¹H NMR (DMSO-d₆) δ 8.40 (s, 0.5H), 8.35 (s, 0.5H), 7.46-7.40 (m, 1H), 7.32-7.28 (m, 1H), 7.17-7.14 (m, 1H), 7.06-7.01 (m, 1H), 4.80-4.39 (m, 2H), 4.59 (br s, 2H), 4.13 (t, *J* = 5.5 Hz, 2H), 2.71 (s, 1.5H), 2.61 (s, 1.5H), 2.58-2.52 (m, 2H), 2.34 (s, 1.5H), 2.32 (s, 1.5H), 2.07 (s, 3H), 2.05 (s, 3H). HPLC (max plot) 94.8%; Rt 2.02 min. UPLC/MS: (MS+) 380.2 ([M+H]⁺).

### Example 33 : (5.6-dimethyl-1H,3H-2,4,7a,8-tetraaza-cyclopenta[a]inden-2-yl)-[2-(4-methyl-[1,4]diazepan-1-yl)-phenyl]-methanone

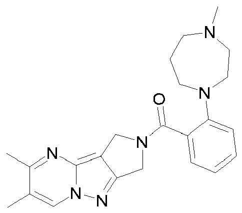

The title compound was prepared following procedure described in Method A starting from Intermediate A7 and 2-(4-methylperhydro-1,4-diazepin-1-yl)benzoic acid hydrochloride. After purification mass directed preparative HPLC, the title compound was obtained as a pale brown solid (42 mg, 45%). ¹H NMR (DMSO-d₆) δ 8.40 (s, 0.5H), 8.34 (s, 0.5H), 7.33-7.29 (m, 1H), 7.24-7.19 (m, 1H), 7.04-7.01 (m, 1H), 6.92-6.87 (m, 1H), 4.90-4.67 (m, 3H), 4.44-4.24 (m, 1H), 3.40-3.21 (m, 6H), 2.71 (s, 1.5H), 2.67 (s, 1.5H), 2.45-2.32 (m, 5H), 2.09 (s, 1.5H), 2.08 (s, 1.5H), 1.81-1.72 (m, 2H). HPLC (max plot) 96.8%; Rt 2.11 min. UPLC/MS: (MS+) 405.5 ([M+H]⁺).

### Example 34 : (5-chloro-2-ethoxy-phenyl)-(5,6,7-trimethyl-1H,3H-2,4,7a,8-tetraaza-cyclopenta[a]inden-2-yl)-methanone

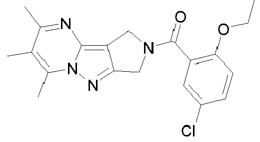

The title compound was prepared following procedure described in Method A starting from Intermediate A1 and 5-chloro-2-ethoxy-benzoic acid (Ukrorgsynthesis Ltd.), and has precipitated out from the reaction mixture. After filtration and slurry in hot ACN, the title compound was obtained as a white powder (40 mg, 29%). ¹H NMR (DMSO-d₆) δ 7.50-7.44 (m, 1H), 7.38-7.35 (m, 1H), 7.17 (d, *J* = 8.9 Hz, 1H), 4.80 (s, 1H), 4.78 (s, 1H), 4.55 (s, 1H), 4.51 (s, 1H), 4.13 (q, *J* = 6.9 Hz, 1H), 4.12 (q, *J* = 6.9 Hz, 1H), 2.72 (s, 1.5H), 2.69 (s, 1.5H), 2.52 (s, 1.5H), 2.46 (s, 1.5H), 2.27 (s, 1.5H), 2.26 (s, 1.5H), 1.23 (t, *J* = 6.9 Hz, 1.5H), 1.22 (t, *J* = 6.9 Hz, 1.5H). HPLC (max plot) 99.5%, Rt 3.66 min. UPLC/MS: (MS+) 385.3 ([M+H]⁺).

### Example 35 : (6-chloro-5,7-dimethyl-1H,3H-2,4,7a,8-tetraaza-cyclopenta[a]inden-2-Vl)-[2-(tetrahydro-pyran-4-yloxy)-phenyl]-methanone

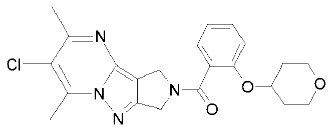

The title compound was prepared following procedure described in Method A starting from Intermediate A3 and 2-(tetrahydropyran-4-yloxy)benzoic acid and has precipitated out from the reaction mixture. After filtration and drying, the title compound was obtained as a beige solid (138 mg, 84%). ¹H NMR (CDCl₃) δ 7.40-7.33 (m, 2H), 7.09-6.96 (m, 2H), 5.02-5.01 (m, 2H), 4.67 (s, 2H), 4.58-4.50 (m, 1H), 3.89-3.81 (m, 2H), 3.56-3.49 (m, 2H), 2.91 (s, 2H), 2.85 (s, 1H), 2.69 (s, 1H), 2.62 (s, 2H), 2.00-1.93 (m, 2H), 1.80-1.69 (m, 2H). HPLC (max plot) 98.8%; Rt 3.42 min. UPLC/MS: (MS+) 427.4 ([M+H]⁺).

### Example 36 : [2-(4-methyl-piperazin-1-yl)-phenyl]-(5,6,7-trimethyl-1H,3H-2,4,7a,8-tetraaza-cyclopenta[a]inden-2-yl)-methanone

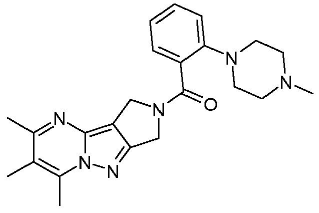

The title compound was prepared following procedure described in Method A starting from Intermediate A1 and 2-(4-methylpiperazin-1-yl)benzoic acid. After purification by flash chromatography (silica, DCM/acetone), followed by crystallization (EtOH), the title compound was obtained as a white powder (10 mg, 6%). ¹H NMR (DMSO-d₆) δ 7.45-7.37 (m, 1H), 7.29-7.22 (m, 1H), 7.13-7.05 (m, 2H), 4.97-4.65 (br m, 3H), 4.27 (br s, 1H), 3.16 (br s, 2H), 2.89 (br s, 2H), 2.73 (s, 1.5H), 2.69 (s, 1.5H), 2.53 (s, 1.5H), 2.46 (s, 1.5H), 2.31-2.20 (m, 7H), 2.03 (s, 1.5H), 2.02 (s, 1.5H). HPLC (max plot) 96.7%; Rt 2.25 min. UPLC/MS: (MS+) 405.2 ([M+H]⁺).

### Example 37 : (2-ethylamino-phenyl)-(5,6,7-trimethyl-1H,3H-2,4,7a,8-tetraaza-cyclopenta[a]inden-2-yl)-methanone

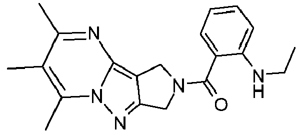

The title compound was prepared following procedure described in Method B starting from Intermediate A1 and 2-ethylaminobenzoic acid. After purification by mass directed preparative HPLC, the title compound was obtained as a white solid (22 mg, 25%). ¹H NMR (CDCl₃) δ 7.33-7.26 (m, 2H), 6.78-6.67 (m, 2H), 5.05-4.85 (m, 4H), 3.16 (q, *J* = 7.0 Hz, 2H), 2.78 (br s, 3H), 2.55 (br s, 3H), 2.32 (s, 3H), 1.26 (t, *J* = 7.0 Hz, 3H). HPLC (max plot) 91.6%; Rt 3.02 min. UPLC/MS: (MS+) 350.2 ([M+H]⁺).

### Example 38 : [2-(2-dimethylamino-ethoxy)-phenyl]-(6,7-dimethyl-1H,3H-2,4,7a,8-tetraaza-cyclopenta[a]inden-2-yl)-methanone

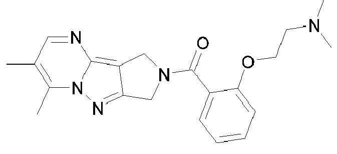

The title compound was prepared following procedure described in Method A starting from Intermediate A6 and 2-[2-(dimethylamino)ethoxy]benzoic acid. After purification by mass directed preparative HPLC, the title compound was obtained as a yellow solid (80 mg, 70%). ¹H NMR (DMSO-d₆) δ 8.86 (s, 0.5H), 8.85 (s, 0.5H), 7.46-7.39 (m, 1H), 7.30-7.27 (m, 1H), 7.15-7.12 (m, 1H), 7.05-7.00 (m, 1H), 4.74 (s, 2H), 4.54-4.50 (m, 2H), 4.13 (t, *J* = 4.5 Hz, 2H), 2.56-2.52 (m, 2H), 2.48 (s, 1.5H), 2.43 (s, 1.5H), 2.24 (br s, 3H), 2.06 (s, 3H), 2.05 (s, 3H). HPLC (max plot) 95.6%; Rt 1.95 min. UPLC/MS: (MS+) 380.2 ([M+H]⁺).

### Example 39 : (2-pyrazol-1-yl-phenyl)-(5,6,7-trimethyl-1H,3H-2,4,7a,8-tetraaza-cyclopenta[a]inden-2-yl)-methanone

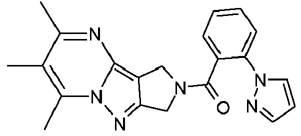

The title compound was prepared following procedure described in Method A starting from Intermediate A1 and 2-(1H-pyrazol-1-yl)benzoic acid. After purification by mass directed preparative HPLC, the title compound was obtained as an off-white solid (91 mg, 49%). ¹H NMR (CDCl₃) δ 7.91-7.89 (m, 1H), 7.68-7.40 (m, 5H), 6.38-6.36 (m, 1H), 4.89 (s, 2H), 4.37 (s, 1.3H), 4.31 (s, 0.7H), 2.74 (s, 2H), 2.69 (s, 1H), 2.56 (s, 1H), 2.48 (s, 2H), 2.28 (s, 1H), 2.27 (s, 2H). HPLC (max plot) 99.4%; Rt 2.81 min. UPLC/MS: (MS+) 373.4 ([M+H]⁺).

### Example 40 : [2-(2-cyclohexyl-ethoxy)-phenyl]-(5,6,7-trimethyl-1H,3H-2,4,7a,8-tetraaza-cyclopenta[a]inden-2-yl)-methanone

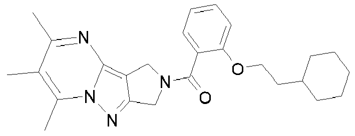

A mixture of Intermediate Z1 (100 mg; 0.31 mmol; 1 eq.), (2-bromoethyl)cyclohexane, (74 µL; 0.47 mmol; 1.5 eq.) and K₂CO₃ (129 mg; 0.93 mmol; 3 eq.) in DMF (2.5 mL) was stirred at 140°C for 20 minutes (MW heating). The reaction mixture was partitioned between DCM and water and the two phases separated. The organic layer was washed with brine, dried over MgSO₄ and concentrated in vacuo. Purification by mass directed preparative HPLC afforded the title compound (93 mg, 69%) as a white solid. ¹H NMR (CDCl₃) δ 7.39-7.31 (m, 2H), 7.04-6.91 (m, 2H), 5.02 (s, 0.7H), 5.00 (s, 1.3H), 4.62 (s, 2H), 4.06-4.01 (m, 2H), 2.78 (s, 2H), 2.72 (s, 1H), 2.59 (s, 1H), 2.51 (s, 2H), 2.31 (s, 1 H), 2.30 (s, 2H), 1.65-1.28 (m, 8H), 1.00-0.79 (m, 5H). HPLC (max plot) 99.6%; Rt 4.46 min. UPLC/MS: (MS+) 433.4 ([M+H]⁺).

### Example 41 : (6,7-dimethyl-1H,3H-2,4,7a,8-tetraaza-cyclopenta[a]inden-2-inden-[2-(4-methyl-[1,4]diazepan-1-y)-phenyl]-methanone

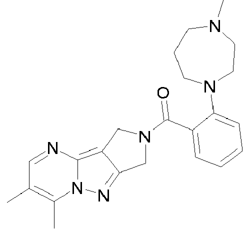

The title compound was prepared following procedure described in Method A starting from Intermediate A6 and 2-(4-methylperhydro-1,4-diazepin-1-yl)benzoic acid hydrochloride hemihydrate. After purification by mass directed preparative HPLC, the title compound was obtained as a pale brown solid (19 mg, 31%). ¹H NMR (DMSO-d₆) δ 8.88 (m, 0.5H), 8.85 (m, 0.5H), 7.34-7.29 (m, 1H), 7.23-7.18 (m, 1H), 7.04-7.01 (m, 1H), 6.92-6.87 (m, 1H), 4.79-4.64 (m, 3H), 4.33-4.21 (m, 1H), 3.29-3.23 (m, 6H), 2.50-2.43 (m, 3H), 2.41-2.30 (m, 2H), 2.25-2.24 (m, 3H), 2.09 (s, 1.5H), 2.08 (s, 1.5H), 1.80-1.73 (m, 2H). HPLC (max plot) 98.2%; Rt 2.06 min. UPLC/MS: (MS+) 405.5 ([M+H]⁺).

### Example 42 : (6-chloro-1H,3H-2,4,7a,8-tetraaza-cyclopenta[a]inden-2-yl)-(2-isopropoxy-phenyl)-methanone

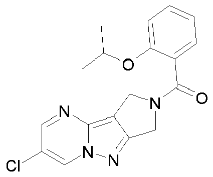

The title compound was prepared following procedure described in Method A starting from Intermediate A4 and 2-isopropoxybenzoic acid (Ukrorgsynthesis Ltd.). After purification by crystallization (ACN), the title compound was obtained as a pale yellow powder (49 mg, 27%). ¹H NMR (DMSO-d₆) δ 9.59 (d, *J* = 2.3 Hz, 0.5H), 9.56 (d, *J* = 2.3 Hz, 0.5H), 8.62 (d, *J* = 2.3 Hz, 0.5H), 8.57 (d, *J* = 2.3 Hz, 0.5H), 7.46-7.38 (m, 1H), 7.32-7.26 (m, 1H), 7.15 (d, *J* = 8.3 Hz, 1 H), 7.06-6.98 (m, 1H), 4.87-4.81 (m, 2H), 4.74-4.61 (m, 1H), 4.56 (s, 1H), 4.53 (s, 1H), 1.21 (d, *J* = 6.0 Hz, 3H), 1.20 (d, *J* = 6.0 Hz, 3H). HPLC (max plot) 100%, Rt 3.65 min. UPLC/MS: (MS+) 357.0 ([M+H]⁺). Melting point: 160°C-161°C (ACN).

### Example 43 : (2-trifluoromethyl-phenyl)-(5,6,7-trimethyl-1H,3H-2,4,7a,8-tetraaza-cyclopenta[a]inden-2-yl)-methanone

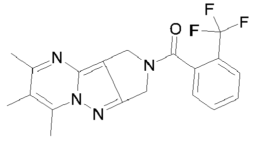

T3P (50% in DMF) (0.18 mL; 0.62 mmol; 3 eq.) was added to a solution of Intermediate A1 (50 mg; 0.21 mmol; 1 eq.), 2-trifluoromethyl-benzoic acid (43 mg; 0.23 mmol; 1.1 eq.) and DIEA (109 µL; 0.64 mmol; 3.1 eq.) in DCE and the resulting mixture was stirred at room temperature for 16 hours. The solution was diluted with DCM and washed successively with sat. aq. NH₄Cl, sat. aq. NaHCO₃ and brine, dried over magnesium sulfate and concentrated in vacuo. Purification by column chromatography (EA) afforded the title compound (35 mg, 45%) as a white foam. ¹H NMR (DMSO-d₆) δ 7.92-7.77 (m, 2H), 7.76-7.66 (m, 2H), 4.86-4.81 (m, 2H), 4.50-4.40 (m, 2H), 2.73 (s, 1.6H), 2.68 (s, 1.4H), 2.53 (s, 1.4H), 2.46 (s, 1.6H), 2.27 (s, 1.4H), 2.26 (s,1.6H). HPLC (max plot) 97.3%, Rt 3.45 min. UPLC/MS: (MS+) 375.4 ([M+H]⁺).

### Example 44 : 2-(5,6,7-trimethyl-1H,3H-2,4,7a,8-tetraaza-cyclopenta[a]indene-2-carbonyl)-benzoic acid methyl ester

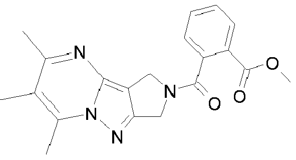

The title compound was prepared following procedure described in Method B starting from Intermediate A1 and monomethyl phthalate. After purification by flash chromatography (petroleum ether/EA, 50/50 to EA), the title compound was obtained as an off-white solid (203 mg, 66%). ¹H NMR (CDCl₃) δ 8.04-8.01 (m, 1H), 7.63-7.38 (m, 3H), 5.02-5.00 (m, 2H), 4.45-4.42 (m, 2H), 3.80-3.65 (m, 3H), 2.72-2.65 (s, 3H), 2.53-2.44 (s, 3H), 2.25-2.24 (s, 3H). HPLC (max plot) 91.8%; Rt 3.20 min. UPLC/MS: (MS+) 365.2 ([M+H]⁺).

### Example 45 : (5-bromo-2-ethoxy-phenyl)-(5,6,7-trimethyl-1H,3H-2,4,7a,8-tetraaza-cyclopenta[a]inden-2-yl)-methanone

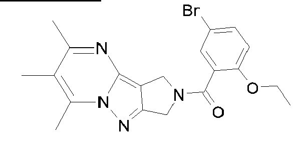

The title compound was prepared following procedure described in Method B starting from Intermediate A1 and 5-bromo-2-ethoxy-benzoic acid. After purification by crystallization (ACN), the title compound was obtained as an off-white solid (99 mg, 55%). ¹H NMR (CDCl₃) δ 7.48-7.43 (m, 2H), 6.85-6.81 (m, 1H), 5.01-4.63 (m, 4H), 4.11-4.02 (m, 2H), 2.78-2.73 (m, 3H), 2.58-2.52 (m, 3H), 2.30 (s, 3H), 1.36-1.30 (m, 3H). HPLC (max plot) 98.0%; Rt 3.63 min. UPLC/MS: (MS+) 429.0 ([M+H]⁺). Melting point: 208-215°C (ACN).

### Example 46 : (6-chloro-1H,3H-2,4,7a,8-tetraaza-cyclopenta[a]inden-2-yl)-(2-ethoxy-phenyl)-methanone

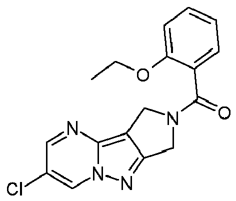

The title compound was prepared following procedure described in Method A starting from Intermediate A4 and 2-ethoxybenzoic acid, and has precipitated out from the reaction mixture. After filtration and washing with water (3x), the title compound was obtained as a white powder (132 mg, 48%). ¹H NMR (DMSO-d₆) δ 9.60 (d, *J* = 2.3 Hz, 0.5H), 9.57 (d, *J* = 2.3 Hz, 0.5H), 8.63 (d, *J* = 2.3 Hz, 0.5H), 8.58 (d, *J* = 2.3 Hz, 0.5H), 7.47-7.40 (m, 1H), 7.33-7.27 (m, 1H), 7.14 (d, *J* = 8.2 Hz, 1H), 7.06-7.00 (m, 1H), 4.85 (s, 1H), 4.84 (s, 1H), 4.57(s, 1H), 4.54 (s, 1H), 4.13 (q, *J* = 7.0 Hz, 1H), 4.12 (q, *J* = 7.0 Hz, 1H), 1.24 (t, *J* = 7.0 Hz, 1.5H), 1.23 (t, *J* = 7.0 Hz, 1.5H). HPLC (max plot) 99.1%, Rt 3.35 min. UPLC/MS: (MS+) 343.0 ([M+H]⁺).

### Example 47 : [2-(2-dimethylamino-ethoxy)-phenyl]-(6-methyl-1H,3H-2,4,7a,8-tetraaza-cyclopenta[a]inden-2-yl)-methanone

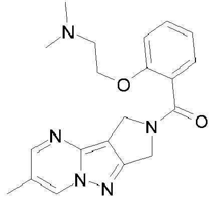

The title compound was prepared following procedure described in Method A starting from Intermediate A5 and 2-[2-(dimethylamino)ethoxy]benzoic acid. After purification by flash chromatography (silica, DCM/EtOH/aqueous NH₃), the title compound was obtained as a beige foam (28 mg, 9%). ¹H NMR (DMSO-d₆) δ 9.01-8.97 (m, 1H), 8.46 (d, *J* = 2.1 Hz, 0.5H), 8.41 (d, *J* = 2.1 Hz, 0.5H), 7.47-7.40 (m, 1H), 7.32-7.28 (m, 1H), 7.16 (d, *J* = 8.3 Hz, 1H), 7.07-7.01 (m, 1H), 4.79 (s, 2H), 4.58 (br s, 2H), 4.14 (t, *J* = 5.2 Hz, 2H), 2.61-2.55 (m, 2H), 2.34-2.29 (m, 3H), 2.09 (s, 3H), 2.07 (s, 3H). HPLC (max plot) 98.5%, Rt 1.87 min. UPLC/MS: (MS+) 366.4 ([M+H]⁺).

### Example 48 : (6-bromo-1H.3H-2,4,7a,8-tetraaza-cyclopenta[a]inden-2-yl)-(2-ethoxy-phenyl)-methanone

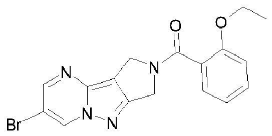

The title compound was prepared following procedure described in Method A starting from Intermediate A8 and 2-ethoxybenzoic acid, and has precipitated out from the reaction mixture. The reaction mixture was diluted with water and the precipitate was filtered off to give the title compound as a pale beige powder (1.97 g, 94%).¹H NMR (DMSO-d₆) δ 9.63 (d, *J* = 2.2 Hz, 0.5H), 9.61 (d, *J* = 2.2 Hz, 0.5H), 8.64 (d, *J* = 2.2 Hz, 0.5H), 8.59 (d, *J* = 2.2 Hz, 0.5H), 7.47-7.40 (m, 1H), 7.33-7.27 (m, 1H), 7.14 (d, *J* = 8.2 Hz, 1H), 7.06-7.00 (m, 1H), 4.84 (s, 2H), 4.56 (s, 1H), 4.53(s, 1H), 4.13 (q, *J* = 7.0 Hz, 1H), 4.13 (q, *J* = 7.0 Hz, 1H), 1.24 (t, *J* = 7.0 Hz, 1.5H), 1.24 (t, *J* = 7.0 Hz, 1.5H). HPLC (max plot) 99.5%, Rt 3.20 min. UPLC/MS: (MS+) 387.3 and 389.3 ([M+H]⁺).

### Example 49 : (2-bromo-phenyl)-(5,6,7-trimethyl-1H,3H-2,4,7a,8-tetraaza-cyclopenta[a]inden-2-yl)-methanone

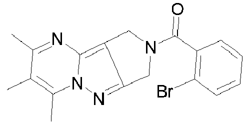

The title compound was prepared following procedure described in Method B starting from Intermediate A1 and 2-bromobenzoic acid. After purification by column chromatography (DCM/MeOH), the title compound was obtained as an off-white powder (570 mg, 35%). ¹H NMR (CDCl₃) δ 7.65-7.61 (m, 1H), 7.45-7.36 (m, 2H), 7.33-7.27 (m, 1H), 5.05-5.03 (m, 2H), 4.59-4.56 (m, 2H), 2.78-2.72 (m, 3H), 2.59-2.51 (m, 3H), 2.31-2.30 (m, 3H). HPLC (max plot) 96.0%, Rt 3.09 min. UPLC/MS: (MS+) 385.0 and 387.0 ([M+H]⁺).

### Example 50 :M1 PAM Assay

M1-CHO cells are plated in culture medium (HAM'sF12,P/S,10%FCS) on the day before the experiment with 10 000 cells/well in a 384 well plate (3750 Corning White 384w plate with lid). On the day of experiment, cells are washed with PBS and IPone buffer is added (glucose 5.5 mM, NaCl 146 mM, MgCl₂ 0.5 mM, HEPES 10 mM, LiCl 50 mM, CaCl₂ 1 mM, KCl 4.2 mM). Then diluted compounds (1%DMSO final concentration) are added together with EC₂₀ of acetylcholine and incubated with the cells for 1 hour at room temperature. The intracellular concentration of IP1 is then measured using the IP-One HTRF assay from Cisbio.

Activity range of the compounds of Formula (I) is the following:

| Ex | Structures | M1 PAM EC₅₀ (nM) |
|---|---|---|
| 1 | 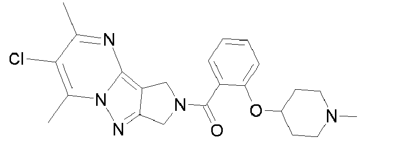 | *** |
| 2 | 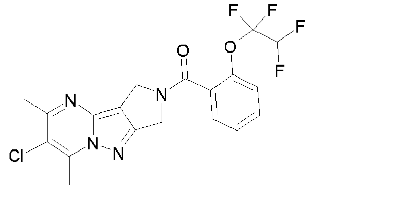 | *** |
| 3 | 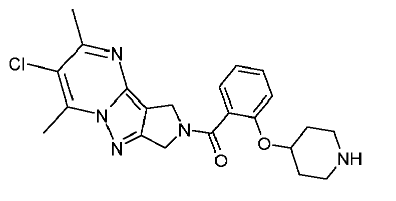 | *** |
| 4 | 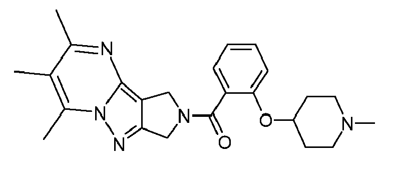 | ** |
| 5 | 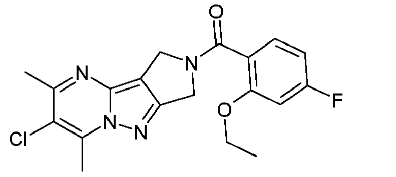 | ** |
| 6 | 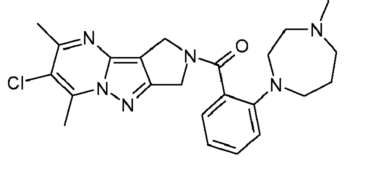 | ** |
| 7 | 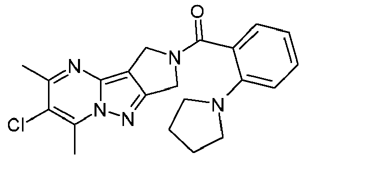 | ** |
| 8 | 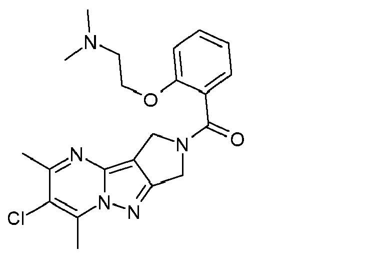 | ** |
| 9 | 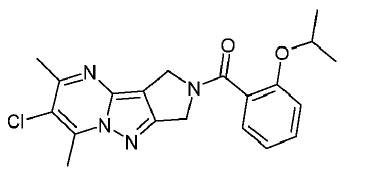 | ** |
| 10 | 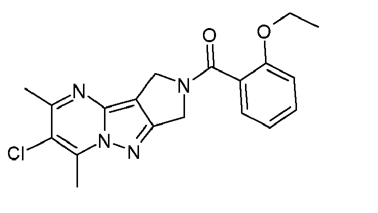 | ** |
| 11 | 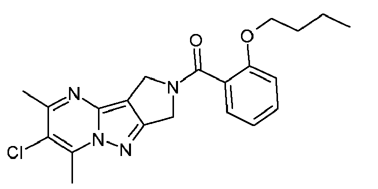 | ** |
| 12 | 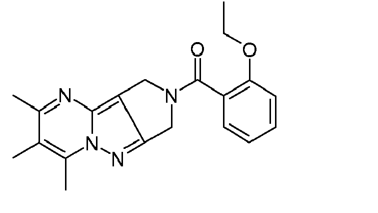 | ** |
| 13 | 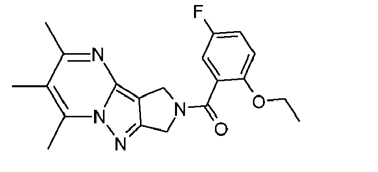 | ** |
| 14 | 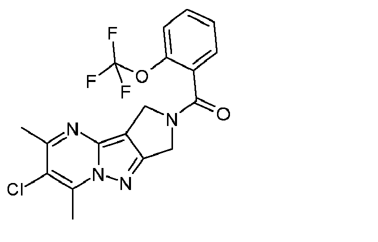 | ** |
| 15 | 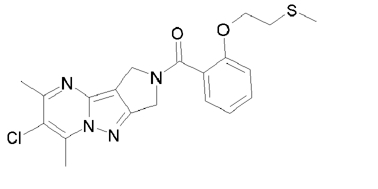 | ** |
| 16 | 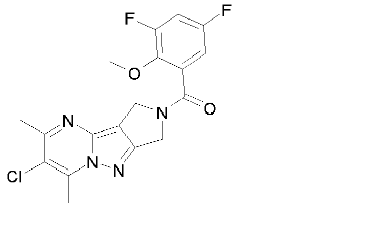 | ** |
| 17 | 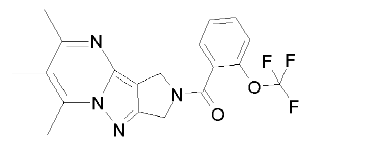 | ** |
| 18 | 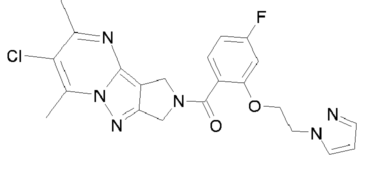 | ** |
| 19 | 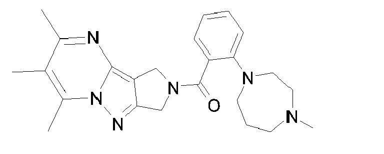 | ** |
| 20 | 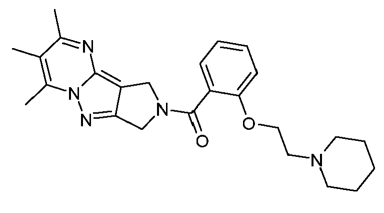 | ** |
| 21 | 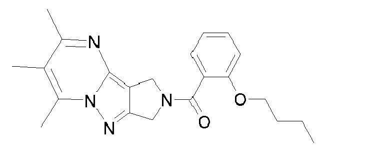 | ** |
| 22 | 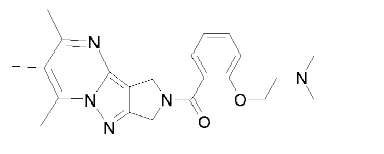 | ** |
| 23 | 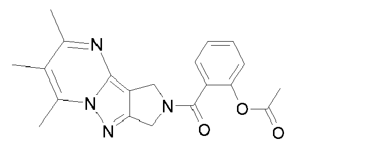 | ** |
| 24 | 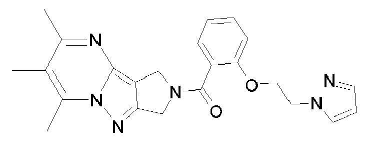 | ** |
| 25 | 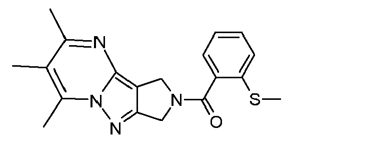 | ** |
| 26 | 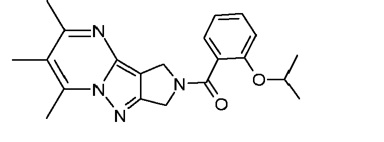 | ** |
| 27 | 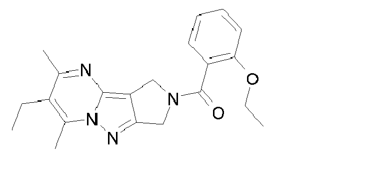 | * |
| 28 | 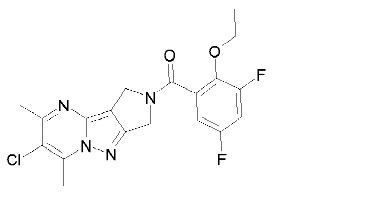 | * |
| 29 | 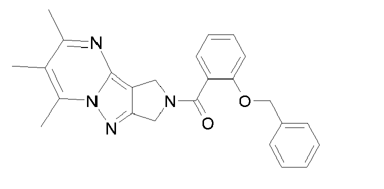 | * |
| 30 | 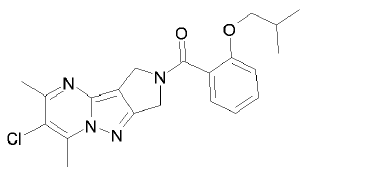 | * |
| 31 | 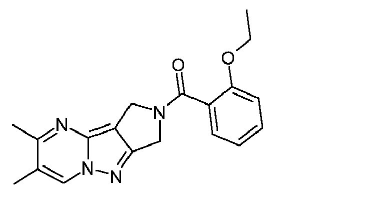 | * |
| 32 | 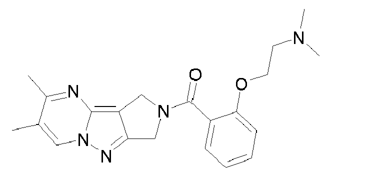 | * |
| 33 | 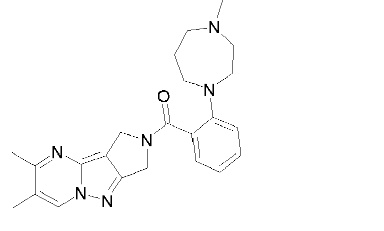 | * |
| 34 | 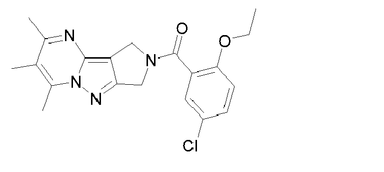 | * |
| 35 | 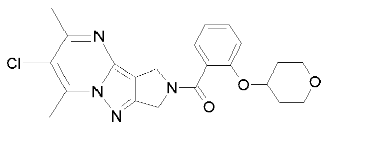 | * |
| 36 | 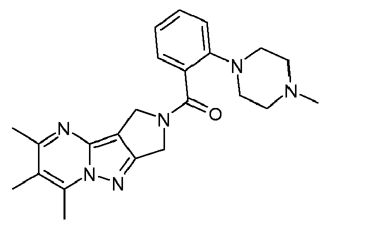 | * |
| 37 | 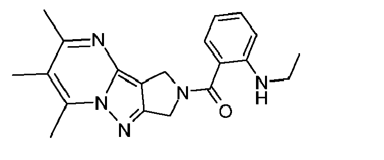 | * |
| 38 | 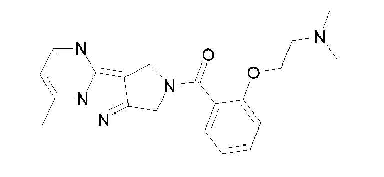 | * |
| 39 | 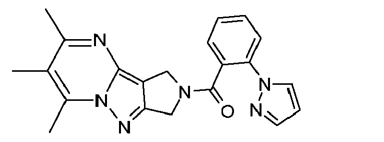 | * |
| 40 | 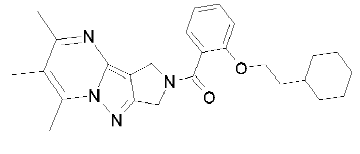 | * |
| 41 | 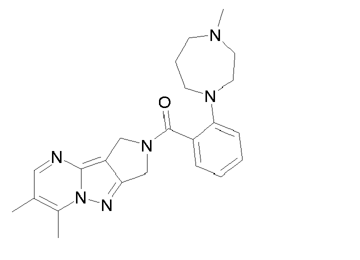 | * |
| 42 | 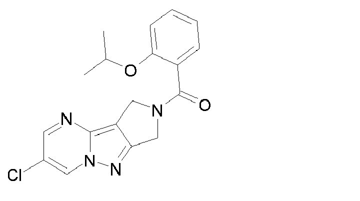 | * |
| 43 | 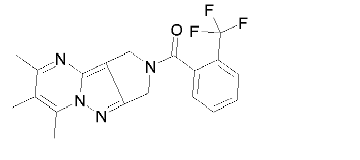 | * |
| 44 | 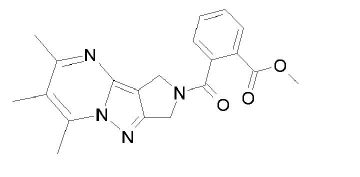 | * |
| 45 | 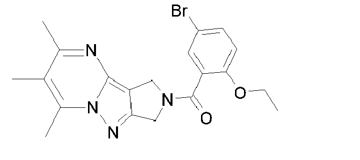 | * |
| 46 | 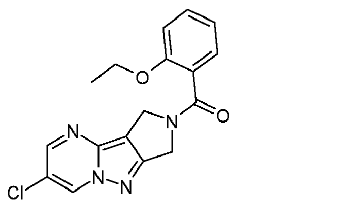 | * |
| 47 | 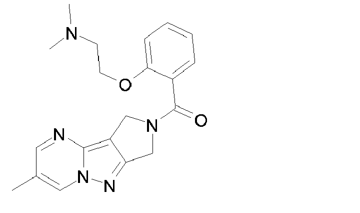 | * |
| 48 | 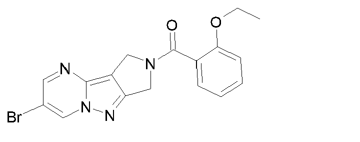 | * |
| 49 | 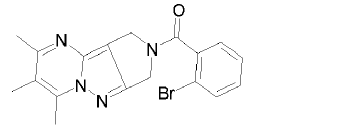 | * |

| | | |
|---|---|---|
| * 1 to 2 µM ** 0.2 to 1 µM *** below 0.2 µM | | |

### Example 51: Preparation of a pharmaceutical formulations

### Formulation 1 - Tablets

A compound of formula (I) is admixed as a dry powder with a dry gelatin binder in an approximate 1:2 weight ratio. A minor amount of magnesium stearate is added as a lubricant. The mixture is formed into 240-270 mg tablets (80-90 mg of active compound according to the invention per tablet) in a tablet press.

### Formulation 2 - Capsules

A compound of formula (I) is admixed as a dry powder with a starch diluent in an approximate 1:1 weight ratio. The mixture is filled into 250 mg capsules (125 mg of active compound according to the invention per capsule).

### Formulation 3 - Liquid

A compound of formula (1) (1250 mg), sucrose (1.75 g) and xanthan gum (4 mg) are blended, passed through a No. 10 mesh U.S. sieve, and then mixed with a previously prepared solution of microcrystalline cellulose and sodium carboxymethyl cellulose (11:89, 50 mg) in water. Sodium benzoate (10 mg), flavor, and color are diluted with water and added with stirring. Sufficient water is then added to produce a total volume of 5 mL.

### Formulation 4 - Tablets

A compound of formula (I) is admixed as a dry powder with a dry gelatin binder in an approximate 1:2 weight ratio. A minor amount of magnesium stearate is added as a lubricant. The mixture is formed into 450-900 mg tablets (150-300 mg of active compound according to the invention) in a tablet press.

### Formulation 5 - Injection

A compound of formula (I) is dissolved in a buffered sterile saline injectable aqueous medium to a concentration of approximately 5 mg/mL.

## Claims

1. A compound of Formula (I) 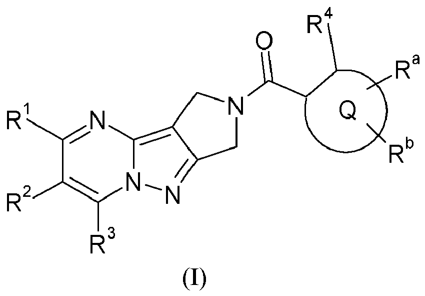 Wherein
R¹, R², R³ are independently from each other selected from H, linear or branched C₁-C₆-alkyl, linear or branched C₁-C₆-alkoxy, Hal, or hydroxyl;
R^{a}, R^{b} are independently from each other selected from H, Hal, hydroxy or A;
Q denotes a 6-membered aromatic group or a 5-6-membered heteroaromatic group having 1 to 4 heteroatoms independently selected from N, O and S.
R⁴ denotes G, OG, SG, NR⁵G, -COOG, or OCOG;
R⁵ denotes H or a linear or branched alkyl having 1 to 6 carbon atoms.
G denotes -CH₃, -CF₃, -CH₂-A, Het, Cyc, Ar, -CH₂-Het, -CH₂-Cyc, -CH₂-Ar, Hal, hydroxy;
Hal denotes F, Cl, Br or I, preferably F, Cl or Br;
A is a linear or branched carbon chain having 1 to 6 carbon atoms, wherein 1 to 3 non adjacent -CH₂-groups may be independently from each other replaced by a group selected from O, NR⁵ S, SO, SO₂, CO, and wherein 1 to 5 hydrogen atoms may be independently from each other replaced by Het, Cyc, Ar, or Hal;
Het denotes a saturated, unsaturated or aromatic ring, being monocyclic or bicyclic or fused-bicyclic and having 3- to 8- members and containing 1 to 4 heteroatoms independently selected from N, NR⁵, O, S, CO, SO or SO₂, which may be substituted by 1 to 3 substituents independently selected from A, Hal, OH and Het¹;
Het¹ denotes a 4, 5 or 6 membered carbocyclic ring wherein 1 or 2 carbon atom are replaced by Oxygen atoms.
Ar denotes a 6-membered carbocyclic aromatic ring or a fused or non fused byclic aromatic ring, and optionally substituted by 1 to 3 substituents independently selected from A or Hal;
Cyc denotes a saturated or unsaturated carbocyclic ring having from 3 to 8 carbon atoms and optionally substituted by 1 to 3 substituents independently selected from A or Hal;
and/or pharmaceutically usable derivatives, tautomers, salts, solvates and stereoisomers thereof.

2. A compound of Formula (I) as defined in claim 1 wherein
Q is a phenyl ring;
R^{a}, R^{b} are independently selected from H, Hal, Hydroxy, or a linear or branched alkyl group having 1 to 6 carbon atoms and wherein 1 to 3 hydrogen atoms may be replaced by Hal;
R⁴ is G or OG;

3. A compound of Formula (I) as defined in claims 1 or 2 wherein the group 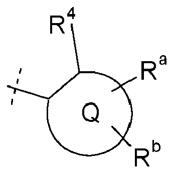 is selected from one of the following groups

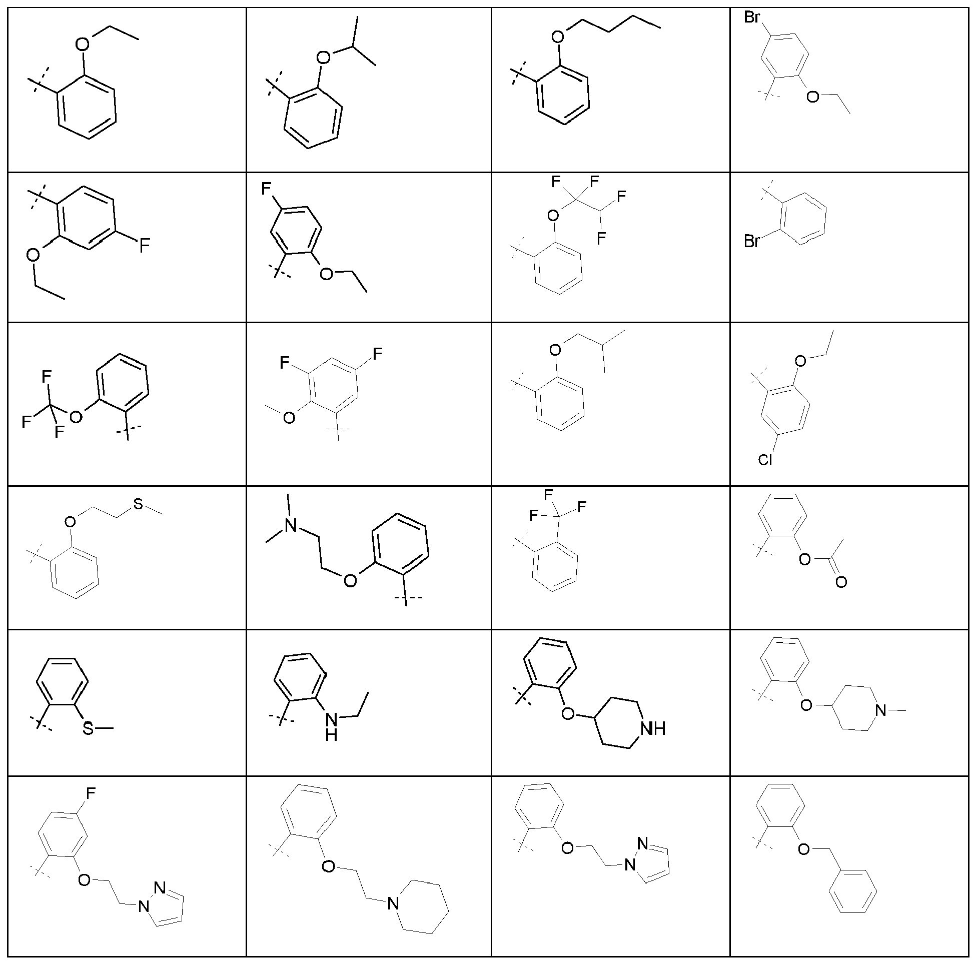

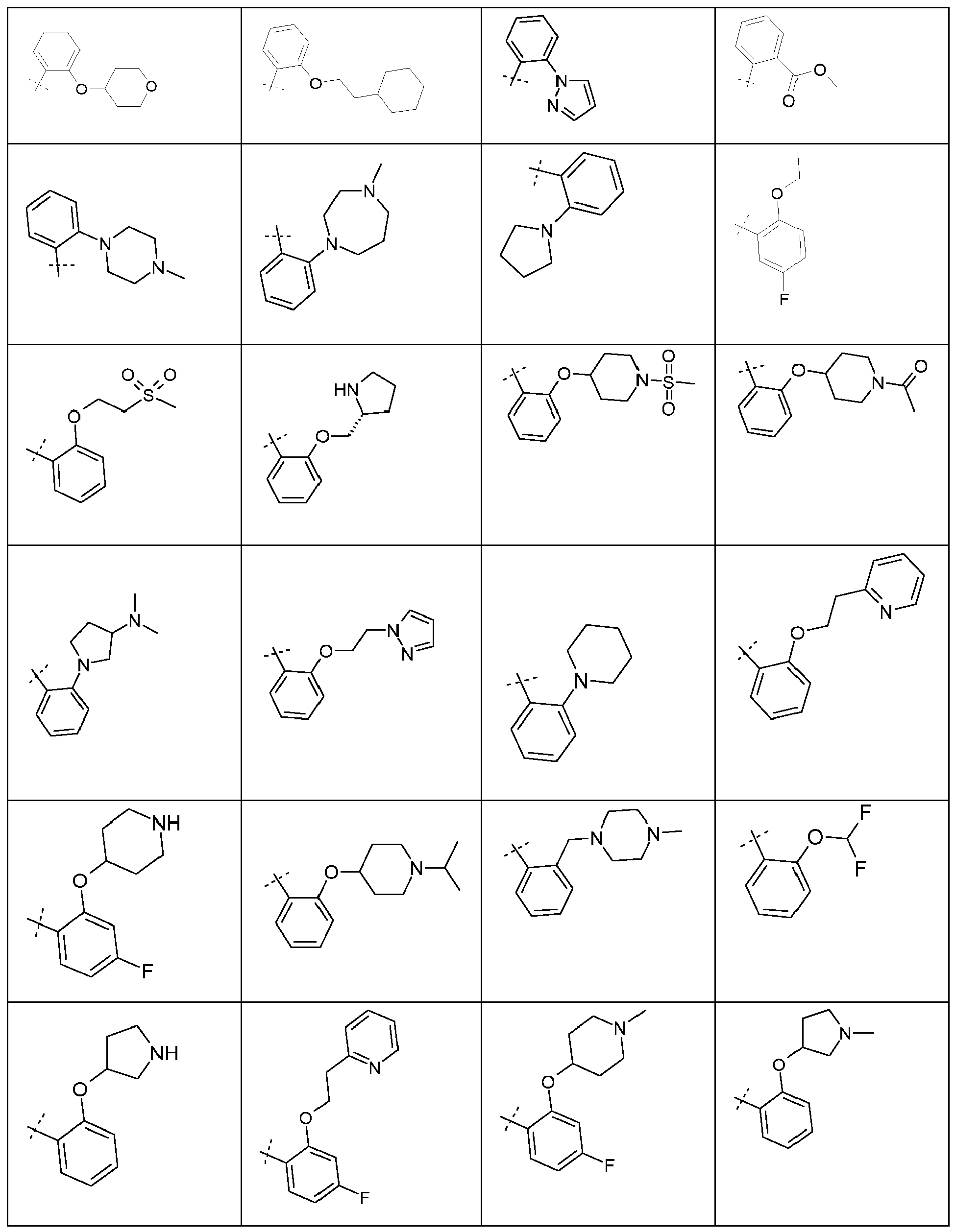

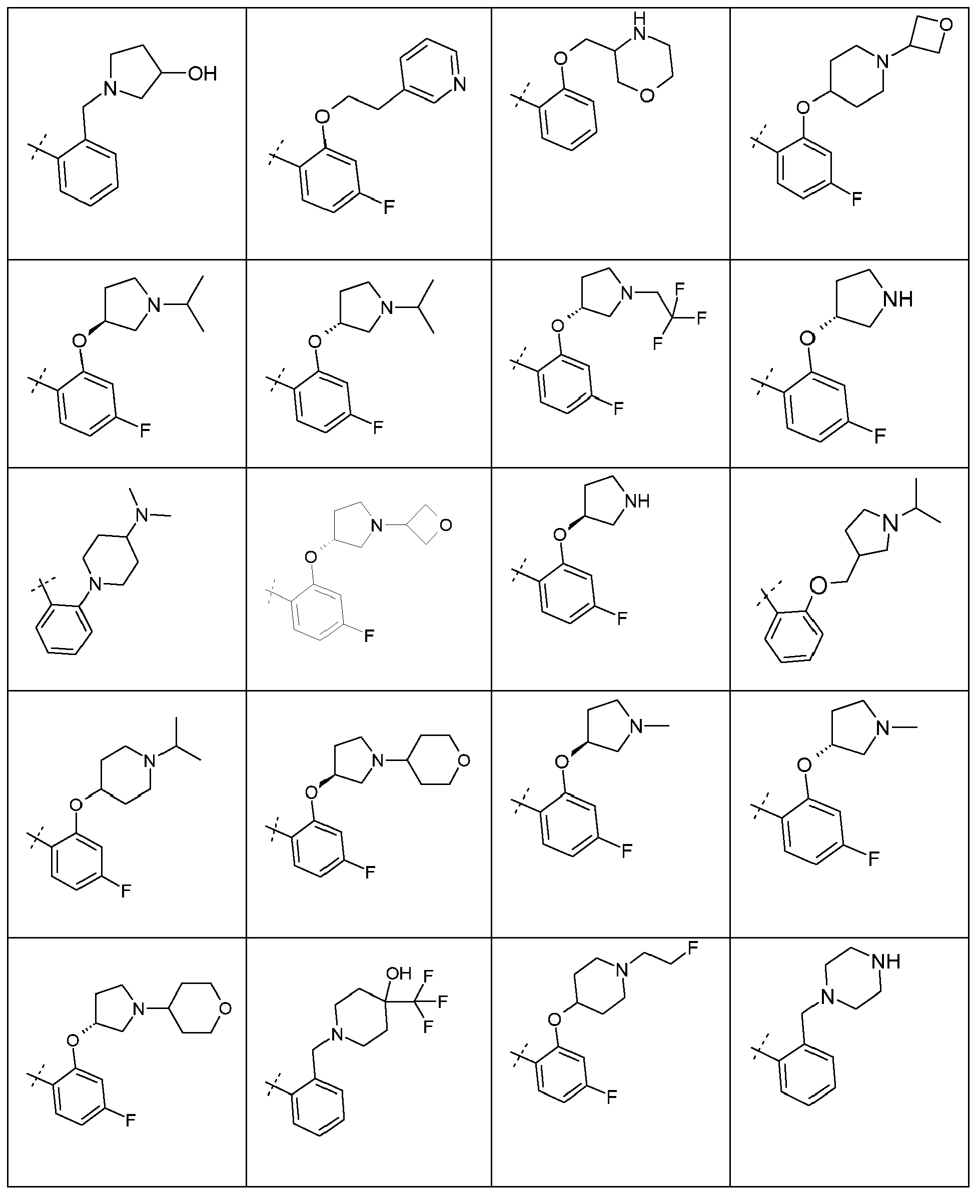

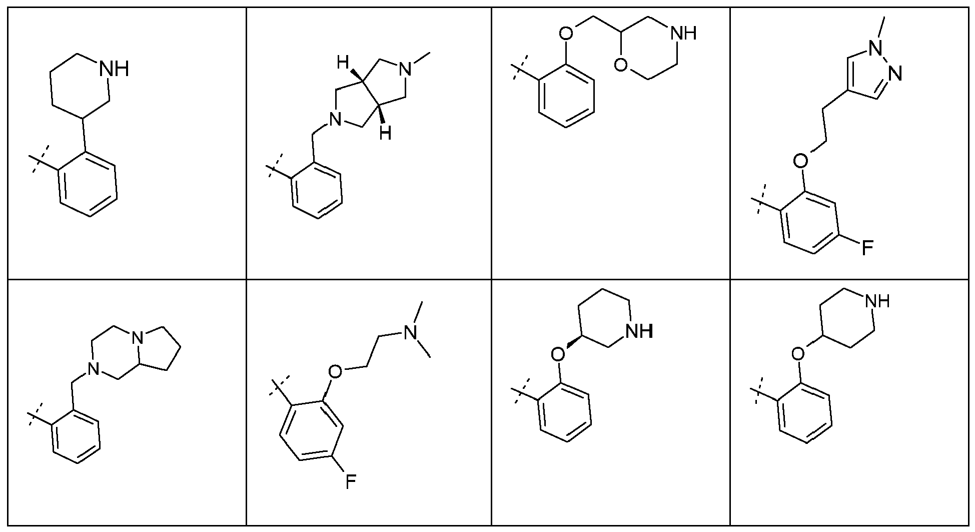

4. A compound of Formula (I) according to claims 1 to 3 wherein the compound is selected from the following group:

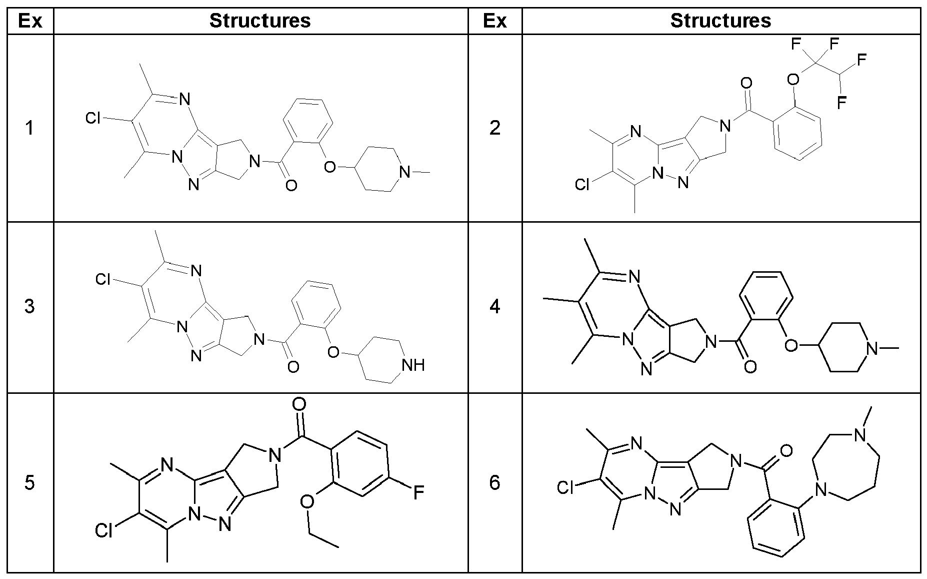

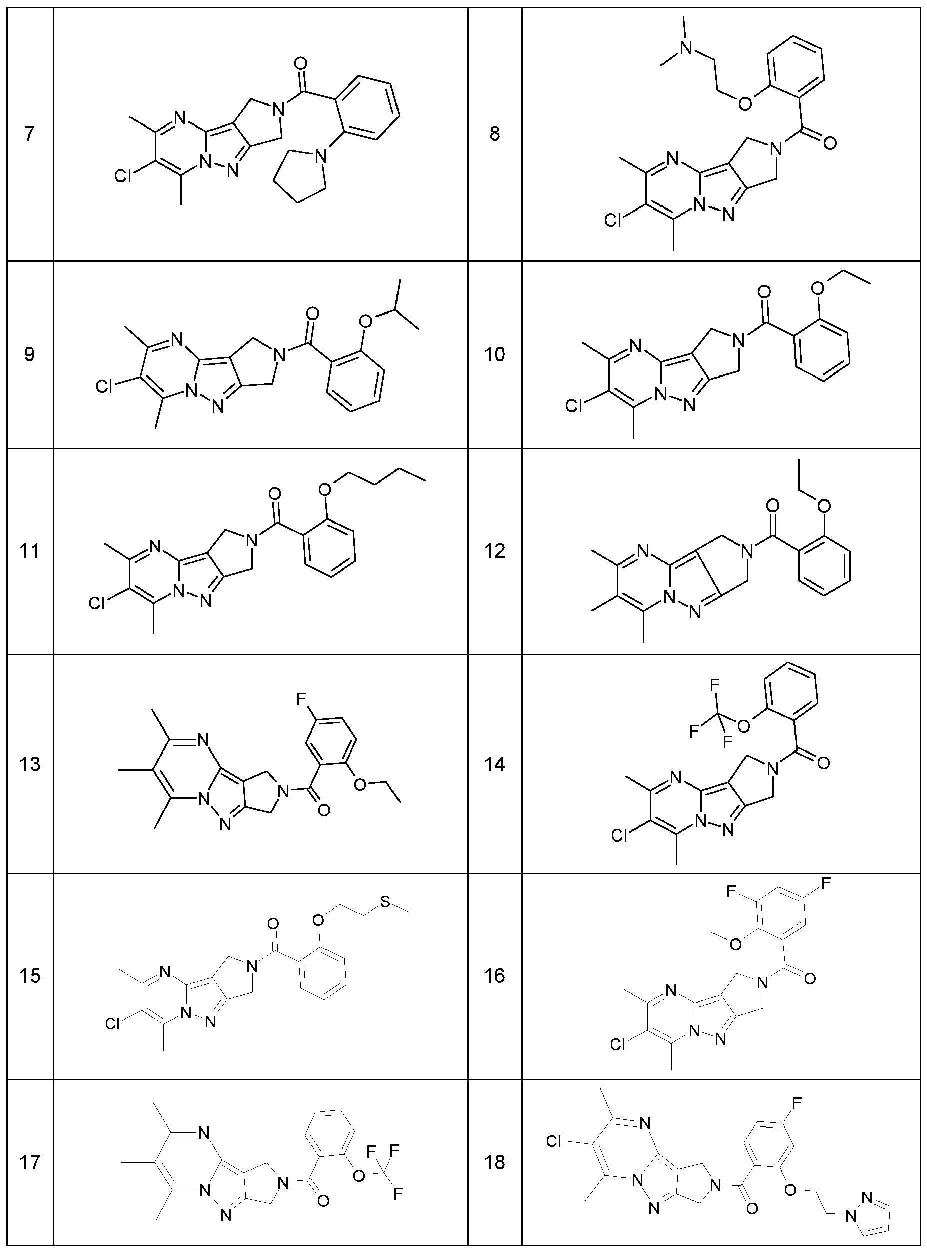

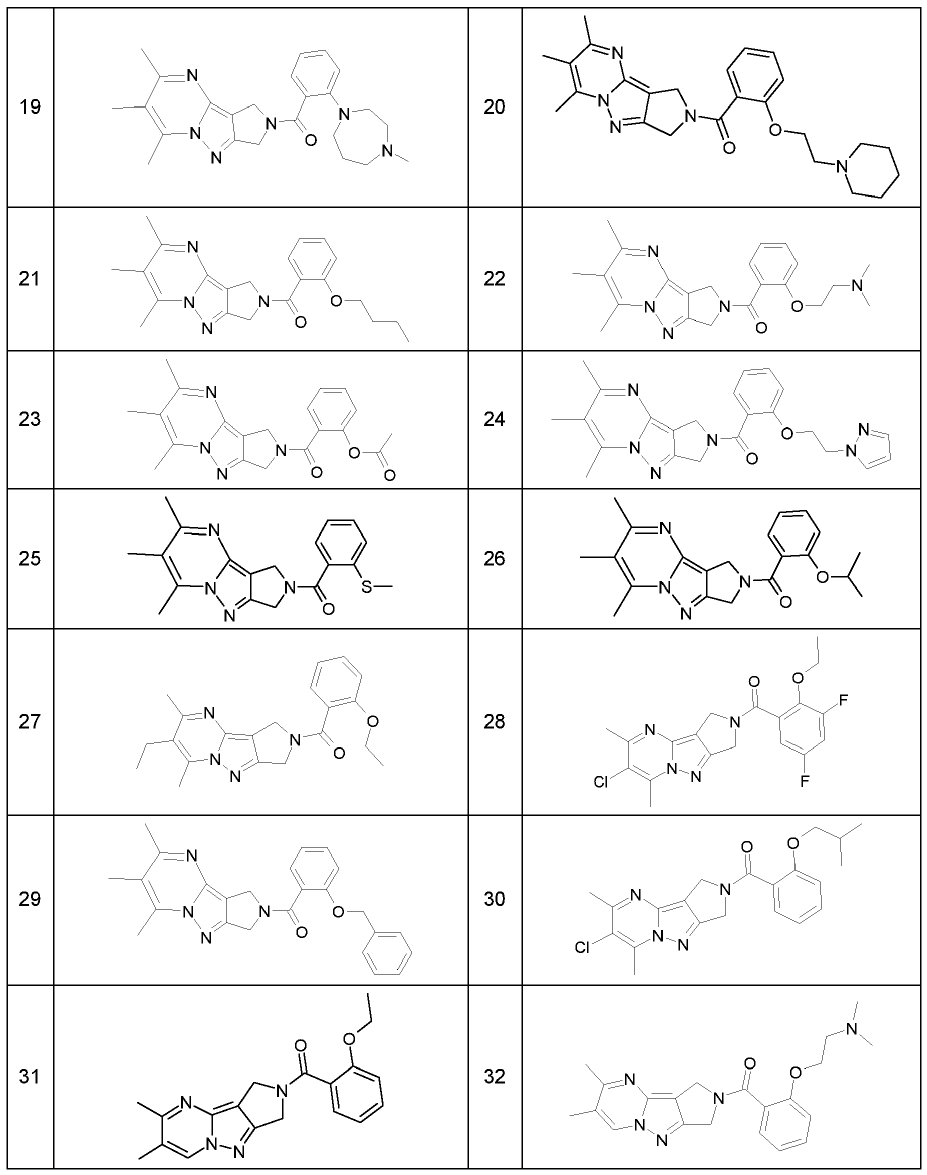

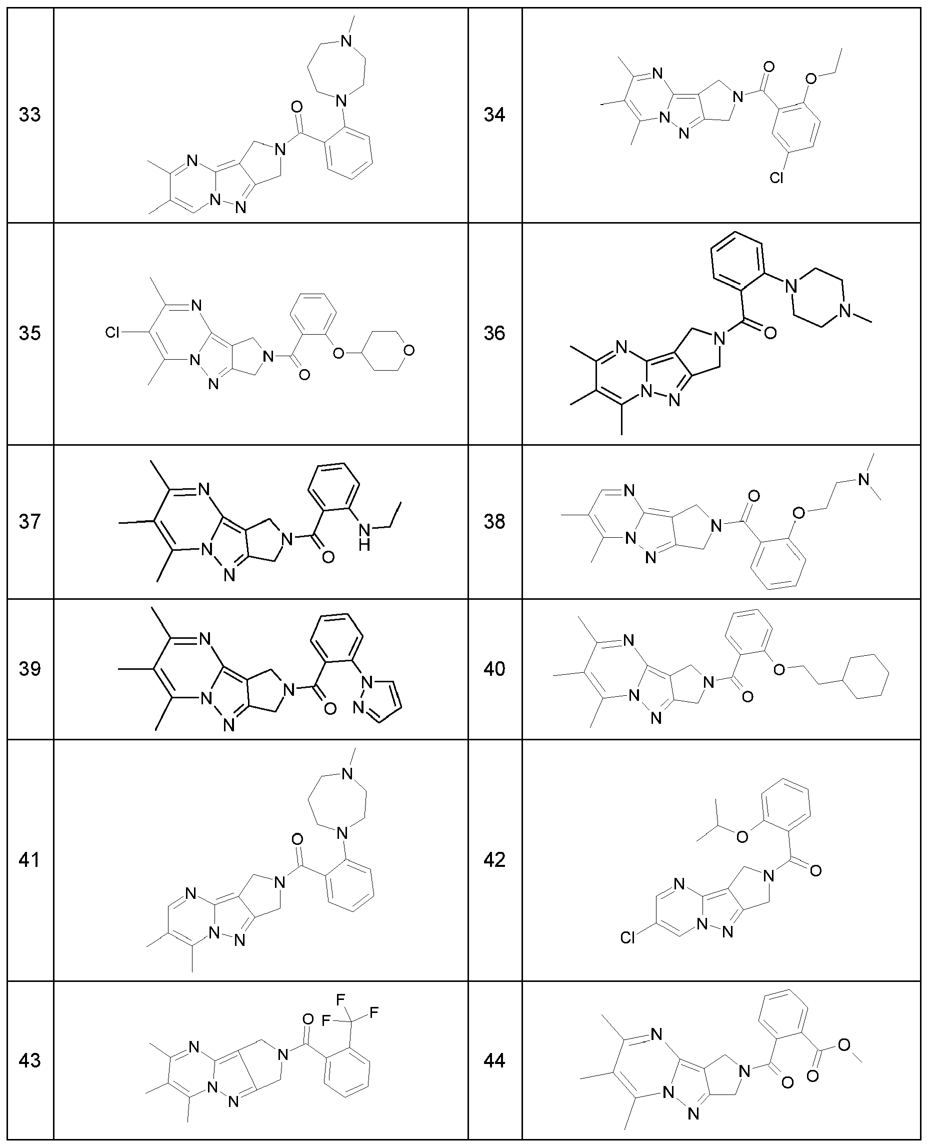

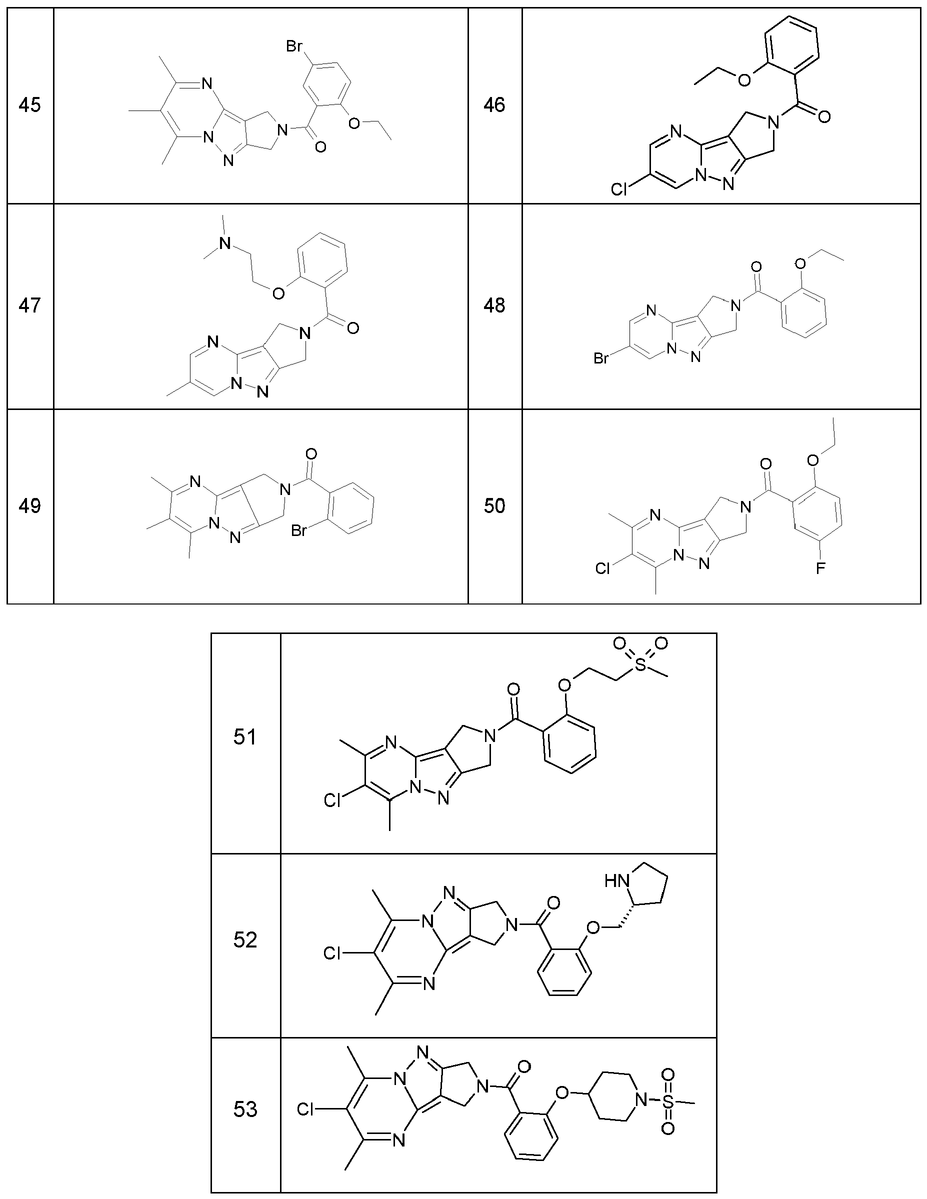

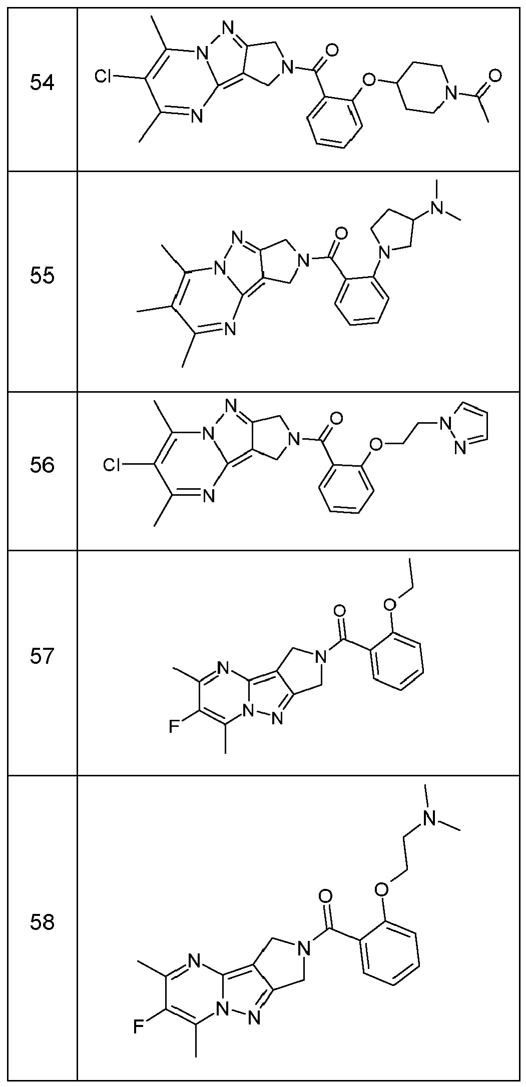

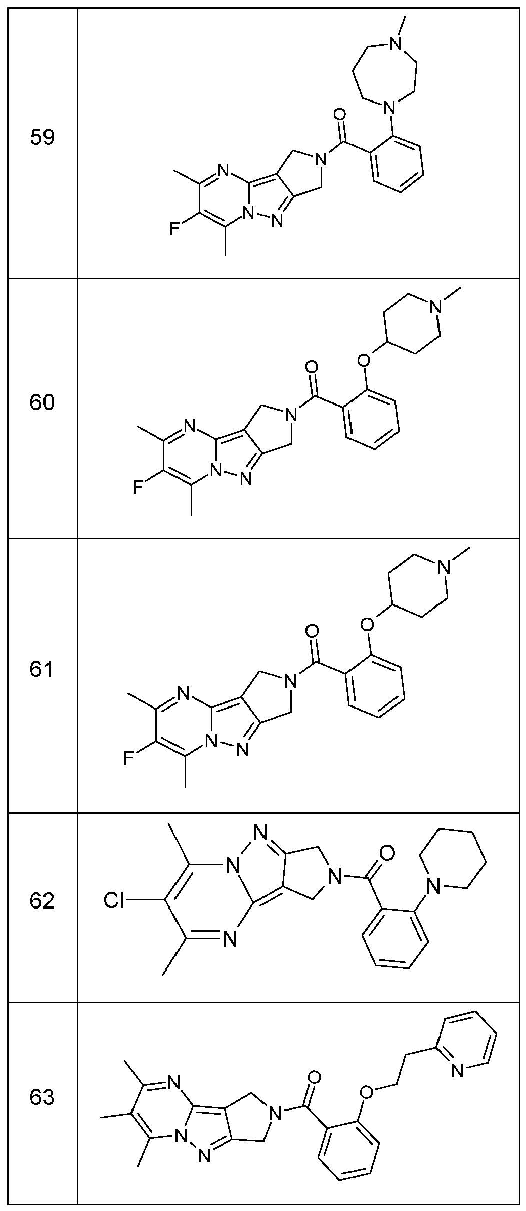

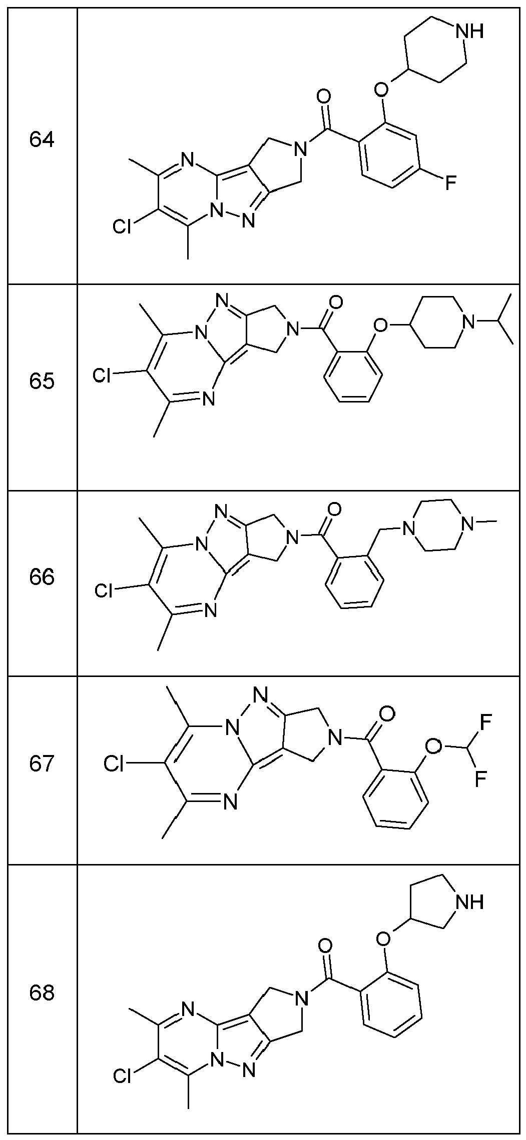

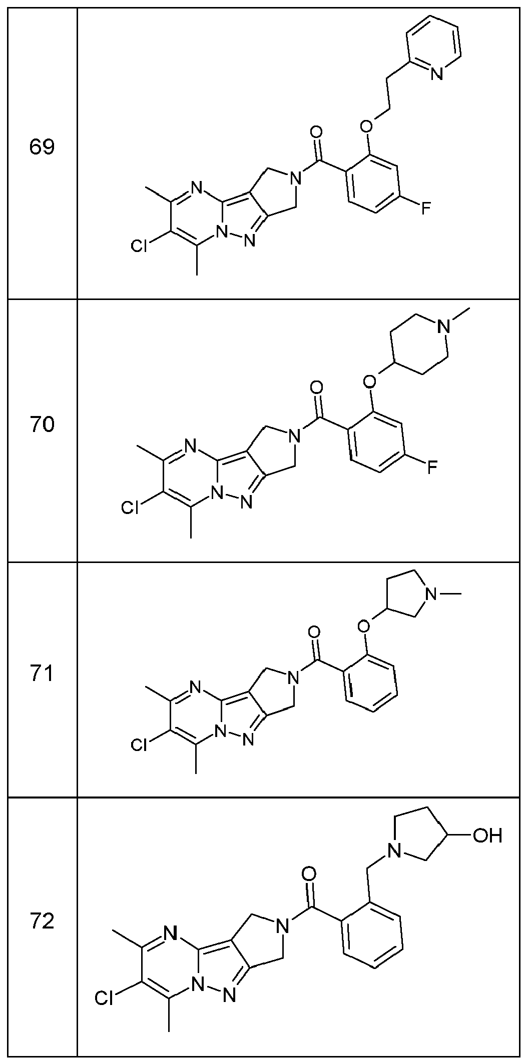

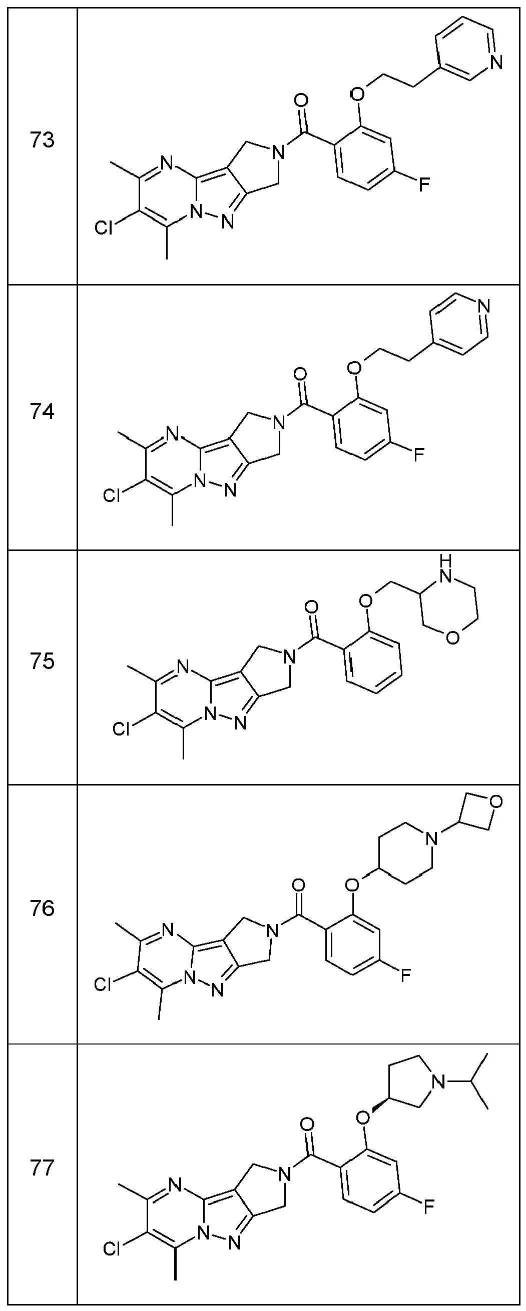

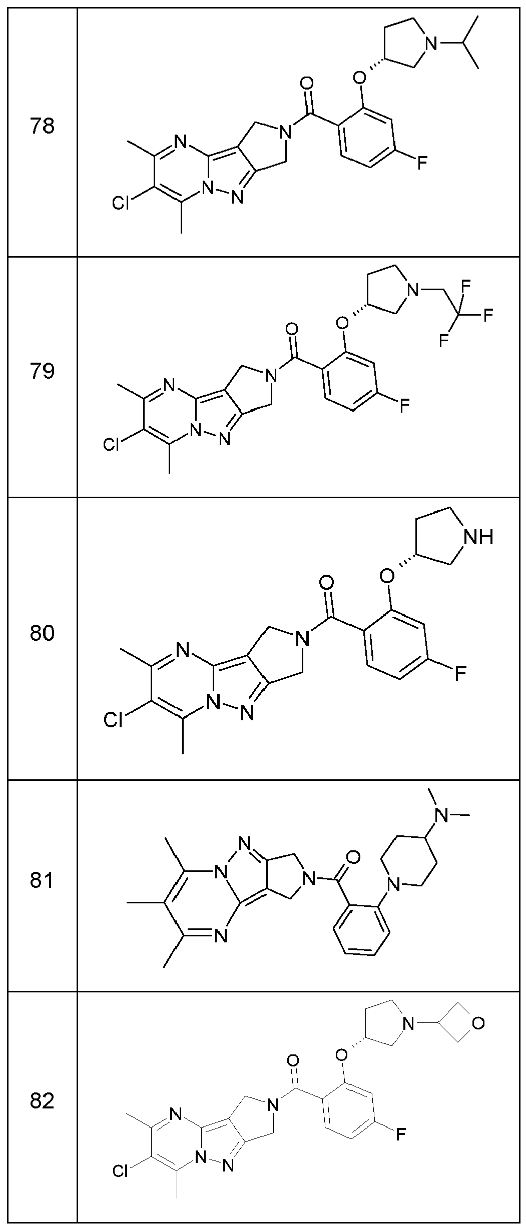

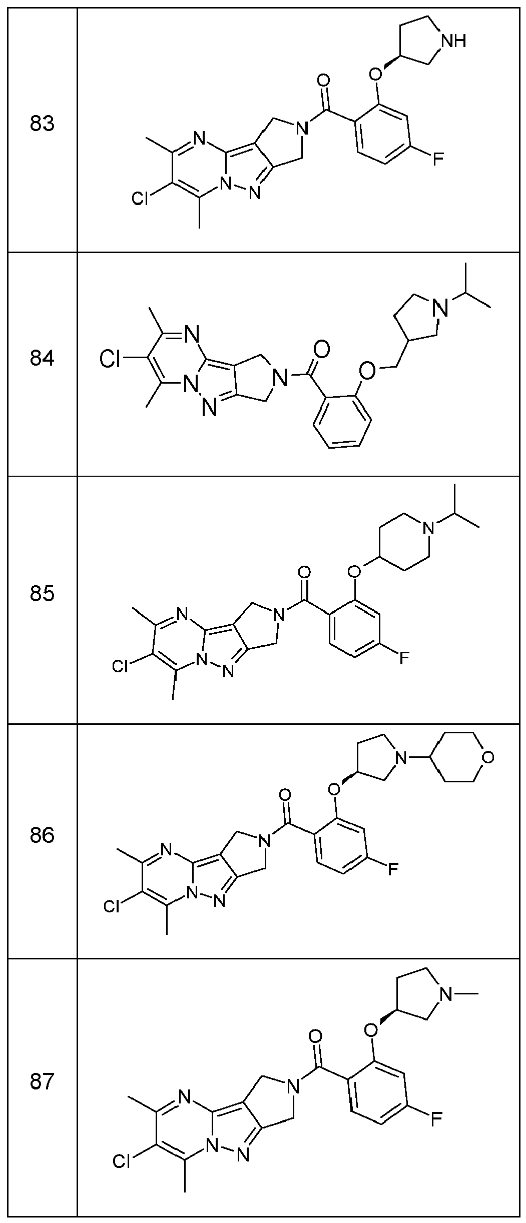

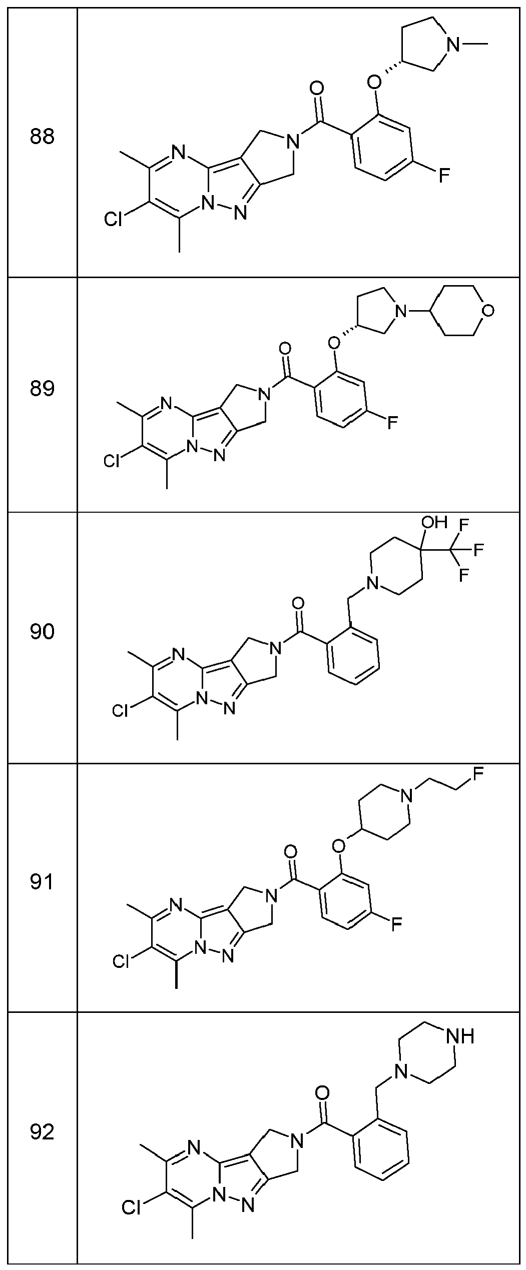

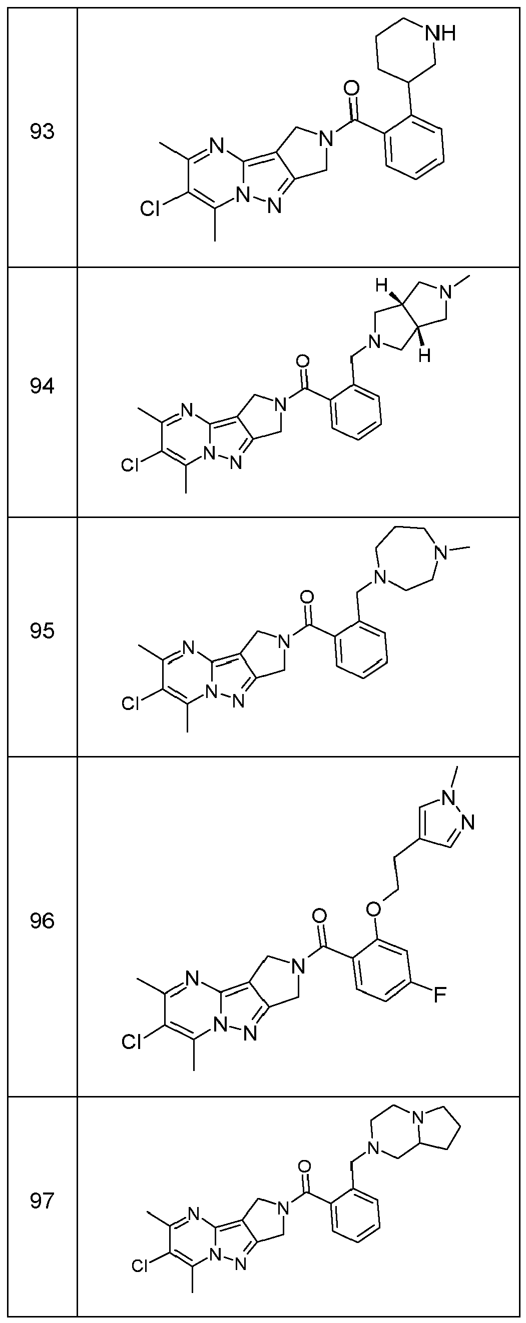

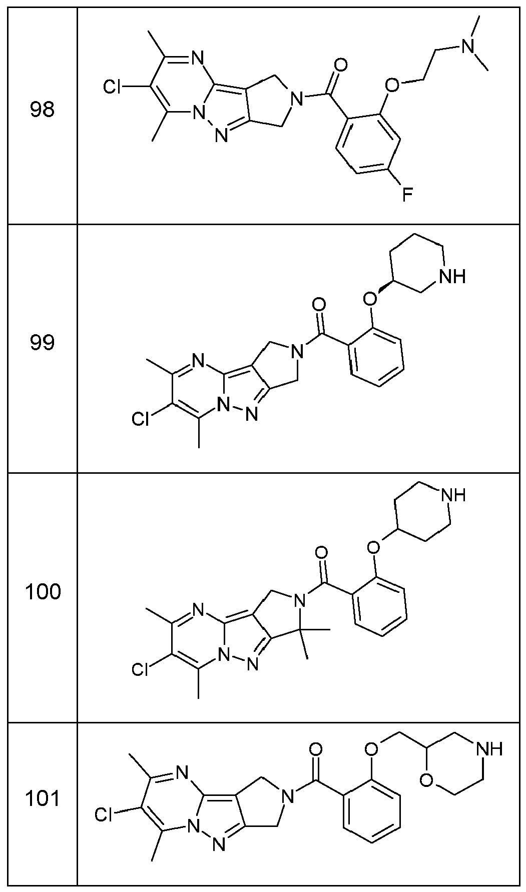

5. A pharmaceutical composition comprising at least one compound of Formula (I) according to one or more of claims 1 to 4 and/or pharmaceutically usable derivatives, tautomers, salts, solvates and stereoisomers thereof, including mixtures thereof in all ratios, and optionally excipients and/or adjuvants.

6. A pharmaceutical composition comprising at least one compound of Formula (I) according to one or more of claims 1 to 4 and/or pharmaceutically usable derivatives, tautomers, salts, solvates and stereoisomers thereof, including mixtures thereof in all ratios, and at least one further active ingredient.

7. Set (kit) consisting of separate packs of
(a) an effective amount of a compound of Formula (I) according to one or more of claims 1 to 4 and/or pharmaceutically usable derivatives, tautomers, salts, solvates and stereoisomers thereof, including mixtures thereof in all ratios, and
(b) an effective amount of a further medicament active ingredient.

8. Compounds according to one or more of claims 1 to 4 and pharmaceutically usable derivatives, salts, tautomers, solvates and stereoisomers thereof, including mixtures thereof in all ratios, for use in the preparation of a medicament for the treatment and/or prophylaxis of a M1 associated disorder.

9. Compounds according to claim 8, wherein the M1 associated disorder is selected from central nervous system disorders.

10. Compounds according to one or more of claims 1 to 4, and pharmaceutically usable derivatives, salts, tautomers, solvates and stereoisomers thereof, including mixtures thereof in all ratios, for use in the preparation of a medicament for the treatment and/or prophylaxis of a central nervous system disorder.

11. Compounds according to claim 10, wherein the central nervous system disorder is Alzheimer's disease, Parkinson disease, schizophrenia, movement disorders and memory disorders, chronic and neuropathic pain, sleep disorders, epilepsy.

12. A process to manufacture the compounds of Formula (I) as defined in claims 1 to 4 comprising the step of reacting a compound of Formula (A) 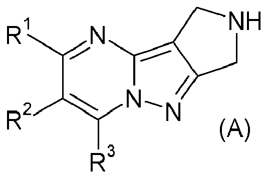 Wherein R¹, R², R³ are as defined in claim 1, with a compound of Formula (B) 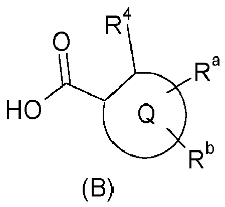 Wherein R⁴, R^{a} and R^{b} are as defined in claim 1, in the presence of a coupling agent. A process according to claim 12 wherein the coupling agent is selected from EDC, HATU, DCC, DIC.
